# EUROPEAN PATENT APPLICATION

(11) **EP 4 613 109 A1**
(43) Date of publication of application: **10.09.2025**
(21) Application number: 23885788.2
(22) Date of filing: 31.10.2023
(51) Int. Cl.: A23L 27/24, A23L 23/00, A23L 27/00, A23L 27/10, A23L 35/00

(54) **METHOD FOR IMPROVING FLAVOR**

(30) Priority: 31.10.2022 JP 2022174054; 12.05.2023 JP 2023079421
(71) Applicant: AJINOMOTO CO., INC., Chuo-ku Tokyo 104-8315 (JP)
(72) Inventor: MIZUNO, Masaki, Kawasaki-shi, Kanagawa 210-8681 (JP); HAYASHI, Kazuyuki, Kawasaki-shi, Kanagawa 210-8681 (JP); MAEDA, Miyoko, Kawasaki-shi, Kanagawa 210-8681 (JP); OOTANI, Keisuke, Kawasaki-shi, Kanagawa 210-8681 (JP); HISHIYA, Naoko, Kawasaki-shi, Kanagawa 210-8681 (JP); ABE, Takaaki, Kawasaki-shi, Kanagawa 210-8681 (JP); SATO, Sumie, Kawasaki-shi, Kanagawa 210-8681 (JP); SATO, Miho, Kawasaki-shi, Kanagawa 210-8681 (JP); ICHINOI, Hirokazu, Kawasaki-shi, Kanagawa 210-8681 (JP); TAKAHASHI, Shun, Kawasaki-shi, Kanagawa 210-8681 (JP)
(74) Representative: Strehl & Partner mbB
(86) International application number: PCT/JP2023/039356
(87) International publication number: WO 2024/096034

(57) **Abstract**

The present invention provides a technique for improving a flavor of food. This technique improves a flavor of food, using a yeast-fermented product of a plant belonging to the family *Solanaceae,* such as tomato.

## Description

### TECHNICAL FIELD

The present invention relates to a technique for improving a flavor of food. Specifically, the present invention can relate to an agent for improving a flavor of food and a method of improving a flavor of food.

### BACKGROUND ART

2-Phenylethanol (2-PE; also referred to as "phenethyl alcohol") is known as a flavor substance contained in soy sauce or miso. Further, 2-phenylethanol, ethyl acetate and diacetyl are all known as constituent components of tomato flavor (Non-patent Document 1).

Yeasts such as *Zygosaccharomyces rouxii* and *Saccharomyces cerevisiae* have been reported to generate flavor components such as 2-phenylethanol (Patent Documents 1 to 4 and Non-patent Documents 2 to 6).

Further, a tomato-containing beverage in which the ratio of the amount of aroma component is adjusted has been reported, and an aromatic alcohol such as 2-phenylethanol is exemplified as such an aroma component (Patent Document 5).

A high lycopene-containing ketchup containing an aroma component(s) such as an ester and/or an alcohol has also been reported, and 2-phenylethanol is exemplified as such an alcohol (Patent Document 6). Patent Document 6 discloses that the ketchup may contain food containing an aroma component, such as a yeast-fermented product of a fruit juice.

In addition, a fruit-fermented product obtained by fermenting a fruit such as tomato with propionic acid-producing bacteria has been reported (Patent Document 7).

There has also been reported a technique of performing lactic acid fermentation using tomato as a raw material, followed by fermentation with yeast and maturation, to produce a soy sauce-like seasoning (Patent Document 8).

Further, a technique of enhancing the spicy aroma of a spice or an aroma component by using methionol and 2-phenylethanol in combination has been reported (Patent Document 9). Patent Document 9 discloses that the spicy aroma of the spice or the aroma component has not been enhanced when 2-phenylethanol alone was used.

### PRIOR ART REFERENCES

### PATENT DOCUMENTS

Patent Document 1: JP6949339B
Patent Document 2: JP2014-204715A
Patent Document 3: CN101723805B
Patent Document 4: CN101016517B
Patent Document 5: JP2014-082944A
Patent Document 6: JP2015-146800A
Patent Document 7: JP2013-544528A
Patent Document 8: JP2013-132221A
Patent Document 9: WO2013/133051

### NON-PATENT DOCUMENTS

Non-patent Document 1: JOHN WRIGTH, Flavor Creation, 2014
Non-patent Document 2: Di Stefano et al., Volatile compounds produced by yeasts, Rivista di Viticoltura e di Enologia (1981), 34 (8), 342-55.
Non-patent Document 3: Dai J et al., Zygosaccharomyces rouxii, an Aromatic Yeast Isolated From Chili Sauce, Is Able to Biosynthesize 2-Phenylethanol via the Shikimate or Ehrlich Pathways, Front. Microbiol. 11: 597454.
Non-patent Document 4: Xuewei Jiang et al., Effect of aroma-producing yeasts in high-salt liquid-state fermentation soy sauce and the biosynthesis pathways of the dominant esters, Food Chemistry Volume 344, 15 May 2021, 128681.
Non-patent Document 5: M.M.W. Etschmann et al., Screening of yeasts for the production of the aroma compound 2-phenylethanol in a molasses-based medium, Biotechnology Letters volume 25, pages 531-536 (2003)
Non-patent Document 6: Davide Ravasio et al., An indirect assay for volatile compound production in yeast strains, Sci Rep. 2014; 4: 3707.

### SUMMARY OF THE INVENTION

### PROBLEM TO BE SOLVED BY THE INVENTION

An aspect of the present invention is to provide a technique for improving a flavor of food. Specifically, an aspect of the present invention can be to provide an agent for improving a flavor of food and a method of improving a flavor of food.

### MEANS FOR SOLVING THE PROBLEM

As a result of intensive studies to achieve the above-mentioned aspect, the present inventors have found out that a yeast-fermented product of a plant belonging to the family *Solanaceae,* such as tomato, has the function of improving a flavor of food, such as a tomato-like flavor-improving function, thereby completing the present invention.

That is, the present invention can be exemplified as follows.
[1] A method of producing a composition for improving a flavor of food, the method comprising a step of culturing yeast in a culture medium containing a plant belonging to the family *Solanaceae,* to obtain a fermented product,
   wherein the composition contains the fermented product.
[2] The method described above (specifically, the method according to [1]), wherein the fermented product contains 2-phenylethanol.
[3] The method described above (specifically, the method according to [1] or [2]), wherein the fermented product contains bacterial cells of the yeast.
[4] A method of producing a composition containing 2-phenylethanol, the method comprising a step of culturing yeast in a culture medium containing a plant belonging to the family *Solanaceae,* to obtain a fermented product containing 2-phenylethanol,
   wherein the composition contains the fermented product.
[5] The method described above (specifically, the method according to [4]), wherein the composition is a composition for improving a flavor of food.
[6] The method described above (specifically, the method according to any one of [1], [2], [3] and [5]), wherein the improvement of the flavor is one or more selected from the group consisting of an improvement in tomato-like flavor, an improvement in spicy flavor and an improvement in matured flavor, in the food.
[7] The method described above (specifically, the method according to [6]), wherein the tomato-like flavor is one or more selected from the group consisting of a fresh flavor of tomato, a ripe flavor of tomato, and a raw tomato-like flavor.
[8] The method described above (specifically, the method according to any one of [2] to [5]), wherein the fermented product further contains ethyl acetate and/or diacetyl.
[9] The method described above (specifically, the method according to any one of [1] to [8]), wherein the yeast is salt-tolerant yeast.
[10] The method described above (specifically, the method according to any one of [1] to [9]), wherein the yeast is yeast belonging to the genus *Zygosaccharomyces,* yeast belonging to the genus *Saccharomyces,* yeast belonging to the genus *Pichia,* yeast belonging to the genus *Candida,* yeast belonging to the genus *Hansenula,* or yeast belonging to the genus *Schizosaccharomyces.*
[11] The method described above (specifically, the method according to any one of [1] to [10]), wherein the yeast is *Zygosaccharomyces rouxii, Zygosaccharomyces sapae, Pichia farinosa, Candida versatilis, Candida etschelsii, Saccharomyces cerevisiae* or *Schizosaccharomyces pombe.*
[12] The method described above (specifically, the method according to any one of [1] to [11]), wherein the yeast is *Zygosaccharomyces rouxii.*
[13] The method described above (specifically, the method according to any one of [1] to [12]), wherein the plant belonging to the family *Solanaceae* is a plant belonging to the genus *Solanum* or a plant belonging to the genus *Capsicum.*
[14] The method described above (specifically, the method according to any one of [1] to [13]), wherein the plant belonging to the family *Solanaceae* is tomato, eggplant, green bell pepper, paprika, Shishito pepper or chili pepper.
[15] The method described above (specifically, the method according to any one of [1] to [14]), wherein the plant belonging to the family *Solanaceae* is tomato.
[16] The method described above (specifically, the method according to any one of [1] to [15]), wherein the content of the plant belonging to the family *Solanaceae* in the culture medium, in terms of salt-free soluble solid content, is from 0.1 to 10% (w/w).
[17] The method described above (specifically, the method according to any one of [1] to [16]), wherein the culture medium further contains phenylalanine.
[18] The method described above (specifically, the method according to [17]),
   wherein the content of phenylalanine in the culture medium is from 0.02 to 2% (w/w).
[19] The method described above (specifically, the method according to any one of [1] to [18]), wherein the culture medium further contains sodium chloride.
[20] The method described above (specifically, the method according to [19]), wherein the content of sodium chloride in the culture medium is from 0 to 20% (w/w).
[21] The method described above (specifically, the method according to any one of [1] to [20]), wherein the composition is a seasoning.
[22] The method described above (specifically, the method according to any one of [1] to [21]), further comprising a step of drying and powdering the fermented product.
[23] The method described above (specifically, the method according to any one of [1] to [22]), wherein the food is tomato-containing food.
[24] The method described above (specifically, the method according to any one of [1] to [23]), wherein the food is spice-containing food.
[25] The method described above (specifically, the method according to [24], wherein the spice is one or more spices selected from the group consisting of cardamom, bay leaf, coriander, clove, nutmeg, mace, allspice, cinnamon, fennel, cumin, ginger, pepper, caraway, anise, basil, parsley, sage, thyme, oregano, rosemary, celery seed, mint, garden cress, dill, marjoram, knotweed, turmeric, lemongrass, chili pepper, Japanese pepper, garlic, shallot, onion, peppermint, Sichuan pepper, star anise, wasabi and celery.
[26] The method described above (specifically, the method according to any one of [1] to [25]), wherein the food is food containing: a seasoning obtained by fermenting beans or wheat; a seasoning containing rice malt or miso; or a seasoning obtained by fermenting beans or wheat and containing rice malt or miso.
[27] The method described above (specifically, the method according to any one of [1] to [26]), wherein the food is milk-containing food and/or a dairy product.
[28] A composition produced by the method described above (specifically, the method according to any one of [1] to [27]).
[29] A composition for improving a flavor of food,
   wherein the composition contains a yeast-fermented product of a plant belonging to the family *Solanaceae.*
[30] The composition described above (specifically, the composition according to [29]), wherein the improvement of the flavor is one or more selected from the group consisting of an improvement in tomato-like flavor, an improvement in spicy flavor and an improvement in matured flavor, in the food.
[31] The composition described above (specifically, the composition according to [29] or [30]), wherein the fermented product contains bacterial cells of the yeast.
[32] The composition described above (specifically, the composition according to any one of [29] to [30]), wherein the fermented product contains 2-phenylethanol.
[33] The composition described above (specifically, the composition according to [32]), wherein the fermented product further contains ethyl acetate and/or diacetyl.
[34] The composition described above (specifically, the composition according to any one of [29] to [33]), wherein the fermented product is produced by a step of culturing yeast in a culture medium containing the plant.
[35] The composition described above (specifically, the composition according to any one of [29] to [4]), wherein the yeast is *Zygosaccharomyces rouxii.*
[36] The composition described above (specifically, the composition according to any one of [29] to [35]), wherein the plant belonging to the family *Solanaceae* is tomato.
[37] The composition described above (specifically, the composition according to [34]), wherein the culture medium further contains phenylalanine.
[38] The composition described above (specifically, the composition according to any one of [29] to [37]), wherein the food is tomato-containing food.
[39] The composition described above (specifically, the composition according to any one of [29] to [38]), wherein the food is spice-containing food.
[40] The composition described above (specifically, the composition according to [39]), wherein the spice is one or more spices selected from the group consisting of cardamom, bay leaf, coriander, clove, nutmeg, mace, allspice, cinnamon, fennel, cumin, ginger, pepper, caraway, anise, basil, parsley, sage, thyme, oregano, rosemary, celery seed, mint, garden cress, dill, marjoram, knotweed, turmeric, lemongrass, chili pepper, Japanese pepper, garlic, shallot, onion, peppermint, Sichuan pepper, star anise, wasabi and celery.
[41] The composition described above (specifically, the composition according to any one of [29] to [40]), wherein the food is food containing: a seasoning obtained by fermenting beans or wheat; a seasoning containing rice malt or miso; or a seasoning obtained by fermenting beans or wheat and containing rice malt or miso.
[42] The composition described above (specifically, the composition according to any one of [29] to [41]), wherein the food is milk-containing food and/or a dairy product.
[43] A method of improving a flavor of food, the method including a step of adding a yeast-fermented product of a plant belonging to the family *Solanaceae* to a raw material of the food.
[44] The method described above (specifically, the method according to [43]),
   wherein the improvement of the flavor is one or more selected from the group consisting of an improvement in tomato-like flavor, an improvement in spicy flavor and an improvement in matured flavor, in the food.
[45] The method described above (specifically, the method according to [43] or [44]), wherein the fermented product contains bacterial cells of the yeast.
[46] The method described above (specifically, the method according to [43] or [44]), wherein the fermented product contains 2-phenylethanol.
[47] The method described above (specifically, the method according to [46]),
   wherein the fermented product further contains ethyl acetate and/or diacetyl.
[48] The method described above (specifically, the method according to any one of [43] to [47]), wherein the fermented product is produced by a step of culturing yeast in a culture medium containing the plant.
[49] The method described above (specifically, the method according to any one of [43] to [48]), wherein the yeast is *Zygosaccharomyces rouxii.*
[50] The method described above (specifically, the method according to any one of [43] to [49]), wherein the plant belonging to the family *Solanaceae* is tomato.
[51] The method described above (specifically, the method according to [48]),
   wherein the culture medium further contains phenylalanine.
[52] The method described above (specifically, the method according to any one of [43] to [51]), wherein the food is tomato-containing food.
[53] The method described above (specifically, the method according to any one of [43] to [52]), wherein the food is spice-containing food.
[54] The method described above (specifically, the method according to [53]), wherein the spice is one or more spices selected from the group consisting of cardamom, bay leaf, coriander, clove, nutmeg, mace, allspice, cinnamon, fennel, cumin, ginger, pepper, caraway, anise, basil, parsley, sage, thyme, oregano, rosemary, celery seed, mint, garden cress, dill, marjoram, knotweed, turmeric, lemongrass, chili pepper, Japanese pepper, garlic, shallot, onion, peppermint, Sichuan pepper, star anise, wasabi and celery.
[55] The method described above (specifically, the method according to any one of [43] to [54]), wherein the food is milk-containing food and/or a dairy product.
[56] The method described above (specifically, the method according to any one of [43] to [55]), wherein the food is food containing: a seasoning obtained by fermenting beans or wheat; a seasoning containing rice malt or miso; or a seasoning obtained by fermenting beans or wheat and containing rice malt or miso.
[57] The method described above (specifically, the method according to any one of [43] to [56]), wherein the fermented product is added such that the eating concentration, in terms of the eating concentration of the original fermented product, is from 1 × 10⁻¹⁴ to 10% (w/w).
[58] The method described above (specifically, the method according to any one of [46]), wherein the fermented product is added such that the eating concentration, in terms of the eating concentration of 2-phenylethanol, is from 1 × 10⁻¹³ to 100 ppm (w/w).
[59] A use of a yeast-fermented product of a plant belonging to the family *Solanaceae,* for improving a flavor of food.
[60] The use described above (specifically, the use according to [59]), wherein the improvement of the flavor is one or more selected from the group consisting of an improvement in tomato-like flavor, an improvement in spicy flavor and an improvement in matured flavor, in the food.
[61] The use described above (specifically, the use according to [59] or [60]), wherein the fermented product contains bacterial cells of the yeast.
[62] The use described above (specifically, the use according to any one of [59] to [61]), wherein the fermented product contains 2-phenylethanol.
[63] The use described above (specifically, the use according to [62]), wherein the fermented product further contains ethyl acetate and/or diacetyl.
[64] The use described above (specifically, the use according to any one of [59] to [63]), wherein the fermented product is produced by a step of culturing yeast in a culture medium containing the plant.
[65] The use described above (specifically, the use according to any one of [59] to [64]), wherein the yeast is *Zygosaccharomyces rouxii.*
[66] The use described above (specifically, the use according to any one of [59] to [65]), wherein the plant belonging to the family *Solanaceae* is tomato.
[67] The use described above (specifically, the use according to [64]), wherein the culture medium further contains phenylalanine.
[68] The use described above (specifically, the use according to any one of [59] to [67]), wherein the food is tomato-containing food.
[69] The use described above (specifically, the use according to any one of [59] to [68]), wherein the food is spice-containing food.
[70] The use described above (specifically, the use according to [69]), wherein the spice is one or more spices selected from the group consisting of cardamom, bay leaf, coriander, clove, nutmeg, mace, allspice, cinnamon, fennel, cumin, ginger, pepper, caraway, anise, basil, parsley, sage, thyme, oregano, rosemary, celery seed, mint, garden cress, dill, marjoram, knotweed, turmeric, lemongrass, chili pepper, Japanese pepper, garlic, shallot, onion, peppermint, Sichuan pepper, star anise, wasabi and celery.
[71] The use described above (specifically, the use according to any one of [59] to [70]), wherein the food is food containing: a seasoning obtained by fermenting beans or wheat; a seasoning containing rice malt or miso; or a seasoning obtained by fermenting beans or wheat and containing rice malt or miso.
[72] The use described above (specifically, the use according to any one of [59] to [71]), wherein the food is milk-containing food and/or a dairy product.

### MODE FOR CARRYING OUT THE INVENTION

The present invention will be described below in detail.

### 1. Active Ingredient

In the present invention, a yeast-fermented product of a plant belonging to the family *Solanaceae* is used as an active ingredient. The yeast-fermented product of a plant belonging to the family *Solanaceae* is also referred to as "active ingredient".

The use of the active ingredient makes it possible to improve a flavor of food, namely, makes it possible to obtain the effect of improving a flavor of food. The effect described above is also referred to as "flavor-improving effect". In other words, the active ingredient has the function of improving a flavor of food. The function described above is also referred to as "flavor-improving function". The improvement of the flavor of food is also simply referred to as "flavor improvement". In an embodiment in which food is heated and/or stored, the flavor-improving effect is preferably maintained even after heating the food and/or after storing the food for a certain period of time. In the case of a retort food, as well, the flavor-improving effect is preferably maintained even after heating. In an embodiment in which food is heated, the flavor-improving effect is preferably maintained even after heating the food at a temperature of 60°C or more, 70°C or more, 80°C or more, 90°C or more, 100°C or more, or 110°C or more.

The improvement of the flavor may be, for example, an improvement in tomato-like flavor in food. That is, the flavor-improving effect may be, for example, the effect of improving the tomato-like flavor in food. The effect described above is also referred to as "tomato-like-flavor-improving effect". Further, the flavor-improving function may be, for example, the function of improving the tomato-like flavor in food. The function described above is also referred to as "tomato-like-flavor-improving function". An improvement in tomato-like flavor in food is also simply referred to as "improvement in tomato-like flavor". The "improvement in (or improving) the tomato-like flavor" is also referred to as "enhancement in (or enhancing) the tomato-like flavor" or "impartment of (or imparting) the tomato-like flavor". The tomato-like flavor may be, for example, a fresh flavor of tomato, a ripe flavor of tomato or a raw tomato-like flavor. The fresh flavor of tomato may be, for example, a sharp tomato flavor which is sensed when food is put into the mouth. The ripe flavor of tomato may be, for example, a tomato flavor with a thick, sweet aroma in the middle taste and aftertaste. The raw tomato-like flavor may be, for example, a tomato flavor with a raw tomato-like grassy smell.

Further, the improvement of the flavor may be, for example, an improvement in spicy flavor in food. That is, the flavor-improving effect may be, for example, the effect of improving the spicy flavor in food. The effect described above is also referred to as "spicy-flavor-improving effect". In addition, the flavor-improving function may be, for example, the function of improving the spicy flavor in food. The function described above is also referred to as "spicy-flavor-improving function". An improvement in spicy flavor in food is also simply referred to as "improvement in spicy flavor". The "improvement in (or improving) the spicy flavor" is also referred to as "enhancement in (or enhancing) the spicy flavor" or "impartment of (or imparting) the spicy flavor". Examples of the spicy flavor include the aroma, flavor, richness, pungency and stimulus intensity of spices, as well as the refreshing flavor and the gorgeousness specific to spices. In particular, examples of the spicy flavor include the aroma, flavor, richness, pungency and stimulus intensity of spices, as well as the refreshing flavor and the gorgeousness specific to spices, of: cardamom, bay leaf, coriander, clove, nutmeg, mace, allspice, cinnamon, fennel, cumin, ginger, pepper, caraway, anise, basil, parsley, sage, thyme, oregano, rosemary, celery seed, mint, garden cress, dill, marjoram, knotweed, turmeric, lemongrass, chili pepper, Japanese pepper, garlic, shallot, onion, peppermint, Sichuan pepper, star anise, wasabi, and celery. In particular, the spicy flavor may be, a pungent and stimulating spicy flavor, such as that of pepper or chili pepper, or may be a refreshing spicy flavor that stands out initially, such as that of wasabi, cardamom, ginger, Japanese pepper or Sichuan pepper. The spicy flavor may particularly be a refreshing spicy flavor that stands out initially, such as that of wasabi, cardamom, ginger, Japanese pepper or Sichuan pepper.

Further, the improvement of the flavor may be, for example, an improvement in matured flavor in food. That is, the flavor-improving effect may be, for example, the effect of improving the matured flavor in food. The effect described above is also referred to as "matured-flavor-improving effect". In addition, the flavor-improving function may be, for example, the function of improving the matured flavor in food. The function described above is also referred to as "matured-flavor-improving function". An improvement in matured flavor in food is also simply referred to as "improvement in matured flavor". The "improvement in (or improving) the matured flavor" is also referred to as "enhancement in (or enhancing) the matured flavor" or "impartment of (or imparting) the matured flavor". The matured flavor may be, for example, the matured aroma, flavor and/or richness of a seasoning obtained by fermenting beans or wheat. The matured flavor may be, for example, the matured aroma, flavor and/or richness of a seasoning containing rice malt or miso. The matured flavor may be, for example, the matured aroma, flavor and/or richness of a seasoning obtained by fermenting beans or wheat and containing rice malt or miso. The matured flavor may be, for example, the matured aroma, flavor, richness and/or thickness, of milk such as cow's milk, or of a dairy product such as cheese, yoghurt or butter. Further, the matured flavor may be, for example, the richness of a vegetable or a spice, or the thickness of matured curry. In particular, the matured flavor may be, for example, the matured aroma, flavor and/or richness of douchi (fermented soy beans), tianmianjiang (sweet bean paste), doubanjiang (broad bean paste), cheese or curry. The matured flavor can also be expressed as "brewed flavor".

The improvement of the flavor may be an improvement in one or more flavors selected from the group consisting of the tomato-like flavor, the spicy flavor and the matured flavor. The improvement of the flavor may be improving a combination of two or three arbitrarily selected from the group consisting of the tomato-like flavor, the spicy flavor and the matured flavor, in one food. Specifically, the improvement of the flavor may be improving the tomato-like flavor and the spicy flavor, improving the tomato-like flavor and the matured flavor, improving the spicy flavor and the matured flavor, or improving the tomato-like flavor, the spicy flavor and the matured flavor.

The flavor (such as the tomato-like flavor, the spicy flavor or the matured flavor) may be divided, for example, into "initial taste", "middle taste" and "aftertaste". In the case of a liquid (in the case of a liquid food), the "initial taste", the "middle taste" and the "aftertaste" of the flavor refer to the flavors perceived during the period of time from 0 to 1 second, from 1 to 3 seconds and from 3 to 5 seconds after eating (after putting the food in the mouth), respectively. In the case of a solid (in the case of a solid food), the "initial taste", the "middle taste" and the "aftertaste" of the flavor refer to the flavors perceived during the period of time from 0 to 4 seconds, from 4 to 10 seconds and from 10 to 15 seconds after eating (after putting the food in the mouth), respectively. In the present invention, the "solid" refers to a form other than a liquid, and the definition thereof includes a paste, a gel and the like. For example, the flavor in the initial taste, the flavor in the middle taste or the flavor in the aftertaste, or any combination thereof may be improved, by using the active ingredient. That is, the use of the active ingredient may improve, specifically, for example, the tomato-like flavor in the initial taste, the tomato-like flavor in the middle taste or the tomato-like flavor in the aftertaste, or any combination thereof. Further, the use of the active ingredient may improve, specifically, for example, the spicy flavor in the initial taste, the spicy flavor in the middle taste or the spicy flavor in the aftertaste, or any combination thereof. In addition, the use of the active ingredient may improve, specifically, for example, the matured flavor in the initial taste, the matured flavor in the middle taste or the matured flavor in the aftertaste, or any combination thereof.

Specifically, the use of the active ingredient makes it possible to improve a flavor of food (for example, to improve the tomato-like flavor, the spicy flavor and/or the matured flavor, in the food), as compared to the case in which the active ingredient is not used. Accordingly, the flavor-improving effect (such as the tomato-like-flavor-improving effect, the spicy flavor-improving effect and/or the matured flavor-improving effect) can be determined by measuring a flavor of food (such as the tomato-like flavor in the food) when not using the active ingredient and when using the active ingredient, and by comparing the measured results. That is, it can be determined that the flavor-improving effect is obtained, in cases where a flavor of food when using the active ingredient is more preferred as compared to the case of not using the active ingredient. Specifically, for example, it can be determined that the tomato-like-flavor-improving effect is obtained, in cases where the tomato-like flavor in food when using the active ingredient is stronger as compared to that in the case of not using the active ingredient. Specifically, for example, it can be determined that the spicy flavor-improving effect is obtained, in cases where the spicy flavor in food when using the active ingredient is stronger as compared to that in the case of not using the active ingredient. Specifically, for example, it can be determined that the matured flavor-improving effect is obtained, in cases where the matured flavor in food when using the active ingredient is stronger as compared to that in the case of not using the active ingredient. The measurement and comparison of a flavor of food (such as the tomato-like flavor, the spicy flavor and/or the matured flavor, in the food) can be carried out, for example, by a sensory evaluation performed by experts (expert panel).

The "yeast-fermented product of a plant belonging to the family *Solanaceae"* refers to a product obtained by fermenting a plant belonging to the family *Solanaceae* with yeast. The term "fermentation (or fermenting)" as used herein may mean being used in culture as a culture medium component. That is, the expression "fermenting a plant belonging to the family *Solanaceae* with yeast" may mean "culturing yeast in a culture medium containing a plant belonging to the family *Solanaceae".* That is, the "yeast-fermented product of a plant belonging to the family *Solanaceae"* may refer specifically to a product obtained by culturing yeast in a culture medium containing a plant belonging to the family *Solanaceae.*

The active ingredient may have the flavor-improving function, by containing a component having the flavor-improving function (such as the tomato-like flavor-improving function, the spicy flavor-improving function and/or the matured flavor-improving function). That is, the active ingredient may contain a component having the flavor-improving function. The component having the flavor-improving function may be, for example, a component that accumulates in a significant amount in a yeast-fermented product obtained by fermenting a plant belonging to the family *Solanaceae* with yeast. Examples of such a component include 2-phenylethanol (2-PE), ethyl acetate and diacetyl. Each of 2-phenylethanol (2-PE), ethyl acetate and diacetyl can be a component having the tomato-like flavor-improving function. Further, each of 2-phenylethanol (2-PE), ethyl acetate and diacetyl can be a component having the spicy flavor-improving function. In one embodiment, 2-PE can be a component which does not have the spicy flavor-improving function by itself alone, or alternatively, a component which has a weak spicy flavor-improving function by itself alone, and whose spicy flavor-improving function is increased by a combination with another component(s) (such as ethyl acetate and/or diacetyl). In addition, each of 2-phenylethanol (2-PE), ethyl acetate and diacetyl can be a component having the matured flavor-improving function. In particular, the component having the flavor-improving function may be, for example, 2-PE. That is, for example, the active ingredient may contain at least 2-PE. For example, the active ingredient may contain 2-PE, and may further contain ethyl acetate and/or diacetyl. In particular, the active ingredient may contain 2-PE, ethyl acetate and diacetyl.

The method of producing the active ingredient will be described below. That is, the active ingredient may be one produced by the method to be described below.

The active ingredient can be produced by fermenting a plant belonging to the family *Solanaceae* with yeast (specifically, by culturing yeast in a culture medium containing a plant belonging to the family *Solanaceae).*

That is, first of all, yeast may be cultured in a culture medium containing a plant belonging to the family *Solanaceae.* In other words, the method of producing the active ingredient may include a step of culturing yeast in a culture medium containing a plant belonging to the family *Solanaceae.* The step described above is also referred to as "culturing step". The culturing step may specifically be a step of culturing yeast in a culture medium containing a plant belonging to the family *Solanaceae,* to obtain a cultured product. The cultured product described above is also referred to as "fermented product".

The type of the yeast is not particularly limited, as long as the active ingredient can be produced. The yeast may be a budding yeast or a fission yeast. The yeast may be haploid yeast, diploid yeast or yeast of a higher ploidy. Examples of the yeast include: yeasts belonging to the genus *Zygosaccharomyces,* such as *Zygosaccharomyces rouxii* and *Zygosaccharomyces sapae;* yeasts belonging to the genus *Saccharomyces,* such as *Saccharomyces cerevisiae;* yeasts belonging to the genus *Pichia* (also referred to as the genus *Wickerhamomyces*), such as *Pichia farinosa, Pichia ciferrii, Pichia sydowiorum* and *Pichia pastoris;* yeasts belonging to the genus *Candida,* such as *Candida versatilis, Candida etschelsii* and *Candida utilis;* yeasts belonging to the genus *Hansenula,* such as *Hansenula polymorpha;* and yeasts belonging to the genus *Schizosaccharomyces,* such as *Schizosaccharomyces pombe.* Examples of the yeast include, in particular, *Zygosaccharomyces rouxii, Zygosaccharomyces sapae, Pichia farinosa, Candida versatilis, Candida etschelsii, Saccharomyces cerevisiae* and *Schizosaccharomyces pombe.* Further, examples of the yeast include, in particular, yeasts belonging to the genus *Zygosaccharomyces.* More particularly, examples of the yeast include *Zygosaccharomyces rouxi.* Examples of *Zygosaccharomyces rouxii* include *Zygosaccharomyces rouxii* NBRC 1130 strain (ATCC 56077). Examples of *Saccharomyces cerevisiae* include *Saccharomyces cerevisiae* NBRC 10217 strain (ATCC 18824), BY4742 strain (ATCC 201389), BY4743 strain (ATCC 201390) and S288C strain (ATCC 26108). Examples of *Schizosaccharomyces pombe* include *Schizosaccharomyces pombe* NBRC 1628 strain. The yeast may be, for example, salt-tolerant yeast. The "salt-tolerant yeast" may refer, for example, to yeast which can be grown in a culture medium containing 8% (w/w) sodium chloride. For example, each of *Zygosaccharomyces rouxii, Zygosaccharomyces sapae, Pichia farinosa, Candida versatilis* and *Candida etschelsii* can be one example of the salt-tolerant yeast. The salt-tolerant yeast may be, for example, a soy sauce yeast. The "soy sauce yeast" may refer, for example, to yeast usually used in the production of soy sauce. For example, *Zygosaccharomyces rouxii, Zygosaccharomyces bailli, Candida* etchellsii or *Candida versatilis* can be one example of the soy sauce yeast.

These bacterial strains can be distributed, for example, from the American Type Culture Collection (ATCC, Address: 10801 University Boulevard Manassas, VA 20110, United States of America). That is, a corresponding accession number is given to each bacterial strain, and each strain can be distributed using the accession number (see, http://www.atcc.org/). The accession number corresponding to each bacterial strain is described in the catalog of the American Type Culture Collection. Further, these bacterial strains can be obtained, for example, from NBRC (NITE Biological Resource Center). These bacterial strains can also be obtained, for example, from depository institutions to which the respective bacterial strains have been deposited.

Culturing conditions are not particularly limited, as long as the active ingredient can be produced. The culture can be carried out, for example, under conditions usually used for culturing yeast, except for using a culture medium containing a plant belonging to the family *Solanaceae.* The culture may be carried out, for example, by solid culture (namely, on a solid culture medium), or by liquid culture (namely, in a liquid culture medium). In particular, the culture may be carried out by liquid culture (namely, in a liquid culture medium).

The culture medium to be used for the culture is not particularly limited, as long as the medium contains a plant belonging to the family *Solanaceae,* and the active ingredient can be produced. For example, a culture medium containing any component selected from carbon sources, nitrogen sources, phosphate sources, sulfur sources, and various types of other organic components and inorganic components, as necessary, in addition to a plant belonging to the family *Solanaceae,* can be used as the culture medium. The plant belonging to the family *Solanaceae* may be used in the culture, for example, as a nitrogen source. The culture medium may but need not contain another nitrogen source, in addition to the plant belonging to the family *Solanaceae.* The plant belonging to the family *Solanaceae* may be used in the culture, for example, as a main nitrogen source. The expression "the plant belonging to the family *Solanaceae* is a main nitrogen source" may mean, for example, that the amount of total nitrogen derived from the plant belonging to the family *Solanaceae* is, for example, 50% (w/w) or more, 70% (w/w) or more, or 90% (w/w) or more, with respect to the amount of total nitrogen derived from all culture medium components excluding phenylalanine. The types and the concentrations of the culture medium components can be set as appropriate, by those skilled in the art. As the specific composition of the culture medium, for example, a culture medium composition usually used for culturing yeast can be referred to.

The type of the plant belonging to the family *Solanaceae* is not particularly limited, as long as the active ingredient can be produced. The plant belonging to the family *Solanaceae* may be, for example, a plant belonging to the genus *Solanum,* or a plant belonging to the genus *Capsicum.* The plant belonging to the genus *Solanum* may be, for example, tomato or eggplant. The plant belonging to the genus *Capsicum* may be, for example, green bell pepper, paprika, Shishito pepper or chili pepper. The green bell pepper may be, for example, green bell pepper or red bell pepper. In particular, the plant belonging to the genus *Solanum* may be, for example, tomato. The plants belonging to the family *Solanaceae* may be used singly, or in combination of two or more kinds thereof.

The form of usage of the plant belonging to the family *Solanaceae* is not particularly limited, as long as the active ingredient can be produced. As the plant belonging to the family *Solanaceae,* for example, a fresh product (specifically, a fresh product such as the edible portion of a fruit, or the like) may be used as it is, or after being subjected to processing, as appropriate, in the culture. Examples of the processing include cutting, crushing, straining, juice extraction, fractionation, dilution, concentration, drying and heating. Such processing can be performed singly, or in an appropriate combination. For example, skin and/or seeds may be removed by such processing. The processed product of a plant belonging to the family *Solanaceae* may be, for example, the juice, puree or paste of the plant belonging to the family *Solanaceae.* That is, examples of the processed product of tomato include tomato juice, tomato puree and tomato paste. In particular, the processed product of a plant belonging to the family *Solanaceae* may be, for example, the paste of the plant belonging to the family *Solanaceae,* such as tomato paste. The "juice" of a plant belonging to the family *Solanaceae,* such as tomato juice, may refer, for example, to one obtained by crushing the edible portion of a fruit or the like, followed by juice extraction or straining, in which the salt-free soluble solid content is less than 8% (w/w) (for example, 4.5% (w/w) or more and less than 24% (w/w)). The "puree" of a plant belonging to the family *Solanaceae,* such as tomato puree, may refer, for example, to a concentrate of the juice of the plant belonging to the family *Solanaceae,* in which the salt-free soluble solid content is 8% (w/w) or more and less than 24% (w/w). The "paste" of a plant belonging to the family *Solanaceae,* such as tomato paste, may refer, for example, to a concentrate of the juice of the plant belonging to the family *Solanaceae,* in which the salt-free soluble solid content is 24% (w/w) or more. Any additive such as sodium chloride may but need not be added to the processed product of a plant belonging to the family *Solanaceae.* The processed product of a plant belonging to the family *Solanaceae* may be a juice from concentrate adjusted so as to achieve the salt-free soluble solid content exemplified above.

The carbon source may be, for example a saccharide. Specific examples of the saccharide include glucose, fructose, galactose, xylose, arabinose, sucrose, lactose, cellobiose, molasses, starch hydrolysates, and biomass hydrolysates. The carbon sources may be used singly, or in combination of two or more kinds thereof.

Specific examples of the nitrogen source include: ammonium salts such as ammonium sulfate, ammonium chloride and ammonium phosphate; organic nitrogen sources such as peptone, casein peptone, soy peptone, potato peptone, wheat peptone, casamino acids, yeast extracts, malt extracts, meat extracts, corn steep liquor and soy protein degradation products; ammonia; and urea. The nitrogen sources may be used singly, or in combination of two or more kinds thereof.

Specific examples of the phosphate source include: phosphate salts such as potassium dihydrogen phosphate and dipotassium hydrogen phosphate, and phosphate polymers such as pyrophosphate. The phosphate sources may be used singly, or in combination of two or more kinds thereof.

Specific examples of the sulfur source include: inorganic sulfur compounds such as sulfate salts, thiosulfate salts and sulfite salts; and sulfur-containing amino acids such as cysteine, cystine and glutathione. The sulfur sources may be used singly, or in combination of two or more kinds thereof.

Specific examples of various types of other organic components and inorganic components include: inorganic salts such as sodium chloride and potassium chloride; trace metals such as iron, manganese, magnesium and calcium; vitamins such as vitamin B1, vitamin B2, vitamin B6, nicotinic acid, nicotinic acid amide and vitamin B 12; amino acids such as phenylalanine; and nucleic acids; as well as organic components such as peptone, casamino acids, yeast extracts, malt extracts, meat extracts, corn steep liquor and soy protein degradation products s, containing any of the components described above. Various types of other organic components and inorganic components may be used singly, or in combination of two or more kinds thereof.

For example, the culture medium may contain, at least phenylalanine. For example, the culture medium may contain at least sodium chloride. Alternatively, for example, the culture medium may contain at least glucose. That is, the culture medium contains a plant belonging to the family *Solanaceae,* and may further contain phenylalanine and/or sodium chloride. Alternatively, the culture medium contains a plant belonging to the family *Solanaceae,* and may further contain phenylalanine, sodium chloride and/or glucose. In particular, the culture medium may contain a plant belonging to the family *Solanaceae,* phenylalanine and sodium chloride. Alternatively, in particular, the culture medium may contain a plant belonging to the family *Solanaceae,* phenylalanine, sodium chloride and glucose.

Phenylalanine can be effective in producing, for example, a composition having a high flavor-improving function (such as a high tomato-like flavor-improving function, a high spicy flavor-improving function and/or a high matured flavor-improving function). That is, when the culture medium contains phenylalanine, an active ingredient having a higher flavor-improving function (such as a higher tomato-like flavor-improving function, a higher spicy flavor-improving function and/or a higher matured flavor-improving function) may be obtained, as compared to the case in which the culture medium does not contain phenylalanine. Further, phenylalanine can be effective in generating, for example, a component (2-PE, in particular) having the flavor-improving function (Davide Ravasio et al., An indirect assay for volatile compound production in yeast strains, Sci Rep. 2014; 4: 3707.). That is, when the culture medium contains phenylalanine, an active ingredient with a higher content of a component (2-PE, in particular) having the flavor-improving function may be obtained, as compared to the case in which the culture medium does not contain phenylalanine. Phenylalanine may be D-phenylalanine, L-phenylalanine, or a combination thereof, unless otherwise specified. The ratio of D-phenylalanine and L-phenylalanine in the combination is not particularly limited. The ratio of D-phenylalanine or L-phenylalanine in the combination may be, for example, from 20 to 80%, from 30 to 70%, from 40 to 60%, or from 45 to 55%, in molar ratio. In particular, phenylalanine may be L-phenylalanine. In cases where D-phenylalanine or L-phenylalanine is selected, the use of D-phenylalanine or L-phenylalanine as the culture medium component is sufficient; however, it does not preclude the further use of L-phenylalanine or D-phenylalanine in combination.

A commercially available phenylalanine, or phenylalanine obtained by production, as appropriate, may be used as the phenylalanine. The method of producing phenylalanine is not particularly limited. Phenylalanine can be produced, for example, by a chemical synthesis, an enzyme reaction, fermentation, extraction, or any combination thereof. Specifically, phenylalanine can be produced, for example, by culturing a microorganism having a phenylalanine-producing capacity, and recovering phenylalanine from the culture liquid or bacterial cells. Phenylalanine may but need not be purified to a desired degree. That is, a purified phenylalanine, or a material containing phenylalanine, may be used as the phenylalanine. The material containing phenylalanine may be, for example, a fermented product such as the culture liquid, bacterial cells or culture supernatant obtained by culturing a microorganism having a phenylalanine-producing capacity; or a processed product thereof. The processed product may be, for example, a processed product obtained by subjecting a material, such as the fermented product described above, to a treatment such as concentration, dilution, drying, fractionation, extraction, purification or the like. For example, a material in which the content of phenylalanine is 1% (w/w) or more, 5% (w/w) or more, 10% (w/w) or more, 30% (w/w) or more, 50% (w/w) or more, 70% (w/w) or more, 90% (w/w) or more, or 95% (w/w) or more, may be used as the phenylalanine.

For each of the other culture medium components, as well, a purified component, or a material containing the component may be used.

In cases where a culture medium component is capable of forming a salt, the culture medium component may be used in a free-form, as a salt thereof, or as a combination thereof. That is, the term "culture medium component" may refer to a free-form culture medium component or a salt of the culture medium component, or a combination thereof, unless otherwise specified. The term "free-form" refers to the form of a component which is not forming a salt. In cases where a culture medium component is capable of forming a hydrate, the culture medium component may be used as a non-hydrate thereof, as a hydrate thereof, or as a combination thereof. That is, the definition of the term "culture medium component" (such as the "free-form culture medium component" or the "salt of the culture medium component") may include a non-hydrate thereof and a hydrate thereof, unless otherwise specified. The culture medium component may be in any form, such as in the form of ions, upon use.

The salt is not particularly limited, as long as the flavor-improving effect is not lost (namely, as long as the flavor-improving effect by the active ingredient can be obtained). In particular, a salt which can be orally ingested may be used as the salt. Specific examples of salts of acidic groups such as carboxyl group include: ammonium salts; salts with alkali metals such as sodium and potassium; salts with alkaline earth metals such as calcium and magnesium; aluminum salts; zinc salts; salts with organic amines such as triethylamine, ethanolamine, morpholine, pyrrolidine, piperidine, piperazine and dicyclohexylamine; and salts with basic amino acids such as arginine and lysine. Further, specific examples of salts of basic groups such as amino group include: salts with inorganic acids such as hydrochloric acid, sulfuric acid, phosphoric acid, nitric acid and hydrobromic acid; salts with organic carboxylic acids such as acetic acid, citric acid, benzoic acid, maleic acid, fumaric acid, tartaric acid, succinic acid, tannic acid, butyric acid, hybenzic acid, pamoic acid, enanthic acid, decanic acid, teoclic acid, salicylic acid, lactic acid, oxalic acid, mandelic acid, malic acid, methylmalonic acid and adipic acid; and salts with organic sulfonic acids such as methanesulfonic acid, benzenesulfonic acid and p-toluene sulfonic acid. The salts may be used singly, or in combination of two or more kinds thereof.

In the case of using a material containing a culture medium component, the amount (such as the content (concentration) or the amount to be used) of the culture medium component is calculated based on the amount of the culture medium component itself in the material, unless otherwise specified. In cases where a culture medium component is forming a salt or a hydrate, the amount (such as the content (concentration) or the amount to be used) of the culture medium component is calculated based on the value obtained by converting the mass of the salt or the hydrate to the mass of an equimolar amount of a free-form non-hydrate, unless otherwise specified.

The culture may be carried out, for example, under aerobic conditions. In cases where the culture is carried out under aerobic conditions, the dissolved oxygen concentration of the culture medium may be controlled, for example, within the range of from 5 to 50%, and preferably from 10 to 20%, as the ratio in which the saturated dissolved oxygen concentration is taken as 100%. Specifically, the culture under aerobic conditions may be carried out, for example, by stationary culture, aeration culture, shaking culture, spinner culture, or any combination thereof. The culture may be carried out at a culture temperature of, for example, from 25 to 35°C, preferably from 27°C to 33°C, and more preferably from 28°C to 32°C. The culture may be carried out for a culture period of, for example, from 10 hours to 200 hours, and preferably from 15 hours to 120 hours. The culture medium may have a pH of, for example, from 3 to 10, and preferably from 3 to 8. If necessary, the pH of the culture medium may be adjusted during the culture. An inorganic or organic, acidic or alkaline substance, such as ammonia gas or the like, can be used for adjusting the pH. The culture may be carried out, for example, by batch culture, fed-batch culture, continuous culture, or any combination thereof. The culture medium to be used at the start of the culture is also referred to as "initial culture medium". The culture medium to be supplied to the culture system (fermentation vessel) in the fed-batch culture or continuous culture is also referred to as "fed-batch culture medium". Supplying the fed-batch culture medium to the culture system in the fed-batch culture or continuous is also referred to as "batch feeding". Further, the culture may be divided into pre-culture and main culture, and carried out separately. For example, the pre-culture may be carried out on a solid culture medium such as an agar culture medium, and the main culture may be carried out in a liquid culture medium. In cases where the culture is divided into seed culture and main culture, and carried out separately, the expression "the culture medium contains a certain component" means that the certain component is contained in the culture medium, at least in the main culture. That is, for example, a plant belonging to the family *Solanaceae* is contained in the culture medium, at least in the main culture. The culture may be continued, for example, until a component having the flavor-improving function is generated to a desired degree.

The contents of the culture medium components, such as the plant belonging to the family *Solanaceae,* phenylalanine, sodium chloride and glucose, in the culture medium, are not particularly limited, as long as the active ingredient can be produced.

The content of the plant belonging to the family *Solanaceae* in the culture medium may be, for example, 0.5% (w/w) or more, 1% (w/w) or more, 2% (w/w) or more, 3% (w/w) or more, 4% (w/w) or more, 5% (w/w) or more, 7% (w/w) or more, 10% (w/w) or more, 15% (w/w) or more, 20% (w/w) or more, 25% (w/w) or more, 30% (w/w) or more, or 40% (w/w) or more; may be 50% (w/w) or less, 40% (w/w) or less, 30% (w/w) or less, 25% (w/w) or less, 20% (w/w) or less, 15% (w/w) or less, 10% (w/w) or less, 7% (w/w) or less, 5% (w/w) or less, 4% (w/w) or less, 3% (w/w) or less, 2% (w/w) or less, or 1% (w/w) or less; or may be any combination that does not contradict with the above-described ranges. Specifically, the content of the plant belonging to the family *Solanaceae* in the culture medium may be, for example, from 0.5 to 1% (w/w), from 1 to 2% (w/w), from 2 to 3% (w/w), from 3 to 4% (w/w), from 4 to 5% (w/w), from 5 to 7% (w/w), from 7 to 10% (w/w), from 10 to 15% (w/w), from 15 to 20% (w/w), from 20 to 25% (w/w), from 25 to 30% (w/w), from 30 to 40% (w/w), or from 40 to 50% (w/w). Specifically, the content of the plant belonging to the family *Solanaceae* in the culture medium may be, for example, from 0.5 to 50% (w/w), from 1 to 30% (w/w), or from 2 to 10% (w/w). In the case of the paste of a plant belonging to the family *Solanaceae,* such as tomato paste, the content thereof in the culture medium may specifically be, for example, from 0.5 to 15% (w/w), from 1 to 10% (w/w), or from 2 to 7% (w/w).

The content of the plant belonging to the family *Solanaceae* in the culture medium, in terms of salt-free soluble solid content, may be, for example, 0.1% (w/w) or more, 0.2% (w/w) or more, 0.5% (w/w) or more, 1% (w/w) or more, 1.5% (w/w) or more, 2% (w/w) or more, 2.5% (w/w) or more, 3% (w/w) or more, 3.5% (w/w) or more, 4% (w/w) or more, 4.5% (w/w) or more, 5% (w/w) or more, 6% (w/w) or more, 7% (w/w) or more, 8% (w/w) or more, or 9% (w/w) or more; may be 10% (w/w) or less, 9% (w/w) or less, 8% (w/w) or less, 7% (w/w) or less, 6% (w/w) or less, 5% (w/w) or less, 4.5% (w/w) or less, 4% (w/w) or less, 3.5% (w/w) or less, 3% (w/w) or less, 2.5% (w/w) or less, 2% (w/w) or less, 1.5% (w/w) or less, 1% (w/w) or less, 0.5% (w/w) or less, or 0.2% (w/w) or less; or may be any combination that does not contradict with the above-described ranges. Specifically, the content of the plant belonging to the family *Solanaceae* in the culture medium, in terms of salt-free soluble solid content, may be, for example, from 0.1 to 0.2% (w/w), from 0.2 to 0.5% (w/w), from 0.5 to 1% (w/w), from 1 to 1.5% (w/w), from 1.5 to 2% (w/w), from 2 to 2.5% (w/w), from 2.5 to 3% (w/w), from 3 to 3.5% (w/w), from 3.5 to 4% (w/w), from 4 to 4.5% (w/w), from 4.5 to 5% (w/w), from 5 to 6% (w/w), from 6 to 7% (w/w), from 7 to 8% (w/w), from 8 to 9% (w/w), or from 9 to 10% (w/w). Specifically, the content of the plant belonging to the family *Solanaceae* in the culture medium, in terms of salt-free soluble solid content, may be, for example, from 0.1 to 10% (w/w), from 0.2 to 7% (w/w), or from 0.5 to 5% (w/w).

The content of the plant belonging to the family *Solanaceae* in the culture medium, in terms of the total nitrogen content, may be, for example, 0.005% (w/w) or more, 0.01% (w/w) or more, 0.02% (w/w) or more, 0.05% (w/w) or more, 0.1% (w/w) or more, 0.2% (w/w) or more, 0.4% (w/w) or more, or 0.7% (w/w) or more; may be 1% (w/w) or less, 0.7% (w/w) or less, 0.4% (w/w) or less, 0.2% (w/w) or less, 0.1% (w/w) or less, 0.05% (w/w) or less, 0.02% (w/w) or less, or 0.01% (w/w) or less; or may be any combination that does not contradict with the above-described ranges. Specifically, the content of the plant belonging to the family *Solanaceae* in the culture medium, in terms of the total nitrogen content, may be, for example, from 0.005 to 0.01% (w/w), from 0.01 to 0.02% (w/w), from 0.02 to 0.05% (w/w), from 0.05 to 0.1% (w/w), from 0.1 to 0.2% (w/w), from 0.2 to 0.4% (w/w), from 0.4 to 0.7% (w/w), or from 0.7 to 1% (w/w). Specifically, the content of the plant belonging to the family *Solanaceae* in the culture medium, in terms of the total nitrogen content, may be, for example, from 0.005 to 1% (w/w), from 0.005 to 0.4% (w/w), or from 0.01 to 0.4% (w/w).

The content of phenylalanine in the culture medium may be, for example, 0.02% (w/w) or more, 0.04% (w/w) or more, 0.06% (w/w) or more, 0.08% (w/w) or more, 0.1% (w/w) or more, 0.12% (w/w) or more, 0.15% (w/w) or more, 0.2% (w/w) or more, 0.25% (w/w) or more, 0.3% (w/w) or more, 0.35% (w/w) or more, 0.4% (w/w) or more, 0.5% (w/w) or more, 0.6% (w/w) or more, 0.7% (w/w) or more, 0.8% (w/w) or more, 0.9% (w/w) or more, 1% (w/w) or more, 1.2% (w/w) or more, or 1.5% (w/w) or more; may be 2% (w/w) or less, 1.5% (w/w) or less, 1.2% (w/w) or less, 1% (w/w) or less, 0.9% (w/w) or less, 0.8% (w/w) or less, 0.7% (w/w) or less, 0.6% (w/w) or less, 0.5% (w/w) or less, 0.4% (w/w) or less, 0.35% (w/w) or less, 0.3% (w/w) or less, 0.25% (w/w) or less, 0.2% (w/w) or less, 0.15% (w/w) or less, 0.12% (w/w) or less, 0.1% (w/w) or less, 0.08% (w/w) or less, 0.06% (w/w) or less, or 0.04% (w/w) or less; or may be any combination that does not contradict with the above-described ranges. Specifically, the content of phenylalanine in the culture medium may be, for example, from 0.02 to 0.04% (w/w), from 0.04 to 0.06% (w/w), from 0.06 to 0.08% (w/w), from 0.08 to 0.1% (w/w), from 0.1 to 0.12% (w/w), from 0.12 to 0.15% (w/w), from 0.15 to 0.2% (w/w), from 0.2 to 0.25% (w/w), from 0.25 to 0.3% (w/w), from 0.3 to 0.35% (w/w), from 0.35 to 0.4% (w/w), from 0.4 to 0.5% (w/w), from 0.5 to 0.6% (w/w), from 0.6 to 0.7% (w/w), from 0.7 to 0.8% (w/w), from 0.8 to 0.9% (w/w), from 0.9 to 1% (w/w), from 1 to 1.2% (w/w), from 1.2 to 1.5% (w/w), or from 1.5 to 2% (w/w). Specifically, the content of phenylalanine in the culture medium may be, for example, from 0.02 to 2% (w/w), from 0.02 to 1.5% (w/w), from 0.02 to 1% (w/w), from 0.02 to 0.5% (w/w), from 0.04 to 0.4% (w/w), or from 0.06 to 0.3% (w/w).

The content of sodium chloride in the culture medium may be, for example, 0% (w/w) or more, 0.1% (w/w) or more, 0.2% (w/w) or more, 0.5% (w/w) or more, 1% (w/w) or more, 2% (w/w) or more, 3% (w/w) or more, 4% (w/w) or more, 5% (w/w) or more, 6% (w/w) or more, 7% (w/w) or more, 8% (w/w) or more, 9% (w/w) or more, 10% (w/w) or more, 12% (w/w) or more, or 15% (w/w) or more; may be 20% (w/w) or less, 15% (w/w) or less, 12% (w/w) or less, 10% (w/w) or less, 9% (w/w) or less, 8% (w/w) or less, 7% (w/w) or less, 6% (w/w) or less, 5% (w/w) or less, 4% (w/w) or less, 3% (w/w) or less, 2% (w/w) or less, 1% (w/w) or less, 0.5% (w/w) or less, 0.2% (w/w) or less, or 0.1% (w/w) or less; or may be any combination that does not contradict with the above-described ranges. Specifically, the content of sodium chloride in the culture medium may be, for example, from 0 to 0.1% (w/w), from 0.1 to 0.2% (w/w), from 0.2 to 0.5% (w/w), from 0.5 to 1% (w/w), from 1 to 2% (w/w), from 2 to 3% (w/w), from 3 to 4% (w/w), from 4 to 5% (w/w), from 5 to 6% (w/w), from 6 to 7% (w/w), from 7 to 8% (w/w), from 8 to 9% (w/w), from 9 to 10% (w/w), from 10 to 12% (w/w), from 12 to 15% (w/w), or from 15 to 20% (w/w). Specifically, the content of sodium chloride in the culture medium may be, for example, from 0 to 20% (w/w), from 0.1 to 20% (w/w), from 1 to 20% (w/w), from 5 to 20% (w/w), from 5 to 15% (w/w), or from 5 to 12% (w/w). The definition of the expression "0% (w/w) or more" regarding the content of sodium chloride in the culture medium, includes both the case in which the culture medium does not contain sodium chloride (namely, the content is 0% (w/w)), and the case in which the culture medium contains sodium chloride (namely, the content is more than 0% (w/w)). Accordingly, for example, the expression "the content of sodium chloride in the culture medium is from 0 to 20% (w/w)" means either that the culture medium does not contain sodium chloride, or that the culture medium contains sodium chloride in a content of 20% (w/w) or less (specifically, in a content of more than 0% (w/w) and 20% (w/w) or less).

The content of the carbon source, such as glucose, in the culture medium may be, for example, 1% (w/w) or more, 3% (w/w) or more, 5% (w/w) or more, 10% (w/w) or more, 15% (w/w) or more, or 20% (w/w) or more; may be 30% (w/w) or less, 20% (w/w) or less, 15% (w/w) or less, 10% (w/w) or less, 5% (w/w) or less, or 3% (w/w) or less; or may be any combination that does not contradict with the above-described ranges. Specifically, the content of the carbon source, such as glucose, in the culture medium may be, for example, from 1 to 3% (w/w), from 3 to 5% (w/w), from 5 to 10% (w/w), from 10 to 15% (w/w), from 15 to 20% (w/w), or from 20 to 30% (w/w). Specifically, the content of the carbon source, such as glucose, in the culture medium may be, for example, from 1 to 30% (w/w), from 3 to 20% (w/w), or from 5 to 15% (w/w).

Each of the culture medium components, such as the plant belonging to the family *Solanaceae,* phenylalanine, sodium chloride and glucose, may be contained in the culture medium throughout the entire period of the culture, or may be contained in the culture medium only in a partial period of the culture. That is, the expression "the culture is carried out in a culture medium containing a certain component" means that it is sufficient if the certain component is contained in the culture medium at least in a partial period of the culture, and the certain component need not be contained in the culture medium throughout the entire period of the culture. Each of the culture medium components, such as the plant belonging to the family *Solanaceae,* phenylalanine, sodium chloride and glucose, may be, for example, contained in the culture medium at the start of the culture, or supplied to the culture medium after the start of the culture. Alternatively, each of the culture medium components, such as the plant belonging to the family *Solanaceae,* phenylalanine, sodium chloride and glucose, may be, for example, contained in the culture medium at the start of the culture, and further supplied to the culture medium after the start of the culture (for example, after the above-mentioned certain component is consumed).

Each of the culture medium components, such as the plant belonging to the family *Solanaceae,* phenylalanine, sodium chloride and glucose, may be, for example, contained in the culture medium at the above-exemplified concentration throughout the entire period of the culture, or contained in the culture medium at the above-exemplified concentration only in a partial period of the culture. That is, the expression "the culture is carried out in a culture medium containing a certain component at a certain concentration" means that it is sufficient if the concentration of the certain component in the culture medium is within the certain concentration at least in a partial period of the culture, and the concentration of the certain component in the culture medium need not be within the certain concentration range throughout the entire period of the culture. Each of the culture medium components, such as the plant belonging to the family *Solanaceae,* phenylalanine, sodium chloride and glucose, may be, for example, contained in the culture medium at the above-exemplified concentration at the start of the culture, or supplied to the culture medium after the start of the culture so as to achieve the above-exemplified concentration. Alternatively, each of the culture medium components, such as the plant belonging to the family *Solanaceae,* phenylalanine, sodium chloride and glucose, may be, for example, contained in the culture medium at the above-exemplified concentration at the start of the culture, and further supplied to the culture medium after the start of the culture (for example, after the above-mentioned certain component is consumed), so as to achieve the above-exemplified concentration.

The length of the "partial period of the culture" is not particularly limited, as long as the active ingredient can be produced. The length of the "partial period of the culture" can be set as appropriate, depending on various conditions, such as the types of the culture medium components, the type of the yeast, the length of the culture period and the desired amount(s) to be generated of the component(s) having the flavor-improving function. The "partial period" may be, for example, a period of 10% or more, 20% or more, 30% or more, 40% or more, 50% or more, 60% or more, 70% or more, 80% or more, 90% or more, 95% or more, 97% or more, or 99% or more, of the entire period of the culture. Further, the "partial period" may be, for example, a period of 10 hours or more, 20 hours or more, 40 hours or more, 60 hours or more, 80 hours or more, 100 hours or more, 120 hours or more, or 150 hours or more. In cases where the culture is divided into seed culture and main culture, and carried out separately, the "entire period of the culture" refers to the entire period of the main culture.

The content of each culture medium component, such as phenylalanine, in the culture medium, can be measured, for example, by a known method used for detecting or identifying a compound. Examples of such a method include HPLC, UPLC, LC/MS, GC/MS and NMR. These methods can also be used for confirming whether any component having the flavor-improving function has been generated or not. These methods can be used singly, or in an appropriate combination.

A cultured product (namely, the active ingredient) is obtained, by culturing the yeast in such a manner.

The content of 2-PE in the cultured product at the completion of the culture may be, for example, 100 ppm (w/w) or more, 200 ppm (w/w) or more, 300 ppm (w/w) or more, 400 ppm (w/w) or more, 500 ppm (w/w) or more, 600 ppm (w/w) or more, 700 ppm (w/w) or more, 800 ppm (w/w) or more, 900 ppm (w/w) or more, 1,000 ppm (w/w) or more, 1,200 ppm (w/w) or more, 1,500 ppm (w/w) or more, or 2,000 ppm (w/w) or more. The content of 2-PE in the cultured product at the completion of the culture may be, for example, 8,000 ppm (w/w) or less, 5,000 ppm (w/w) or less, or 3,000 ppm (w/w) or less.

The content of ethyl acetate in the cultured product at the completion of the culture may be, for example, 1 ppm (w/w) or more, 2 ppm (w/w) or more, 3 ppm (w/w) or more, 4 ppm (w/w) or more, 5 ppm (w/w) or more, 6 ppm (w/w) or more, 7 ppm (w/w) or more, 8 ppm (w/w) or more, 9 ppm (w/w) or more, 10 ppm (w/w) or more, 12 ppm (w/w) or more, 15 ppm (w/w) or more, or 20 ppm (w/w) or more. The content of ethyl acetate in the cultured product at the completion of the culture may be, for example, 80 ppm (w/w) or less, 50 ppm (w/w) or less, or 30 ppm (w/w) or less.

The content of diacetyl in the cultured product at the completion of the culture may be, for example, 1 ppm (w/w) or more, 2 ppm (w/w) or more, 3 ppm (w/w) or more, 4 ppm (w/w) or more, 5 ppm (w/w) or more, 6 ppm (w/w) or more, 7 ppm (w/w) or more, 8 ppm (w/w) or more, 9 ppm (w/w) or more, 10 ppm (w/w) or more, 12 ppm (w/w) or more, 15 ppm (w/w) or more, or 20 ppm (w/w) or more. The content of diacetyl in the cultured product at the completion of the culture may be, for example, 80 ppm (w/w) or less, 50 ppm (w/w) or less, or 30 ppm (w/w) or less.

The content of yeast bacterial cells in the cultured product at the completion of the culture may be, for example, 1 × 10³ CFU/g or more, 1 × 10⁴ CFU/g or more, 1 × 10⁵ CFU/g or more, 1 × 10⁶ CFU/g or more, or 1 × 10⁷ CFU/g or more. The content of yeast bacterial cells in the cultured product at the completion of the culture may be, for example, 1 × 10¹⁰ CFU/g or less, 1 × 10⁹ CFU/g or less, 1 × 10⁸ CFU/g or less, 1 × 10⁷ CFU/g or less, or 1 × 10⁶ CFU/g or less.

The cultured product can be used as the active ingredient as it is, or after being subjected to a treatment, as appropriate. That is, the method of producing the active ingredient may include a step of subjecting the cultured product to a treatment. For example, the cultured product can be used as the active ingredient as it is, or after being subjected to a treatment, as appropriate, in the production of a composition according to the present invention. Alternatively, for example, the cultured product can be used as the active ingredient as it is, or after being subjected to a treatment, as appropriate, in a method according to the present invention. The treatment is not particularly limited, as long as the flavor-improving effect is not lost (namely, as long as the flavor-improving effect by the active ingredient can be obtained). Examples of the treatment include fractionation, dilution, concentration, drying, heating and sterilization. In the case of fractionation, for example, a fraction having the flavor-improving function can be collected. The fraction to be collected by the fractionation may specifically be a fraction containing a component having the flavor-improving function. The form of the active ingredient is not particularly limited. For example, the active ingredient may be in an arbitrary form, such as in the form of a powder, flakes, a paste, a liquid or the like. That is, the cultured product may be processed into a desired form. In other words, the method of producing the active ingredient may include a step of processing the active ingredient into a desired form. For example, the cultured product may be dried and powdered to be used as the active ingredient. The method of producing the active ingredient may include, for example, a step of drying and powdering the active ingredient. The drying can be carried out, for example, by a drying means known as a means for drying food, such as freeze drying, spray drying, drum drying or vacuum drum drying. The active ingredient may contain an additive such as a vehicle. The vehicle may be, for example, starch or dextrin.

The cultured product preferably contains bacterial cells of the yeast, and more preferably contains both the bacterial cells of the yeast, and a component (namely, the culture supernatant) other than the bacterial cells of the yeast contained in the cultured product. Preferred embodiments include: drying and powdering the cultured product at the completion of the culture as it is, or after being concentrated, and using the resulting powder as the active ingredient; crushing the yeast bacterial cells in the cultured product or in the concentrate of the cultured product by a known method, followed by drying and powdering, and using the resulting powder as the active ingredient; and separating the cultured product into the supernatant and precipitates by a centrifugal operation or the like, followed by drying and powdering both the supernatant and precipitates, mixing the dried supernatant and the dried precipitates, and using the resulting mixture as the active ingredient.

### 2. Composition According to Present Invention

The composition according to the present invention is a composition containing an active ingredient (namely, a yeast-fermented product of a plant belonging to the family *Solanaceae).*

The active ingredient may contain a component having the flavor-improving function (such as the tomato-like flavor-improving function, the spicy flavor-improving function and/or the matured flavor-improving function). Accordingly, the composition according to the present invention may contain a component having the flavor-improving function. That is, the composition according to the present invention may be, for example, a composition containing a component having the flavor-improving function. For example, the composition according to the present invention may contain at least 2-PE. For example, the composition according to the present invention may contain 2-PE, and may further contain ethyl acetate and/or diacetyl. In particular, the composition according to the present invention may contain 2-PE, ethyl acetate, and diacetyl.

The use of the composition according to the present invention makes it possible to improve a flavor of food (for example, to improve the tomato-like flavor, the spicy flavor and/or the matured flavor, in the food); namely, the use of the present composition makes it possible to obtain the flavor-improving effect (such as the tomato-like-flavor-improving effect, the spicy flavor-improving effect and/or the matured flavor-improving effect). In other words, the composition according to the present invention has the flavor-improving function (such as the tomato-like flavor-improving function, the spicy flavor-improving function and/or the matured flavor-improving function). Accordingly, the composition according to the present invention may be used for improving a flavor of food (for example, improving the tomato-like flavor, the spicy flavor and/or the matured flavor, in the food). That is, the composition according to the present invention may be, for example, a composition for improving a flavor of food (for example, a composition for improving the tomato-like flavor, the spicy flavor and/or the matured flavor, in the food).

Further, the use of the composition according to the present invention makes it possible to produce food with an improved flavor (for example, food with an improved tomato-like flavor, spicy flavor and/or matured flavor). Accordingly, the composition according to the present invention may be used for producing food (specifically, for producing food with an improved flavor, such as food with an improved tomato-like flavor, spicy flavor and/or matured flavor). That is, the composition according to the present invention may be, for example, a composition for producing food (specifically, for producing food with an improved flavor, such as food with an improved tomato-like flavor, spicy flavor and/or matured flavor).

The composition according to the present invention may be, for example, a seasoning. Specifically, the composition according to the present invention may be, for example, a seasoning for improving a flavor of food (for example, a seasoning for improving the tomato-like flavor, the spicy flavor and/or the matured flavor, in the food), or may be a seasoning for producing food (specifically, for producing food with an improved flavor, such as food with an improved tomato-like flavor, spicy flavor and/or matured flavor).

The composition according to the present invention may but need not have an improved flavor by itself. In cases where the composition according to the present invention contains a spice, for example, the composition according to the present invention may have an enhanced spicy flavor (specifically, an enhanced spicy flavor as compared to the case in which the composition does not contain the active ingredient) by itself.

The composition according to the present invention may be used for improving a flavor of food or producing food, in embodiments described in the method according to the present invention to be described later.

The composition according to the present invention may be a composition composed of the active ingredient, or may contain a component other than the active ingredient. That is, the active ingredient may be used as the composition according to the present invention, as it is, or in combination with another component(s).

The component other than the active ingredient is not particularly limited, as long as the flavor-improving effect is not lost (namely, as long as the flavor-improving effect by the active ingredient can be obtained). The component other than the active ingredient can be selected as appropriate, depending on various conditions, such as the type of the food and the like. The component other than the active ingredient may be, for example a component to be incorporated into food or a pharmaceutical. The component other than the active ingredient may specifically be, for example, a raw material of the food to be described later. In particular, the component other than the active ingredient may be, for example, a spice. That is, the composition according to the present invention may be, for example, a composition containing the active ingredient and a spice (such as a seasoning).

The term "spice" may refer to leaves, stems, bark, roots, flowers, buds, seeds, fruits or fruit skin, of a plant, which is used for imparting a special flavor to food. The definition of the "spice" may include so-called "herbs". Examples of the spice include spices belonging to the family *Lauraceae* (such as Laurel and cinnamon), spices belonging to the family *Piperaceae* (such as black pepper, white pepper), spices belonging to the family *Lamiaceae* spice (such as thyme, sage, basil, oregano, marjoram, rosemary and mint), spices belonging to the family *Apiaceae* (such as fennel, caraway, cumin, coriander, parsley, Italian parsley, celery, celery seed and dill), spices belonging to the family *Solanaceae* (such as chili pepper and cayenne pepper), spices belonging to the family *Myristicaceae* (such as nutmeg and mace), spices obtained from the plants belonging to the family *Alliaceae* (such as garlic powder and onion powder), spices belonging to the family *Myrtaceae* (such as allspice and clove), spices belonging to the family *Schisandraceae* (such as star anise), spices belonging to the family *Fabaceae* (such as Fenugreek), spices belonging to the family *Polygonaceae* (such as knotweed), spices belonging to the family *Brassicaceae* (such as garden cress, wasabi and Japanese mustard), spices belonging to the family *Zingiberaceae* (such as cardamom, turmeric and ginger), and spices belonging to the family *Rutaceae* (such as Japanese pepper and Sichuan pepper). In particular, examples of the spice include cardamom, bay leaf, coriander, clove, nutmeg, mace, allspice, cinnamon, fennel, cumin, ginger, pepper, caraway, anise, basil, parsley, sage, thyme, oregano, rosemary, celery seed, mint, garden cress, dill, marjoram, knotweed, turmeric, lemongrass, chili pepper, Japanese pepper, garlic, shallot, onion, and celery. In one embodiment, in the present invention, garlic and onion used as fresh vegetables, for cocking in a raw state, may be excluded from the definition of the spice, according to the general practice. In the present invention, the spices may be used singly, or in combination of two or more kinds thereof.

The spice may contain an aroma component that contributes to the spicy flavor. Examples of the aroma component that contributes to the spicy flavor include linalyl acetate, α-terpinyl acetate, linalool, cineol, eugenol, limonene, α-terpineol, and α-terpinene.

The composition according to the present invention may be produced, for example, as a pharmaceutical formulation, as appropriate. In the production of the formulation, any additive may be used as appropriate. Examples of the additive include vehicles, binders, disintegrating agents, lubricants, stabilizers, flavor corrigents, diluents, surfactants and solvents. The additive can be selected as appropriate, for example, depending on various conditions, such as the form of the composition according to the present invention.

The form of the composition according to the present invention is not particularly limited. For example, the composition according to the present invention may be in an arbitrary form, such as in the form of a powder, flakes, tablets, a paste, a liquid, or the like.

The content or the content ratio of each component (namely, each of the active ingredient and any other optional components) in the composition according to the present invention is not particularly limited, as long as the flavor-improving effect can be obtained. The content or the content ratio of each component in the composition according to the present invention can be set as appropriate, depending on various conditions, such as the embodiment of use of the composition according to the present invention.

The content of the active ingredient (namely, a yeast-fermented product of a plant belonging to the family *Solanaceae)* in the composition according to the present invention, in terms of the content of the original cultured product, may be, for example, 1 × 10⁻¹²% (w/w) or more, 1 × 10⁻¹¹% (w/w) or more, 1 × 10⁻¹⁰% (w/w) or more, 1 × 10⁻⁹% (w/w) or more, 1 × 10⁻⁸% (w/w) or more, 1 × 10⁻⁷% (w/w) or more, 1 × 10⁻⁶% (w/w) or more, 1 × 10⁻⁵% (w/w) or more, 1 × 10⁻⁴% (w/w) or more, 1 × 10⁻³% (w/w) or more, 0.01% (w/w) or more, 0.1% (w/w) or more, 0.2% (w/w) or more, 0.5% (w/w) or more, 1% (w/w) or more, 2% (w/w) or more, 5% (w/w) or more, 10% (w/w) or more, 20% (w/w) or more, 50% (w/w) or more, 100% (w/w) or more, 200% (w/w) or more, 500% (w/w) or more, 1,000% (w/w) or more, 2,000% (w/w) or more, or 5,000% (w/w) or more; may be 10,000% (w/w) or less, 5,000% (w/w) or less, 2,000% (w/w) or less, 1,000% (w/w) or less, 500% (w/w) or less, 200% (w/w) or less, 100% (w/w) or less, 50% (w/w) or less, 20% (w/w) or less, 10% (w/w) or less, 5% (w/w) or less, 2% (w/w) or less, 1% (w/w) or less, 0.5% (w/w) or less, 0.2% (w/w) or less, 0.1% (w/w) or less, 0.01% (w/w) or less, 1 × 10⁻³% (w/w) or less, 1 × 10⁻⁴% (w/w) or less, 1 × 10⁻⁵% (w/w) or less, 1 × 10⁻⁶% (w/w) or less, 1 × 10⁻⁷% (w/w) or less, 1 × 10⁻⁸% (w/w) or less, 1 × 10⁻⁹% (w/w) or less, 1 × 10⁻¹⁰% (w/w) or less, or 1 × 10⁻¹¹% (w/w) or less; or may be any combination that does not contradict with the above-described ranges. Specifically, the content of the active ingredient in the composition according to the present invention, in terms of the content of the original cultured product, may be, for example, from 1 × 10⁻¹² to 1 × 10⁻¹¹% (w/w), from 1 × 10⁻¹¹ to 1 × 10⁻¹⁰% (w/w), from 1 × 10⁻¹⁰ to 1 × 10⁻⁹% (w/w), from 1 × 10⁻⁹ to 1 × 10⁻⁸% (w/w), from 1 × 10⁻⁸ to 1 × 10⁻⁷% (w/w), from 1 × 10⁻⁷ to 1 × 10⁻⁶% (w/w), from 1 × 10⁻⁶ to 1 × 10⁻⁵% (w/w), from 1 × 10⁻⁵ to 1 × 10⁻⁴% (w/w), from 1 × 10⁻⁴ to 1 × 10⁻³% (w/w), from 1 × 10⁻³ to 0.01% (w/w), from 0.01 to 0.1% (w/w), from 0.1 to 0.2% (w/w), from 0.2 to 0.5% (w/w), from 0.5 to 1% (w/w), from 1 to 2% (w/w), from 2 to 5% (w/w), from 5 to 10% (w/w), from 10 to 20% (w/w), from 20 to 50% (w/w), from 50 to 100% (w/w), from 100 to 200% (w/w), from 200 to 500% (w/w), from 500 to 1,000% (w/w), from 1,000 to 2,000% (w/w), from 2,000 to 5,000% (w/w), or from 5,000 to 10,000% (w/w). Specifically, the content of the active ingredient in the composition according to the present invention, in terms of the content of the original cultured product, may be, for example, from 1 × 10⁻¹² to 10,000% (w/w), from 1 × 10⁻¹¹ to 5,000% (w/w), or from 1 × 10⁻¹⁰ to 2,000% (w/w). The "original cultured product" refers to a cultured product in which no change in the concentration due to concentration, dilution or the like has occurred after the culture, and specifically, may refer to a cultured product at the completion of the culture. The "original cultured product" is also referred to as "original fermented product". When the content is more than 100% (w/w), it means that the cultured product is contained in the composition according to the present invention, in a concentrated state. That is, for example, when the content is 200% (w/w), it means that the cultured product is contained in the composition according to the present invention, in a state concentrated two-fold.

In cases where the active ingredient in the composition according to the present invention is a dry powder produced by the production method including a step of drying and powdering the active ingredient, in particular, the content of the active ingredient in the composition according to the present invention, in terms of the content of the dry powder, may be, for example, 1 × 10⁻¹²% (w/w) or more, 1 × 10⁻¹¹% (w/w) or more, 1 × 10⁻¹⁰% (w/w) or more, 1 × 10⁻⁹% (w/w) or more, 1 × 10⁻⁸% (w/w) or more, 1 × 10⁻⁷% (w/w) or more, 1 × 10⁻⁶% (w/w) or more, 1 × 10⁻⁵% (w/w) or more, 1 × 10⁻⁴% (w/w) or more, 1 × 10⁻³% (w/w) or more, 0.01% (w/w) or more, 0.1% (w/w) or more, 0.2% (w/w) or more, 0.5% (w/w) or more, 1% (w/w) or more, 2% (w/w) or more, 5% (w/w) or more, 10% (w/w) or more, 20% (w/w) or more, or 50% (w/w) or more; may be 100% (w/w) or less, 50% (w/w) or less, 20% (w/w) or less, 10% (w/w) or less, 5% (w/w) or less, 2% (w/w) or less, 1% (w/w) or less, 0.5% (w/w) or less, 0.2% (w/w) or less, 0.1% (w/w) or less, 0.01% (w/w) or less, 1 × 10⁻³% (w/w) or less, 1 × 10⁻⁴% (w/w) or less, 1 × 10⁻⁵% (w/w) or less, 1 × 10⁻⁶% (w/w) or less, 1 × 10⁻⁷% (w/w) or less, 1 × 10⁻⁸% (w/w) or less, 1 × 10⁻⁹% (w/w) or less, 1 × 10⁻¹⁰% (w/w) or less, or 1 × 10⁻¹¹% (w/w) or less; or may be any combination that does not contradict with the above-described ranges. Specifically, the content of the active ingredient in the composition according to the present invention, in terms of the content of the dry powder, may be, for example, from 1 × 10⁻¹² to 1 × 10⁻¹¹% (w/w), from 1 × 10⁻¹¹ to 1 × 10⁻¹⁰% (w/w), from 1 × 10⁻¹⁰ to 1 × 10⁻⁹% (w/w), from 1 × 10⁻⁹ to 1 × 10⁻⁸% (w/w), from 1 × 10⁻⁸ to 1 × 10⁻⁷% (w/w), from 1 × 10⁻⁷ to 1 × 10⁻⁶% (w/w), from 1 × 10⁻⁶ to 1 × 10⁻⁵% (w/w), from 1 × 10⁻⁵ to 1 × 10⁻⁴% (w/w), from 1 × 10⁻⁴ to 1 × 10⁻³% (w/w), from 1 × 10⁻³ to 0.01% (w/w), from 0.01 to 0.1% (w/w), from 0.1 to 0.2% (w/w), from 0.2 to 0.5% (w/w), from 0.5 to 1% (w/w), from 1 to 2% (w/w), from 2 to 5% (w/w), from 5 to 10% (w/w), from 10 to 20% (w/w), from 20 to 50% (w/w), or from 50 to 100% (w/w). Specifically, the content of the active ingredient in the composition according to the present invention, in terms of the content of the dry powder, may be, for example, from 1 × 10⁻¹² to 100% (w/w), from 1 × 10⁻¹¹ to 50% (w/w), or from 1 × 10⁻¹⁰ to 20% (w/w).

The content of the active ingredient in the composition according to the present invention, in terms of the content of 2-PE, may be, for example, 1 × 10⁻¹³ ppm (w/w) or more, 1 × 10⁻¹² ppm (w/w) or more, 1 × 10⁻¹¹ ppm (w/w) or more, 1 × 10⁻¹⁰ ppm (w/w) or more, 1 × 10⁻⁹ ppm (w/w) or more, 1 × 10⁻⁸ ppm (w/w) or more, 1 × 10⁻⁷ ppm (w/w) or more, 1 × 10⁻⁶ ppm (w/w) or more, 1 × 10⁻⁵ ppm (w/w) or more, 1 × 10⁻⁴ ppm (w/w) or more, 1 × 10⁻³ ppm (w/w) or more, 0.01 ppm (w/w) or more, 0.1 ppm (w/w) or more, 1 ppm (w/w) or more, 2 ppm (w/w) or more, 5 ppm (w/w) or more, 10 ppm (w/w) or more, 20 ppm (w/w) or more, 50 ppm (w/w) or more, 100 ppm (w/w) or more, 200 ppm (w/w) or more, 500 ppm (w/w) or more, 1,000 ppm (w/w) or more, 2,000 ppm (w/w) or more, 5,000 ppm (w/w) or more, 10,000 ppm (w/w) or more, 20,000 ppm (w/w) or more, or 50,000 ppm (w/w) or more; may be 100,000 ppm (w/w) or less, 50,000 ppm (w/w) or less, 20,000 ppm (w/w) or less, 10,000 ppm (w/w) or less, 5,000 ppm (w/w) or less, 2,000 ppm (w/w) or less, 1,000 ppm (w/w) or less, 500 ppm (w/w) or less, 200 ppm (w/w) or less, 100 ppm (w/w) or less, 50 ppm (w/w) or less, 20 ppm (w/w) or less, 10 ppm (w/w) or less, 5 ppm (w/w) or less, 2 ppm (w/w) or less, 1 ppm (w/w) or less, 0.1 ppm (w/w) or less, 0.01 ppm (w/w) or less, 1 × 10⁻³ ppm (w/w) or less, 1 × 10⁻⁴ ppm (w/w) or less, 1 × 10⁻⁵ ppm (w/w) or less, 1 × 10⁻⁶ ppm (w/w) or less, 1 × 10⁻⁷ ppm (w/w) or less, 1 × 10⁻⁸ ppm (w/w) or less, 1 × 10⁻⁹ ppm (w/w) or less, 1 × 10⁻¹⁰ ppm (w/w) or less, 1 × 10⁻¹¹ ppm (w/w) or less, or 1 × 10⁻¹² ppm (w/w) or less; or may be any combination that does not contradict with the above-described ranges. Specifically, the content of the active ingredient in the composition according to the present invention, in terms of the content of 2-PE, may be, for example, from 1 × 10⁻¹³ to 1 × 10⁻¹² ppm (w/w), from 1 × 10⁻¹² to 1 × 10⁻¹¹ ppm (w/w), from 1 × 10⁻¹¹ to 1 × 10⁻¹⁰ ppm (w/w), from 1 × 10⁻¹⁰ to 1 × 10⁻⁹ ppm (w/w), from 1 × 10⁻⁹ to 1 × 10⁻⁸ ppm (w/w), from 1 × 10⁻⁸ to 1 × 10⁻⁷ ppm (w/w), from 1 × 10⁻⁷ to 1 × 10⁻⁶ ppm (w/w), from 1 × 10⁻⁶ to 1 × 10⁻⁵ ppm (w/w), from 1 × 10⁻⁵ to 1 × 10⁻⁴ ppm (w/w), from 1 × 10⁻⁴ to 1 × 10⁻³ ppm (w/w), from 1 × 10⁻³ to 0.01 ppm (w/w), from 0.01 to 0.1 ppm (w/w), from 0.1 to 1 ppm (w/w), from 1 to 2 ppm (w/w), from 2 to 5 ppm (w/w), from 5 to 10 ppm (w/w), from 10 to 20 ppm (w/w), from 20 to 50 ppm (w/w), from 50 to 100 ppm (w/w), from 100 to 200 ppm (w/w), from 200 to 500 ppm (w/w), from 500 to 1,000 ppm (w/w), from 1,000 to 2,000 ppm (w/w), from 2,000 to 5,000 ppm (w/w), from 5,000 to 10,000 ppm (w/w), from 10,000 to 20,000 ppm (w/w), from 20,000 to 50,000 ppm (w/w), or from 50,000 to 100,000 ppm (w/w). Specifically, the content of the active ingredient in the composition according to the present invention, in terms of the content of 2-PE, may be, for example, from 1 × 10⁻¹³ to 100,000 ppm (w/w), from 1 × 10⁻¹² to 50,000 ppm (w/w), or from 1 × 10⁻¹¹ to 20,000 ppm (w/w).

The content of the active ingredient in the composition according to the present invention, in terms of the content of ethyl acetate, may be, for example, 1 × 10⁻¹³ ppm (w/w) or more, 1 × 10⁻¹² ppm (w/w) or more, 1 × 10⁻¹¹ ppm (w/w) or more, 1 × 10⁻¹⁰ ppm (w/w) or more, 1 × 10⁻⁹ ppm (w/w) or more, 1 × 10⁻⁸ ppm (w/w) or more, 1 × 10⁻⁷ ppm (w/w) or more, 1 × 10⁻⁶ ppm (w/w) or more, 1 × 10⁻⁵ ppm (w/w) or more, 1 × 10⁻⁴ ppm (w/w) or more, 1 × 10⁻³ ppm (w/w) or more, 0.01 ppm (w/w) or more, 0.02 ppm (w/w) or more, 0.05 ppm (w/w) or more, 0.1 ppm (w/w) or more, 0.2 ppm (w/w) or more, 0.5 ppm (w/w) or more, 1 ppm (w/w) or more, 2 ppm (w/w) or more, 5 ppm (w/w) or more, 10 ppm (w/w) or more, 20 ppm (w/w) or more, 50 ppm (w/w) or more, 100 ppm (w/w) or more, 200 ppm (w/w) or more, or 500 ppm (w/w) or more; may be 1,000 ppm (w/w) or less, 500 ppm (w/w) or less, 200 ppm (w/w) or less, 100 ppm (w/w) or less, 50 ppm (w/w) or less, 20 ppm (w/w) or less, 10 ppm (w/w) or less, 5 ppm (w/w) or less, 2 ppm (w/w) or less, 1 ppm (w/w) or less, 0.5 ppm (w/w) or less, 0.2 ppm (w/w) or less, 0.1 ppm (w/w) or less, 0.05 ppm (w/w) or less, 0.02 ppm (w/w) or less, 0.01 ppm (w/w) or less, 1 × 10⁻³ ppm (w/w) or less, 1 × 10⁻⁴ ppm (w/w) or less, 1 × 10⁻⁵ ppm (w/w) or less, 1 × 10⁻⁶ ppm (w/w) or less, 1 × 10⁻⁷ ppm (w/w) or less, 1 × 10⁻⁸ ppm (w/w) or less, 1 × 10⁻⁹ ppm (w/w) or less, 1 × 10⁻¹⁰ ppm (w/w) or less, 1 × 10⁻¹¹ ppm (w/w) or less, or 1 × 10⁻¹² ppm (w/w) or less; or may be any combination that does not contradict with the above-described ranges. Specifically, the content of the active ingredient in the composition according to the present invention, in terms of the content of ethyl acetate, may be, for example, from 1 × 10⁻¹³ to 1 × 10⁻¹² ppm (w/w), from 1 × 10⁻¹² to 1 × 10⁻¹¹ ppm (w/w), from 1 × 10⁻¹¹ to 1 × 10⁻¹⁰ ppm (w/w), from 1 × 10⁻¹⁰ to 1 × 10⁻⁹ ppm (w/w), from 1 × 10⁻⁹ to 1 × 10⁻⁸ ppm (w/w), from 1 × 10⁻⁸ to 1 × 10⁻⁷ ppm (w/w), from 1 × 10⁻⁷ to 1 × 10⁻⁶ ppm (w/w), from 1 × 10⁻⁶ to 1 × 10⁻⁵ ppm (w/w), from 1 × 10⁻⁵ to 1 × 10⁻⁴ ppm (w/w), from 1 × 10⁻⁴ to 1 × 10⁻³ ppm (w/w), from 1 × 10⁻³ to 0.01 ppm (w/w), from 0.01 to 0.02 ppm (w/w), from 0.02 to 0.05 ppm (w/w), from 0.05 to 0.1 ppm (w/w), from 0.1 to 0.2 ppm (w/w), from 0.2 to 0.5 ppm (w/w), from 0.5 to 1 ppm (w/w), from 1 to 2 ppm (w/w), from 2 to 5 ppm (w/w), from 5 to 10 ppm (w/w), from 10 to 20 ppm (w/w), from 20 to 50 ppm (w/w), from 50 to 100 ppm (w/w), from 100 to 200 ppm (w/w), from 200 to 500 ppm (w/w), or from 500 to 1,000 ppm (w/w). Specifically, the content of the active ingredient in the composition according to the present invention, in terms of the content of ethyl acetate, may be, for example, from 1 × 10⁻¹³ to 1000 ppm (w/w), from 1 × 10⁻¹² to 500 ppm (w/w), or from 1 × 10⁻¹¹ to 200 ppm (w/w).

The content of the active ingredient in the composition according to the present invention, in terms of the content of diacetyl, may be, for example, 1 × 10⁻¹³ ppm (w/w) or more, 1 × 10⁻¹² ppm (w/w) or more, 1 × 10⁻¹¹ ppm (w/w) or more, 1 × 10⁻¹⁰ ppm (w/w) or more, 1 × 10⁻⁹ ppm (w/w) or more, 1 × 10⁻⁸ ppm (w/w) or more, 1 × 10⁻⁷ ppm (w/w) or more, 1 × 10⁻⁶ ppm (w/w) or more, 1 × 10⁻⁵ ppm (w/w) or more, 1 × 10⁻⁴ ppm (w/w) or more, 1 × 10⁻³ ppm (w/w) or more, 0.01 ppm (w/w) or more, 0.02 ppm (w/w) or more, 0.05 ppm (w/w) or more, 0.1 ppm (w/w) or more, 0.2 ppm (w/w) or more, 0.5 ppm (w/w) or more, 1 ppm (w/w) or more, 2 ppm (w/w) or more, 5 ppm (w/w) or more, 10 ppm (w/w) or more, 20 ppm (w/w) or more, 50 ppm (w/w) or more, 100 ppm (w/w) or more, 200 ppm (w/w) or more, or 500 ppm (w/w) or more; may be 1,000 ppm (w/w) or less, 500 ppm (w/w) or less, 200 ppm (w/w) or less, 100 ppm (w/w) or less, 50 ppm (w/w) or less, 20 ppm (w/w) or less, 10 ppm (w/w) or less, 5 ppm (w/w) or less, 2 ppm (w/w) or less, 1 ppm (w/w) or less, 0.5 ppm (w/w) or less, 0.2 ppm (w/w) or less, 0.1 ppm (w/w) or less, 0.05 ppm (w/w) or less, 0.02 ppm (w/w) or less, 0.01 ppm (w/w) or less, 1 × 10⁻³ ppm (w/w) or less, 1 × 10⁻⁴ ppm (w/w) or less, 1 × 10⁻⁵ ppm (w/w) or less, 1 × 10⁻⁶ ppm (w/w) or less, 1 × 10⁻⁷ ppm (w/w) or less, 1 × 10⁻⁸ ppm (w/w) or less, 1 × 10⁻⁹ ppm (w/w) or less, 1 × 10⁻¹⁰ ppm (w/w) or less, 1 × 10⁻¹¹ ppm (w/w) or less, or 1 × 10⁻¹² ppm (w/w) or less; or may be any combination that does not contradict with the above-described ranges. Specifically, the content of the active ingredient in the composition according to the present invention, in terms of the content of diacetyl, may be, for example, from 1 × 10⁻¹³ to 1 × 10⁻¹² ppm (w/w), from 1 × 10⁻¹² to 1 × 10⁻¹¹ ppm (w/w), from 1 × 10⁻¹¹ to 1 × 10⁻¹⁰ ppm (w/w), from 1 × 10⁻¹⁰ to 1 × 10⁻⁹ ppm (w/w), from 1 × 10⁻⁹ to 1 × 10⁻⁸ ppm (w/w), from 1 × 10⁻⁸ to 1 × 10⁻⁷ ppm (w/w), from 1 × 10⁻⁷ to 1 × 10⁻⁶ ppm (w/w), from 1 × 10⁻⁶ to 1 × 10⁻⁵ ppm (w/w), from 1 × 10⁻⁵ to 1 × 10⁻⁴ ppm (w/w), from 1 × 10⁻⁴ to 1 × 10⁻³ ppm (w/w), from 1 × 10⁻³ to 0.01 ppm (w/w), from 0.01 to 0.02 ppm (w/w), from 0.02 to 0.05 ppm (w/w), from 0.05 to 0.1 ppm (w/w), from 0.1 to 0.2 ppm (w/w), from 0.2 to 0.5 ppm (w/w), from 0.5 to 1 ppm (w/w), from 1 to 2 ppm (w/w), from 2 to 5 ppm (w/w), from 5 to 10 ppm (w/w), from 10 to 20 ppm (w/w), from 20 to 50 ppm (w/w), from 50 to 100 ppm (w/w), from 100 to 200 ppm (w/w), from 200 to 500 ppm (w/w), or from 500 to 1,000 ppm (w/w). Specifically, the content of the active ingredient in the composition according to the present invention, in terms of the content of diacetyl, may be, for example, from 1 × 10⁻¹³ to 1,000 ppm (w/w), from 1 × 10⁻¹² to 500 ppm (w/w), or from 1 × 10⁻¹¹ to 200 ppm (w/w).

The content of the active ingredient in the composition according to the present invention, in terms of the content of yeast bacterial cells, may be, for example, 1 × 10⁻⁶ CFU/g or more, 1 × 10⁻⁵ CFU/g or more, 1 × 10⁻⁴ CFU/g or more, 1 × 10⁻³ CFU/g or more, 0.01 CFU/g or more, 0.1 CFU/g or more, 1 CFU/g or more, 10 CFU/g or more, 1 × 10² CFU/g or more, 1 × 10³ CFU/g or more, 1 × 10⁴ CFU/g or more, 1 × 10⁵ CFU/g or more, 1 × 10⁶ CFU/g or more, 1 × 10⁷ CFU/g or more, 1 × 10⁸ CFU/g or more, 1 × 10⁹ CFU/g or more, 1 × 10¹⁰ CFU/g or more, 1 × 10¹¹ CFU/g or more, or 1 × 10¹² CFU/g or more; may be 1 × 10¹³ CFU/g or less, 1 × 10¹² CFU/g or less, 1 × 10¹¹ CFU/g or less, 1 × 10¹⁰ CFU/g or less, 1 × 10⁹ CFU/g or less, 1 × 10⁸ CFU/g or less, 1 × 10⁷ CFU/g or less, 1 × 10⁶ CFU/g or less, 1 × 10⁵ CFU/g or less, 1 × 10⁴ CFU/g or less, 1 × 10³ CFU/g or less, 1 × 10² CFU/g or less, 10 CFU/g or less, 1 CFU/g or less, 0.1 CFU/g or less, 0.01 CFU/g or less, 1 × 10⁻³ CFU/g or less, 1 × 10⁻⁴ CFU/g or less, or 1 × 10⁻⁵ CFU/g or less; or may be any combination that does not contradict with the above-described ranges. Specifically, the content of the active ingredient in the composition according to the present invention, in terms of the content of yeast bacterial cells, may be, for example, from 1 × 10⁻⁶ to 1 × 10⁻⁵ CFU/g, from 1 × 10⁻⁵ to 1 × 10⁻⁴ CFU/g, from 1 × 10⁻⁴ to 1 × 10⁻³ CFU/g, from 1 × 10⁻³ to 0.01 CFU/g, from 0.01 to 0.1 CFU/g, from 0.1 to 1 CFU/g, from 1 to 10 CFU/g, from 10 to 1 × 10² CFU/g, from 1 × 10² to 1 × 10³ CFU/g, from 1 × 10³ to 1 × 10⁴ CFU/g, from 1 × 10⁴ to 1 × 10⁵ CFU/g, from 1 × 10⁵ to 1 × 10⁶ CFU/g, from 1 × 10⁶ to 1 × 10⁷ CFU/g, from 1 × 10⁷ to 1 × 10⁸ CFU/g, from 1 × 10⁸ to 1 × 10⁹ CFU/g, from 1 × 10⁹ to 1 × 10¹⁰ CFU/g, from 1 × 10¹⁰ to 1 × 10¹¹ CFU/g, from 1 × 10¹¹ to 1 × 10¹² CFU/g, or from 1 × 10¹² to 1 × 10¹³ CFU/g. Specifically, the content of the active ingredient in the composition according to the present invention, in terms of the content of yeast bacterial cells, may be, for example, from 1 × 10⁻⁶ to 1 × 10¹³ CFU/g, from 1 × 10⁻⁵ to 1 × 10¹² CFU/g, or from 1 × 10⁻⁴ to 1 × 10¹¹ CFU/g.

The content of each component (namely, each of the active ingredient and any other optional components) in the composition according to the present invention can be set, for example, such that the added amount of each component in the method according to the present invention to be described later is achieved.

The respective components (namely, the active ingredient and any other optional components) contained in the composition according to the present invention, may be contained in the composition according to the present invention in a state mixed with one another, or alternatively, each may be contained separately, or contained separately in an arbitrary combination, in the composition according to the present invention. For example, the composition according to the present invention may be provided as a set of components each separately packaged. In such a case, each component included in the set can be used in combination, as appropriate, at the time of use.

The method of producing the composition according to the present invention will now be described below. That is, the composition according to the present invention may be one produced by the method to be described below.

The method of producing the composition according to the present invention may include a step of producing the active ingredient. The active ingredient to be contained in the composition according to the present invention may be one produced by the above-described method of producing the active ingredient. Accordingly, the step of producing the active ingredient, in the method of producing the composition according to the present invention, may be a step of producing the active ingredient by the above-described method of producing the active ingredient. Specifically, the step of producing the active ingredient, in the method of producing the composition according to the present invention, may be a step of culturing yeast in a culture medium containing a plant belonging to the family *Solanaceae,* to obtain a cultured product. The "cultured product" described above is also referred to as "fermented product".

Specifically, the method of producing the composition according to the present invention may be, for example, a method of producing a composition for improving a flavor of food (for example, a composition for improving the tomato-like flavor, the spicy flavor and/or the matured flavor, in the food), the method including a step of culturing yeast in a culture medium containing a plant belonging to the family *Solanaceae,* to obtain a fermented product, wherein the composition contains the fermented product.

Specifically, the method of producing the composition according to the present invention may be, for example, a method of producing a composition containing 2-phenylethanol, the method including a step of culturing yeast in a culture medium containing a plant belonging to the family *Solanaceae,* to obtain a fermented product containing 2-phenylethanol, wherein the composition contains the fermented product.

The active ingredient can be used as the composition according to the present invention, as it is, or in combination with any other components, as appropriate. That is, the composition according to the present invention can be produced by combining the active ingredient and any other optional components, as appropriate. Further, the method of producing the composition according to the present invention may include a step of processing the active ingredient into a desired form. For example, the active ingredient may be dried and powdered to be used as the composition according to the present invention. The method of producing the composition according to the present invention may include, for example, a step of drying and powdering the active ingredient. The drying can be carried out, for example, by a drying means known as a means for drying food, such as freeze drying, spray drying, drum drying or vacuum drum drying.

### 3. Method According to Present Invention

The method according to the present invention is a method including a step of using the active ingredient (namely, a yeast-fermented product of a plant belonging to the family *Solanaceae).*

The method according to the present invention, specifically, the use of the active ingredient, makes it possible to improve a flavor of food (for example, to improve the tomato-like flavor, the spicy flavor and/or the matured flavor, in the food); namely, the present method makes it possible to obtain the flavor-improving effect (such as the tomato-like-flavor-improving effect, the spicy flavor-improving effect and/or the matured flavor-improving effect). Accordingly, the method according to the present invention, may be carried out for improving a flavor of food (for example, improving the tomato-like flavor, the spicy flavor and/or the matured flavor, in the food). That is, the method according to the present invention may be, for example, a method of improving a flavor of food (for example, a method of improving the tomato-like flavor, the spicy flavor, and/or the matured flavor, in the food). The method described above is also referred to as the "method of improving a flavor of food according to the present invention".

Further, the method according to the present invention, specifically, the use of the active ingredient, makes it possible to produce food with an improved flavor (for example, food with an improved tomato-like flavor, spicy flavor and/or matured flavor). Accordingly, the method according to the present invention may be carried out for producing food (specifically, for producing food with an improved flavor, such as food with an improved tomato-like flavor, spicy flavor and/or matured flavor). That is, the method according to the present invention may be, for example, a method of producing food (specifically, a method of producing food with an improved flavor, such as food with an improved tomato-like flavor, spicy flavor and/or matured flavor). The method described above is also referred to as the "method of producing food according to the present invention".

The active ingredient can be used for improving a flavor of food or producing food, by adding the active ingredient to a raw material of the food, in the production of the food. That is, the use of the active ingredient may be, for example, adding the active ingredient to a raw material of the food. That is, the method according to the present invention may specifically be, for example, a method of improving a flavor of food, the method including adding the active ingredient to a raw material of the food (for example, a method of improving the tomato-like flavor, the spicy flavor, and/or the matured flavor, in the food). Further, the method according to the present invention may specifically be, for example, a method of producing food (specifically, a method of producing food with an improved flavor, such as food with an improved tomato-like flavor, spicy flavor and/or matured flavor), the method including adding the active ingredient to a raw material of the food. The "addition (or adding)" is also referred to as "incorporation (or incorporating)".

The active ingredient may be used in the method according to the present invention, for example, in the form of the composition according to the present invention. That is, the definition of the expression "the use of the active ingredient" includes the use of the composition according to the present invention. For example, the definition of the expression "the addition of the active ingredient" includes the addition of the composition according to the present invention.

The food to be obtained by the method according to the present invention is also referred to as the "food according to the present invention". Specifically, the food according to the present invention is food with an improved flavor (for example, food with an improved tomato-like flavor, spicy flavor and/or matured flavor). The food according to the present invention is, in other words, food to which the active ingredient is added. Further, the food according to the present invention is, in other words, food containing the active ingredient.

The improvement of the flavor of food or the production of food may be carried out, for example, in the same manner as the production of a regular food, except for using the active ingredient. That is, the improvement of the flavor of food or the production of food may be carried out, for example, using the same raw material or materials and under the same production conditions as those of a regular food, except for using the active ingredient. Further, both the raw material of the food and the production conditions may be modified as appropriate, and used for improving a flavor of food or producing food.

The food is not particularly limited, as long as the improvement of the flavor of the food is desired. The definition of the food includes a beverage. The definition of the food also includes a seasoning. The food may be, for example, a liquid or a solid.

The food may be, for example, tomato-containing food. Specific examples of such food include: beverages such as soft drinks, alcohol beverages and soups; cooked rice products such as Japanese chicken rice, Japanese omelet rice and paella; stewed dishes such as curry, beef stew, Japanese hashed beef (Hayashi Rice), hashed beef, pork beans, chili con carne and Feijoada; roux such as curry roux; sauces such as tomato sauce and meat sauce; pizzas such as Pizza Margherita; pasta dishes such as Vongole Rosso, Puttanesca, Amatriciana, Arrabiata, Pescatore and Bolognese; and seasonings such as tomato ketchup. The "soft drink" may refer to any non-alcoholic beverage (a beverage having an alcohol concentration of less than 1%) excluding cow's milk and a dairy product. Specific examples of the soft drink include soft drinks containing tomato, such as tomato juice. Specific examples of the soup include soups containing tomato, such as tomato potage soup and minestrone.

Further, the food may be, for example, spice-containing food. Specific examples of such food include: soups such as dahl soup, Tom Yum Goong, potage soup, soups for instant noodles; sauces such as Genova sauce, Worcestershire sauce, chili sauce, Tabasco sauce and salsa; seasoning oils such as chili oil; consommé such as solid consommé and liquid consommé; flavor seasonings such as bonito flavor seasonings, chicken flavor seasonings, pork flavor seasonings and beef flavor seasonings; spicy seasonings such as Japanese mixed chili pepper, doubanjiang, gochujang (red chili paste) and wasabi paste; spice-flavored foods such as chili-flavored foods, chili pepper-flavored foods, Japanese pepper-flavored foods, Sichuan pepper-flavored foods, wasabi-flavored foods and cardamom-flavored foods; basic seasonings; menu condiments (special seasonings prepared specifically for the menus of the dishes to be cooked, such as chutney); roux such as stew roux and curry roux; stewed dishes such as curry, stew, chili con carne, Feijoada, Mapo tofu, Mapo eggplant, shrimp chili and Chige (Korean stew); cooked rice products such as Mapo rice bowl; noodle dishes such as Dandan noodles; confectioneries such as cinnamon cookies, and snacks such as potato snacks and corn snacks; beverages such as chai, cinnamon tea, ginger ale and Dr Pepper; and frying compositions such as fried chicken flour.

Further, the food may be, for example, a seasoning obtained by fermenting beans or wheat, a seasoning containing rice malt or miso, a seasoning obtained by fermenting beans or wheat and containing rice malt or miso, and food containing such a seasoning. Specific examples of such a seasoning include douchi, tianmianjiang, doubanjiang, gochujang, soy sauce and miso. Specific examples of food containing such a seasoning include Mapo tofu, Mapo rice bowl, Mapo eggplant, Hui Guo Rou (twice-cooked pork), Dandan noodles, Bang bang chicken, shrimp chili, stir-fried liver and chives, and Peking duck.

Further, the food may be, for example, milk-containing food and/or a dairy product. Specific examples of such food include ice cream, yoghurt, lactic acid bacteria drinks, cheese sauce, clam chowder, cream stew, and potato potage.

The form for providing the food is not particularly limited. For example, the food may be provided in a form edible as it is, or in a form that requires cooking before eating or at the time of eating, such as a concentrated product or a dried product. Further, the food may be provided in a form contained in an arbitrary container, such as, for example: a retort pouch, a paper pack, a plastic bottle such as a pet bottle, a metal can such as a steel can or an aluminum can, or a glass bottle. The food may be provided in the form of food that requires heating. The form of the food that requires heating may be, for example, a retort food. The food is not limited to a general food, and the definition thereof includes a so-called health food or medical food, such as a nutritional supplement, a Food with Nutrient Function Claims and a Food for Specified Health Uses. That is, for example, each of the foods exemplified above may be provided as a general food, or as a health food or a medical food.

The "raw material of the food" refers to food material for producing the food. The raw material of the food is not particularly limited, as long as the food can be produced. The raw material of the food can be selected as appropriate, for example, depending on various conditions such as the type of the food and the like. The raw material of the food may be, for example, a raw material which can be usually used in the production of food such as any of those exemplified above. Specific examples of the raw material of the food include: tomato; cereal grains such as rice and wheat; seasoning component such as saccharides, inorganic salts, organic acids, nucleic acids, amino acids and protein hydrolysates; dairy products such as cow's milk, cheese and butter; fruits; vegetables; meat; fish; eggs; spices; flavoring agents; fats and oils; alcohols; dietary fibers; and pH buffers.

The active ingredient may be added to the raw material of the food, at any stage of the production process of the food as long as the flavor-improving effect can be obtained. That is, the "raw material of the food" to which the active ingredient is added may be the raw material at any stage of the production process of the food. For example, the definition of the "raw material of the food" to which the active ingredient is added may include a finished food which is before the active ingredient is added thereto. The active ingredient can be added to the raw material of the food, as it is, or after being prepared in a desired form, such as a solution, as appropriate. The expression "the addition of the active ingredient" may generally refer to the operation of allowing the active ingredient to coexist with the raw material of the food. A component other than the active ingredient (such as tomato or a spice) may also be added as appropriate, to the raw material of the food. The description of the addition of the active ingredient can also be applied to the addition of a component other than the active ingredient. The respective components (namely, the active ingredient and any other optional components) may be added to the raw material of the food all at once, or alternatively, each may be added separately, or added separately in an arbitrary combination, to the raw material of the food. The order of adding the respective components to the raw material of the food is not particularly limited.

The added amount or the ratio of the added amount of each component (namely, each of the active ingredient and any other optional components) in the composition according to the present invention is not particularly limited, as long as the flavor-improving effect can be obtained. The added amount or the ratio of the added amount of each component in the method according to the present invention can be set as appropriate, depending on various conditions, such as the type of the raw material of the food and the type of the food.

The active ingredient may be added to the raw material of the food, for example, such that the eating concentration of the active ingredient is within a desired range (for example, the range of the eating concentration of the active ingredient to be described later).

The eating concentration of the active ingredient, in terms of the eating concentration of the original cultured product, may be, for example, 1 × 10⁻¹⁴% (w/w) or more, 1 × 10⁻¹³% (w/w) or more, 1 × 10⁻¹²% (w/w) or more, 1 × 10⁻¹¹% (w/w) or more, 1 × 10⁻¹⁰% (w/w) or more, 1 × 10⁻⁹% (w/w) or more, 1 × 10⁻⁸% (w/w) or more, 1 × 10⁻⁷% (w/w) or more, 1 × 10⁻⁶% (w/w) or more, 1 × 10⁻⁵% (w/w) or more, 1 × 10⁻⁴% (w/w) or more, 1 × 10⁻³% (w/w) or more, 0.01% (w/w) or more, 0.02% (w/w) or more, 0.05% (w/w) or more, 0.1% (w/w) or more, 0.2% (w/w) or more, 0.5% (w/w) or more, 1% (w/w) or more, 2% (w/w) or more, or 5% (w/w) or more; may bel0% (w/w) or less, 5% (w/w) or less, 2% (w/w) or less, 1% (w/w) or less, 0.5% (w/w) or less, 0.2% (w/w) or less, 0.1% (w/w) or less, 0.05% (w/w) or less, 0.02% (w/w) or less, 0.01% (w/w) or less, 1 × 10⁻³% (w/w) or less, 1 × 10⁻⁴% (w/w) or less, 1 × 10⁻⁵% (w/w) or less, 1 × 10⁻⁶% (w/w) or less, 1 × 10⁻⁷% (w/w) or less, 1 × 10⁻⁸% (w/w) or less, 1 × 10⁻⁹% (w/w) or less, 1 × 10⁻¹⁰% (w/w) or less, 1 × 10⁻¹¹% (w/w) or less, 1 × 10⁻¹²% (w/w) or less, or 1 × 10⁻¹³% (w/w) or less; or may be any combination that does not contradict with the above-described ranges. Specifically, the eating concentration of the active ingredient, in terms of the eating concentration of the original cultured product, may be, for example, from 1 × 10⁻¹⁴ to 1 × 10⁻¹³% (w/w), from 1 × 10⁻¹³ to 1 × 10⁻¹²% (w/w), from 1 × 10⁻¹² to 1 × 10⁻¹¹% (w/w), from 1 × 10⁻¹¹ to 1 × 10⁻¹⁰% (w/w), from 1 × 10⁻¹⁰ to 1 × 10⁻⁹% (w/w), from 1 × 10⁻⁹ to 1 × 10⁻⁸% (w/w), from 1 × 10⁻⁸ to 1 × 10⁻⁷% (w/w), from 1 × 10⁻⁷ to 1 × 10⁻⁶% (w/w), from 1 × 10⁻⁶ to 1 × 10⁻⁵% (w/w), from 1 × 10⁻⁵ to 1 × 10⁻⁴% (w/w), from 1 × 10⁻⁴ to 1 × 10⁻³% (w/w), from 1 × 10⁻³ to 0.01% (w/w), from 0.01 to 0.02% (w/w), from 0.02 to 0.05% (w/w), from 0.05 to 0.1% (w/w), from 0.1 to 0.2% (w/w), from 0.2 to 0.5% (w/w), from 0.5 to 1% (w/w), from 1 to 2% (w/w), from 2 to 5% (w/w), or from 5 to 10% (w/w). Specifically, the eating concentration of the active ingredient, in terms of the eating concentration of the original cultured product, may be, for example, from 1 × 10⁻¹⁴ to 10% (w/w), from 1 × 10⁻¹³ to 2% (w/w), or from 1 × 10⁻¹² to 0.5% (w/w). The "original cultured product" refers to a cultured product in which no change in the concentration due to concentration, dilution or the like has occurred after the culture, and specifically, may refer to a cultured product at the completion of the culture. The "original cultured product" is also referred to as "original fermented product". For example, in cases where the cultured product which has been concentrated two-fold is added such that the eating concentration is 1% (w/w), the eating concentration of the active ingredient, in terms of the eating concentration of the original cultured product, is 2% (w/w).

In cases where the active ingredient is a dry powder produced by the production method including a step of drying and powdering the active ingredient, in particular, the eating concentration of the active ingredient, in terms of the eating concentration of the dry powder, may be, for example, 1 × 10⁻¹⁰% (w/w) or more, 1 × 10⁻⁹% (w/w) or more, 1 × 10⁻⁸% (w/w) or more, 1 × 10⁻⁷% (w/w) or more, 1 × 10⁻⁶% (w/w) or more, 1 × 10⁻⁵% (w/w) or more, 1 × 10⁻⁴% (w/w) or more, 1 × 10⁻³% (w/w) or more, 0.01% (w/w) or more, 0.02% (w/w) or more, 0.05% (w/w) or more, 0.1% (w/w) or more, 0.2% (w/w) or more, 0.5% (w/w) or more, 1% (w/w) or more, 2% (w/w) or more, 5% (w/w) or more, or 10% (w/w) or more; may be 20% (w/w) or less, 10% (w/w) or less, 5% (w/w) or less, 2% (w/w) or less, 1% (w/w) or less, 0.5% (w/w) or less, 0.2% (w/w) or less, 0.1% (w/w) or less, 0.05% (w/w) or less, 0.02% (w/w) or less, 0.01% (w/w) or less, 1 × 10⁻³% (w/w) or less, 1 × 10⁻⁴% (w/w) or less, 1 × 10⁻⁵% (w/w) or less, 1 × 10⁻⁶% (w/w) or less, 1 × 10⁻⁷% (w/w) or less, 1 × 10⁻⁸% (w/w) or less, or 1 × 10⁻⁹% (w/w) or less; or may be any combination that does not contradict with the above-described ranges. Specifically, the eating concentration of the active ingredient, in terms of the eating concentration of the dry powder, may be, for example, from 1 × 10⁻¹⁰ to 1 × 10⁻⁹% (w/w), from 1 × 10⁻⁹ to 1 × 10⁻⁸% (w/w), from 1 × 10⁻⁸ to 1 × 10⁻⁷% (w/w), from 1 × 10⁻⁷ to 1 × 10⁻⁶% (w/w), from 1 × 10⁻⁶ to 1 × 10⁻⁵% (w/w), from 1 × 10⁻⁵ to 1 × 10⁻⁴% (w/w), from 1 × 10⁻⁴ to 1 × 10⁻³% (w/w), from 1 × 10⁻³ to 0.01% (w/w), from 0.01 to 0.02% (w/w), from 0.02 to 0.05% (w/w), from 0.05 to 0.1% (w/w), from 0.1 to 0.2% (w/w), from 0.2 to 0.5% (w/w), from 0.5 to 1% (w/w), from 1 to 2% (w/w), from 2 to 5% (w/w), from 5 to 10% (w/w), or from 10 to 20% (w/w). Specifically, the eating concentration of the active ingredient, in terms of the eating concentration of the dry powder, may be, for example, from 1 × 10⁻¹⁰ to 20% (w/w), from 1 × 10⁻⁹ to 10% (w/w), or from 1 × 10⁻⁸ to 5% (w/w).

The added amount of the active ingredient, with respect to the amount of the food whose flavor is to be improved, may be, for example, 1 × 10⁻¹⁰% (w/w) or more, 1 × 10⁻⁹% (w/w) or more, 1 × 10⁻⁸% (w/w) or more, 1 × 10⁻⁷% (w/w) or more, 1 × 10⁻⁶% (w/w) or more, 1 × 10⁻⁵% (w/w) or more, 1 × 10⁻⁴% (w/w) or more, 1 × 10⁻³% (w/w) or more, 0.01% (w/w) or more, 0.02% (w/w) or more, 0.05% (w/w) or more, 0.1% (w/w) or more, 0.2% (w/w) or more, 0.5% (w/w) or more, 1% (w/w) or more, 2% (w/w) or more, 5% (w/w) or more, 10% (w/w) or more, or 20% (w/w) or more; may be 30% (w/w) or less, 20% (w/w) or less, 10% (w/w) or less, 5% (w/w) or less, 2% (w/w) or less, 1% (w/w) or less, 0.5% (w/w) or less, 0.2% (w/w) or less, 0.1% (w/w) or less, 0.05% (w/w) or less, 0.02% (w/w) or less, 0.01% (w/w) or less, 1 × 10⁻³% (w/w) or less, 1 × 10⁻⁴% (w/w) or less, 1 × 10⁻⁵% (w/w) or less, 1 × 10⁻⁶% (w/w) or less, 1 × 10⁻⁷% (w/w) or less, 1 × 10⁻⁸% (w/w) or less, or 1 × 10⁻⁹% (w/w) or less; or may be any combination that does not contradict with the above-described ranges. Specifically, the added amount of the active ingredient, with respect to the amount of the food whose flavor is to be improved, may be, for example, from 1 × 10⁻¹⁰ to 1 × 10⁻⁹% (w/w), from 1 × 10⁻⁹ to 1 × 10⁻⁸% (w/w), from 1 × 10⁻⁸ to 1 × 10⁻⁷% (w/w), from 1 × 10⁻⁷ to 1 × 10⁻⁶% (w/w), from 1 × 10⁻⁶ to 1 × 10⁻⁵% (w/w), from 1 × 10⁻⁵ to 1 × 10⁻⁴% (w/w), from 1 × 10⁻⁴ to 1 × 10⁻³% (w/w), from 1 × 10⁻³ to 0.01% (w/w), from 0.01 to 0.02% (w/w), from 0.02 to 0.05% (w/w), from 0.05 to 0.1% (w/w), from 0.1 to 0.2% (w/w), from 0.2 to 0.5% (w/w), from 0.5 to 1% (w/w), from 1 to 2% (w/w), from 2 to 5% (w/w), from 5 to 10% (w/w), from 10 to 20% (w/w), or from 20 to 30% (w/w). Specifically, the added amount of the active ingredient, with respect to the amount of the food whose flavor is to be improved, may be, for example, from 1 × 10⁻¹⁰ to 30% (w/w), from 1 × 10⁻⁹ to 20% (w/w), or from 1 × 10⁻⁸ to 10% (w/w).

In cases where the active ingredient is a dry powder produced by the production method including a step of drying and powdering the active ingredient, in particular, the added amount of the active ingredient, with respect to the amount of the food whose flavor is to be improved, may be, for example, 1 × 10⁻¹⁰% (w/w) or more, 1 × 10⁻⁹% (w/w) or more, 1 × 10⁻⁸% (w/w) or more, 1 × 10⁻⁷% (w/w) or more, 1 × 10⁻⁶% (w/w) or more, 1 × 10⁻⁵% (w/w) or more, 1 × 10⁻⁴% (w/w) or more, 1 × 10⁻³% (w/w) or more, 0.01% (w/w) or more, 0.02% (w/w) or more, 0.05% (w/w) or more, 0.1% (w/w) or more, 0.2% (w/w) or more, 0.5% (w/w) or more, 1% (w/w) or more, 2% (w/w) or more, 5% (w/w) or more, or 10% (w/w) or more; may be 20% (w/w) or less, 10% (w/w) or less, 5% (w/w) or less, 2% (w/w) or less, 1% (w/w) or less, 0.5% (w/w) or less, 0.2% (w/w) or less, 0.1% (w/w) or less, 0.05% (w/w) or less, 0.02% (w/w) or less, 0.01% (w/w) or less, 1 × 10⁻³% (w/w) or less, 1 × 10⁻⁴% (w/w) or less, 1 × 10⁻⁵% (w/w) or less, 1 × 10⁻⁶% (w/w) or less, 1 × 10⁻⁷% (w/w) or less, 1 × 10⁻⁸% (w/w) or less, or 1 × 10⁻⁹% (w/w) or less; or may be any combination that does not contradict with the above-described ranges. Specifically, the added amount of the active ingredient, with respect to the amount of the food whose flavor is to be improved, may be, for example, from 1 × 10⁻¹⁰ to 1 × 10⁻⁹% (w/w), from 1 × 10⁻⁹ to 1 × 10⁻⁸% (w/w), from 1 × 10⁻⁸ to 1 × 10⁻⁷% (w/w), from 1 × 10⁻⁷ to 1 × 10⁻⁶% (w/w), from 1 × 10⁻⁶ to 1 × 10⁻⁵% (w/w), from 1 × 10⁻⁵ tₒ 1 × 10⁻⁴% (w/w), from 1 × 10⁻⁴ to 1 × 10⁻³% (w/w), from 1 × 10⁻³ to 0.01% (w/w), from 0.01 to 0.02% (w/w), from 0.02 to 0.05% (w/w), from 0.05 to 0.1% (w/w), from 0.1 to 0.2% (w/w), from 0.2 to 0.5% (w/w), from 0.5 to 1% (w/w), from 1 to 2% (w/w), from 2 to 5% (w/w), from 5 to 10% (w/w), or from 10 to 20% (w/w). Specifically, the added amount of the active ingredient, with respect to the amount of the food whose flavor is to be improved, may be, for example, from 1 × 10⁻¹⁰ to 20% (w/w), from 1 × 10⁻⁹ to 10% (w/w), or from 1 × 10⁻⁸ to 5%.

The eating concentration of the active ingredient, in terms of the eating concentration of 2-PE, may be, for example, 1 × 10⁻¹³ ppm (w/w) or more, 1 × 10⁻¹² ppm (w/w) or more, 1 × 10⁻¹¹ ppm (w/w) or more, 1 × 10⁻¹⁰ ppm (w/w) or more, 1 × 10⁻⁹ ppm (w/w) or more, 1 × 10⁻⁸ ppm (w/w) or more, 1 × 10⁻⁷ ppm (w/w) or more, 1 × 10⁻⁶ ppm (w/w) or more, 1 × 10⁻⁵ ppm (w/w) or more, 1 × 10⁻⁴ ppm (w/w) or more, 1 × 10⁻³ ppm (w/w) or more, 0.01 ppm (w/w) or more, 0.1 ppm (w/w) or more, 0.2 ppm (w/w) or more, 0.5 ppm (w/w) or more, 1 ppm (w/w) or more, 2 ppm (w/w) or more, 5 ppm (w/w) or more, 10 ppm (w/w) or more, 20 ppm (w/w) or more, or 50 ppm (w/w) or more; may be 100 ppm (w/w) or less, 50 ppm (w/w) or less, 20 ppm (w/w) or less, 10 ppm (w/w) or less, 5 ppm (w/w) or less, 2 ppm (w/w) or less, 1 ppm (w/w) or less, 0.5 ppm (w/w) or less, 0.2 ppm (w/w) or less, 0.1 ppm (w/w) or less, 0.01 ppm (w/w) or less, 1 × 10⁻³ ppm (w/w) or less, 1 × 10⁻⁴ ppm (w/w) or less, 1 × 10⁻⁵ ppm (w/w) or less, 1 × 10⁻⁶ ppm (w/w) or less, 1 × 10⁻⁷ ppm (w/w) or less, 1 × 10⁻⁸ ppm (w/w) or less, 1 × 10⁻⁹ ppm (w/w) or less, 1 × 10⁻¹⁰ ppm (w/w) or less, 1 × 10⁻¹¹ ppm (w/w) or less, or 1 × 10⁻¹² ppm (w/w) or less; or may be any combination that does not contradict with the above-described ranges. Specifically, the eating concentration of the active ingredient, in terms of the eating concentration of 2-PE, may be, for example, from 1 × 10⁻¹³ to 1 × 10⁻¹² ppm (w/w), from 1 × 10⁻¹² to 1 × 10⁻¹¹ ppm (w/w), from 1 × 10⁻¹¹ to 1 × 10⁻¹⁰ ppm (w/w), from 1 × 10⁻¹⁰ to 1 × 10⁻⁹ ppm (w/w), from 1 × 10⁻⁹ to 1 × 10⁻⁸ ppm (w/w), from 1 × 10⁻⁸ to 1 × 10⁻⁷ ppm (w/w), from 1 × 10⁻⁷ to 1 × 10⁻⁶ ppm (w/w), from 1 × 10⁻⁶ to 1 × 10⁻⁵ ppm (w/w), from 1 × 10⁻⁵ to 1 × 10⁻⁴ ppm (w/w), from 1 × 10⁻⁴ to 1 × 10⁻³ ppm (w/w), from 1 × 10⁻³ to 0.01 ppm (w/w), from 0.01 to 0.1 ppm (w/w), from 0.1 to 0.2 ppm (w/w), from 0.2 to 0.5 ppm (w/w), from 0.5 to 1 ppm (w/w), from 1 to 2 ppm (w/w), from 2 to 5 ppm (w/w), from 5 to 10 ppm (w/w), from 10 to 20 ppm (w/w), from 20 to 50 ppm (w/w), or from 50 to 100 ppm (w/w). Specifically, the eating concentration of the active ingredient, in terms of the eating concentration of 2-PE, may be, for example, from 1 × 10⁻¹³ to 100 ppm (w/w), from 1 × 10⁻¹² to 20 ppm (w/w), or from 1 × 10⁻¹¹ to 5 ppm (w/w).

The eating concentration of the active ingredient, in terms of the eating concentration of ethyl acetate, may be, for example, 1 × 10⁻¹⁵ ppm (w/w) or more, 1 × 10⁻¹⁴ ppm (w/w) or more, 1 × 10⁻¹³ ppm (w/w) or more, 1 × 10⁻¹² ppm (w/w) or more, 1 × 10⁻¹¹ ppm (w/w) or more, 1 × 10⁻¹⁰ ppm (w/w) or more, 1 × 10⁻⁹ ppm (w/w) or more, 1 × 10⁻⁸ ppm (w/w) or more, 1 × 10⁻⁷ ppm (w/w) or more, 1 × 10⁻⁶ ppm (w/w) or more, 1 × 10⁻⁵ ppm (w/w) or more, 1 × 10⁻⁴ ppm (w/w) or more, 0.001 ppm (w/w) or more, 0.002 ppm (w/w) or more, 0.005 ppm (w/w) or more, 0.01 ppm (w/w) or more, 0.02 ppm (w/w) or more, 0.05 ppm (w/w) or more, 0.1 ppm (w/w) or more, 0.2 ppm (w/w) or more, or 0.5 ppm (w/w) or more; may be 1 ppm (w/w) or less, 0.5 ppm (w/w) or less, 0.2 ppm (w/w) or less, 0.1 ppm (w/w) or less, 0.05 ppm (w/w) or less, 0.02 ppm (w/w) or less, 0.01 ppm (w/w) or less, 0.005 ppm (w/w) or less, 0.002 ppm (w/w) or less, 0.001 ppm (w/w) or less, 1 × 10⁻⁴ ppm (w/w) or less, 1 × 10⁻⁵ ppm (w/w) or less, 1 × 10⁻⁶ ppm (w/w) or less, 1 × 10⁻⁷ ppm (w/w) or less, 1 × 10⁻⁸ ppm (w/w) or less, 1 × 10⁻⁹ ppm (w/w) or less, 1 × 10⁻¹⁰ ppm (w/w) or less, 1 × 10⁻¹¹ ppm (w/w) or less, 1 × 10⁻¹² ppm (w/w) or less, 1 × 10⁻¹³ ppm (w/w) or less, or 1 × 10⁻¹⁴ ppm (w/w) or less; or may be any combination that does not contradict with the above-described ranges. Specifically, the eating concentration of the active ingredient, in terms of the eating concentration of ethyl acetate, may be, for example, from 1 × 10⁻¹⁵ to 1 × 10⁻¹⁴ ppm (w/w), from 1 × 10⁻¹⁴ to 1 × 10⁻¹³ ppm (w/w), from 1 × 10⁻¹³ to 1 × 10⁻¹² ppm (w/w), from 1 × 10⁻¹² to 1 × 10⁻¹¹ ppm (w/w), from 1 × 10⁻¹¹ to 1 × 10⁻¹⁰ ppm (w/w), from 1 × 10⁻¹⁰ to 1 × 10⁻⁹ ppm (w/w), from 1 × 10⁻⁹ to 1 × 10⁻⁸ ppm (w/w), from 1 × 10⁻⁸ to 1 × 10⁻⁷ ppm (w/w), from 1 × 10⁻⁷ to 1 × 10⁻⁶ ppm (w/w), from 1 × 10⁻⁶ to 1 × 10⁻⁵ ppm (w/w), from 1 × 10⁻⁵ to 1 × 10⁻⁴ ppm (w/w), from 1 × 10⁻⁴ to 0.001 ppm (w/w), from 0.001 to 0.002 ppm (w/w), from 0.002 to 0.005 ppm (w/w), from 0.005 to 0.01 ppm (w/w), from 0.01 to 0.02 ppm (w/w), from 0.02 to 0.05 ppm (w/w), from 0.05 to 0.1 ppm (w/w), from 0.1 to 0.2 ppm (w/w), from 0.2 to 0.5 ppm (w/w), or from 0.5 to 1 ppm (w/w). Specifically, the eating concentration of the active ingredient, in terms of the eating concentration of ethyl acetate, may be, for example, from 1 × 10⁻¹⁵ to 1 ppm (w/w), from 1 × 10⁻¹⁴ to 0.2 ppm (w/w), or from 1 × 10⁻¹³ to 0.05 ppm (w/w).

The eating concentration of the active ingredient, in terms of the eating concentration of diacetyl, may be, for example, 1 × 10⁻¹⁵ ppm (w/w) or more, 1 × 10⁻¹⁴ ppm (w/w) or more, 1 × 10⁻¹³ ppm (w/w) or more, 1 × 10⁻¹² ppm (w/w) or more, 1 × 10⁻¹¹ ppm (w/w) or more, 1 × 10⁻¹⁰ ppm (w/w) or more, 1 × 10⁻⁹ ppm (w/w) or more, 1 × 10⁻⁸ ppm (w/w) or more, 1 × 10⁻⁷ ppm (w/w) or more, 1 × 10⁻⁶ ppm (w/w) or more, 1 × 10⁻⁵ ppm (w/w) or more, 1 × 10⁻⁴ ppm (w/w) or more, 0.001 ppm (w/w) or more, 0.002 ppm (w/w) or more, 0.005 ppm (w/w) or more, 0.01 ppm (w/w) or more, 0.02 ppm (w/w) or more, 0.05 ppm (w/w) or more, 0.1 ppm (w/w) or more, 0.2 ppm (w/w) or more, or 0.5 ppm (w/w) or more; may be 1 ppm (w/w) or less, 0.5 ppm (w/w) or less, 0.2 ppm (w/w) or less, 0.1 ppm (w/w) or less, 0.05 ppm (w/w) or less, 0.02 ppm (w/w) or less, 0.01 ppm (w/w) or less, 0.005 ppm (w/w) or less, 0.002 ppm (w/w) or less, 0.001 ppm (w/w) or less, 1 × 10⁻⁴ ppm (w/w) or less, 1 × 10⁻⁵ ppm (w/w) or less, 1 × 10⁻⁶ ppm (w/w) or less, 1 × 10⁻⁷ ppm (w/w) or less, 1 × 10⁻⁸ ppm (w/w) or less, 1 × 10⁻⁹ ppm (w/w) or less, 1 × 10⁻¹⁰ ppm (w/w) or less, 1 × 10⁻¹¹ ppm (w/w) or less, 1 × 10⁻¹² ppm (w/w) or less, 1 × 10⁻¹³ ppm (w/w) or less, or 1 × 10⁻¹⁴ ppm (w/w) or less; or may be any combination that does not contradict with the above-described ranges. Specifically, the eating concentration of the active ingredient, in terms of the eating concentration of diacetyl, may be, for example, from 1 × 10⁻¹⁵ to 1 × 10⁻¹⁴ ppm (w/w), from 1 × 10⁻¹⁴ to 1 × 10⁻¹³ ppm (w/w), from 1 × 10⁻¹³ to 1 × 10⁻¹² ppm (w/w), from 1 × 10⁻¹² to 1 × 10⁻¹¹ ppm (w/w), from 1 × 10⁻¹¹ to 1 × 10⁻¹⁰ ppm (w/w), from 1 × 10⁻¹⁰ to 1 × 10⁻⁹ ppm (w/w), from 1 × 10⁻⁹ to 1 × 10⁻⁸ ppm (w/w), from 1 × 10⁻⁸ to 1 × 10⁻⁷ ppm (w/w), from 1 × 10⁻⁷ to 1 × 10⁻⁶ ppm (w/w), from 1 × 10⁻⁶ to 1 × 10⁻⁵ ppm (w/w), from 1 × 10⁻⁵ to 1 × 10⁻⁴ ppm (w/w), from 1 × 10⁻⁴ to 0.001 ppm (w/w), from 0.001 to 0.002 ppm (w/w), from 0.002 to 0.005 ppm (w/w), from 0.005 to 0.01 ppm (w/w), from 0.01 to 0.02 ppm (w/w), from 0.02 to 0.05 ppm (w/w), from 0.05 to 0.1 ppm (w/w), from 0.1 to 0.2 ppm (w/w), from 0.2 to 0.5 ppm (w/w), or from 0.5 to 1 ppm (w/w). Specifically, the eating concentration of the active ingredient, in terms of the eating concentration of diacetyl, may be, for example, from 1 × 10⁻¹⁵ to 1 ppm (w/w), from 1 × 10⁻¹⁴ to 0.2 ppm (w/w), or from 1 × 10⁻¹³ to 0.05 ppm (w/w).

The eating concentration of the active ingredient, in terms of the eating concentration of yeast bacterial cells, may be, for example, 1 × 10⁻⁸ CFU/g or more, 1 × 10⁻⁷ CFU/g or more, 1 × 10⁻⁶ CFU/g or more, 1 × 10⁻⁵ CFU/g or more, 1 × 10⁻⁴ CFU/g or more, 1 × 10⁻³ CFU/g or more, 0.01 CFU/g or more, 0.1 CFU/g or more, 1 CFU/g or more, 10 CFU/g or more, 1 × 10² CFU/g or more, 1 × 10³ CFU/g or more, 1 × 10⁴ CFU/g or more, 1 × 10⁵ CFU/g or more, 1 × 10⁶ CFU/g or more, 1 × 10⁷ CFU/g or more, or 1 × 10⁸ CFU/g or more; may be 1 × 10⁹ CFU/g or less, 1 × 10⁸ CFU/g or less, 1 × 10⁷ CFU/g or less, 1 × 10⁶ CFU/g or less, 1 × 10⁵ CFU/g or less, 1 × 10⁴ CFU/g or less, 1 × 10³ CFU/g or less, 1 × 10² CFU/g or less, 10 CFU/g or less, 1 CFU/g or less, 0.1 CFU/g or less, 0.01 CFU/g or less, 1 × 10⁻³ CFU/g or less, 1 × 10⁻⁴ CFU/g or less, 1 × 10⁻⁵ CFU/g or less, 1 × 10⁻⁶ CFU/g or less, or 1 × 10⁻⁷ CFU/g or less; or may be any combination that does not contradict with the above-described ranges. Specifically, the eating concentration of the active ingredient, in terms of the eating concentration of yeast bacterial cells, may be, for example, from 1 × 10⁻⁸ to 1 × 10⁻⁷ CFU/g, from 1 × 10⁻⁷ to 1 × 10⁻⁶ CFU/g, from 1 × 10⁻⁶ to 1 × 10⁻⁵ CFU/g, from 1 × 10⁻⁵ to 1 × 10⁻⁴ CFU/g, from 1 × 10⁻⁴ to 1 × 10⁻³ CFU/g, from 1 × 10⁻³ to 0.01 CFU/g, from 0.01 to 0.1 CFU/g, from 0.1 to 1 CFU/g, from 1 to 10 CFU/g, from 10 to 1 × 10² CFU/g, from 1 × 10² to 1 × 10³ CFU/g, from 1 × 10³ to 1 × 10⁴ CFU/g, from 1 × 10⁴ to 10⁵ CFU/g, from 1 × 10⁵ to 10⁶ CFU/g, from 1 × 10⁶ to 10⁷ CFU/g, from 1 × 10⁷ to 1 × 10⁸ CFU/g, or from 1 × 10⁸ to 1 × 10⁹ CFU/g. Specifically, the eating concentration of the active ingredient, in terms of the eating concentration of yeast bacterial cells, may be, for example, from 1 × 10⁻⁸ to 1 × 10⁹ CFU/g, from 1 × 10⁻⁷ to 1 × 10⁸ CFU/g, or from 1 × 10⁻⁶ to 1 × 10⁷ CFU/g.

The description of the addition of the active ingredient can also be applied to the case of adding the composition according to the present invention. For example, the composition according to the present invention can be added such that the added amount of the active ingredient exemplified above is achieved.

The food according to the present invention may contain tomato. That is, the food according to the present invention may be produced to contain tomato. The tomato-containing food can be produced, for example, by the addition of tomato. That is, the method according to the present invention may further include adding tomato to the raw material of the food. As the tomato, fresh tomato may be used as it is, or after being subjected to processing, as appropriate, in the production of the food according to the present invention. The description of the processing of a plant belonging to the family *Solanaceae,* as a culture medium component, can also be applied to the processing of tomato. Examples of the processed product of tomato include tomato juice, tomato puree, and tomato paste. The addition of tomato can be carried out in the same manner as the addition of the active ingredient. Further, the tomato-containing food can be produced, for example, using a raw material of the tomato-containing food. That is, the raw material of the food may contain tomato. For example, another tomato-containing food may be produced, using tomato-containing food such as tomato sauce or tomato ketchup as the raw material.

The food according to the present invention may be tomato-containing food, in which the content of tomato is reduced. The "food in which the content of tomato is reduced" refers to food in which the content of tomato is lower than usual (namely, food in which the content of tomato is lower than the usual content of tomato). The food containing the usual amount of tomato (namely, food having the usual content of tomato) is also referred to as "usual tomato-containing food". That is, the "food in which the content of tomato is reduced" may refer specifically to the same type of food as the usual tomato-containing food, in which the content of tomato is lower than that in the usual tomato-containing food. More specifically, the "food in which the content of tomato is reduced" may refer to the same type of food as the usual tomato-containing food, in which the content of tomato is 0.9 times or less, 0.8 times or less, 0.7 times or less, 0.6 times or less, or 0.5 times or less, of that in the usual tomato-containing food. The food in which the content of tomato is reduced may be, for example, tomato-containing food, such as any of the foods exemplified above, which is produced so that the content of tomato is lower than usual. The usual tomato-containing food may be, for example, tomato-containing food, such as any of the foods exemplified above, which is produced so as to contain the usual amount of tomato. According to the present invention, for example, a decrease in tomato-like flavor due to a reduction in the content of tomato may be compensated. That is, the definition of the "improvement in tomato-like flavor" may include a compensation for a decrease in the tomato-like flavor due to a reduction in the content of tomato.

The food according to the present invention may contain a spice. That is, the food according to the present invention may be produced to contain a spice. The spice-containing food can be produced, for example, by the addition of a spice. That is, the method according to the present invention may further include adding a spice to the raw material of the food. The spice may be used as it is, or after being subjected to processing, as appropriate, in the food according to the present invention. The addition of a spice can be carried out in the same manner as the addition of the active ingredient. Further, the spice-containing food can be produced, for example, using a raw material of the spice-containing food. That is, the raw material of the food may contain a spice. For example, another spice-containing food may be produced, using spice-containing food such as curry roux as the raw material. The details of the spice are as described above. Examples of the spice which may be contained in the food according to the present invention include, in particular, cardamom, bay leaf, coriander, clove, nutmeg, mace, allspice, cinnamon, fennel, cumin, ginger, pepper, caraway, anise, basil, parsley, sage, thyme, oregano, rosemary, celery seed, mint, garden cress, dill, marjoram, knotweed, turmeric, lemongrass, chili pepper, Japanese pepper, garlic, shallot, onion, peppermint, Sichuan pepper, star anise, wasabi, and celery.

The food according to the present invention may be spice-containing food, in which the content of the spice is reduced. The "food in which the content of the spice is reduced" refers to food in which the content of the spice is lower than usual (namely, food in which the content of the spice is lower than the usual content of the spice). The food containing the usual amount of a spice (namely, food having the usual content of the spice) is also referred to as "usual spice-containing food". That is, the "food in which the content of the spice is reduced" may refer specifically to the same type of food as the usual spice-containing food, in which the content of the spice is lower than that in the usual spice-containing food. More specifically, the "food in which the content of the spice is reduced" may refer to the same type of food as the usual spice-containing food, in which the content of the spice is 0.9 times or less, 0.8 times or less, 0.7 times or less, 0.6 times or less, or 0.5 times or less, of that in the usual spice-containing food. The food in which the content of the spice is reduced may be, for example, spice-containing food, such as any of the foods exemplified above, which is produced so that the content of the spice is lower than usual. The usual spice-containing food may be, for example, spice-containing food, such as any of the foods exemplified above, which is produced so as to contain the usual amount of the spice. According to the present invention, for example, a decrease in spicy flavor due to a reduction in the content of the spice may be compensated. That is, the definition of the "improvement in spicy flavor" may include a compensation for a decrease in the spicy flavor due to a reduction in the content of the spice.

The food according to the present invention may contain a seasoning obtained by fermenting beans or wheat. Further, the food according to the present invention may contain a seasoning containing rice malt or miso. In addition, the food according to the present invention may contain a seasoning obtained by fermenting beans or wheat and containing rice malt or miso. That is, the food according to the present invention may be produced to contain such a seasoning. The food containing such a seasoning can be produced, for example, by the addition of such a seasoning. That is, the method according to the present invention may further include adding such a seasoning to the raw material of the food.

The food according to the present invention may contain milk and/or a dairy product food as the raw material of the food. Examples of the milk include raw milk, cow's milk, special cow's milk, raw goat's milk, sterilized goat's milk, raw sheep's milk, modified cow's milk, low-fat cow's milk, non-fat cow's milk, and processed milk. Examples of the dairy product include cream, butter, butter oil, cheese, whey concentrate, concentrated milk, skimmed concentrated milk, unsweetened condensed milk, unsweetened condensed skim milk, sweetened condensed milk, sweetened condensed skim milk, whole milk powder, skim milk powder, cream powder, whey powder, protein concentrate whey powder, buttermilk powder, sweetened milk powder, modified milk powder, and fermented milk.

The food according to the present invention may be food containing: a seasoning obtained by fermenting beans or wheat; a seasoning containing rice malt or miso; or a seasoning obtained by fermenting beans or wheat and containing rice malt or miso, in which the content of the seasoning obtained by fermenting beans or wheat, the seasoning containing rice malt or miso, or the seasoning obtained by fermenting beans or wheat and containing rice malt or miso, is reduced. The "food in which the content of the seasoning obtained by fermenting beans or wheat is reduced" refers to food in which the content of the seasoning obtained by fermenting beans or wheat is lower than usual (namely, food in which the content of the seasoning obtained by fermenting beans or wheat is lower than the usual content of the seasoning obtained by fermenting beans or wheat). The food containing the usual amount of a seasoning obtained by fermenting beans or wheat (namely, food having the usual content of the seasoning obtained by fermenting beans or wheat) is also referred to as "usual seasoning-containing food". That is, the "food in which the content of the seasoning obtained by fermenting beans or wheat is reduced" may refer specifically to the same type of food as the usual seasoning-containing food, in which the content of the seasoning obtained by fermenting beans or wheat is lower than that in the usual seasoning-containing food. More specifically, the "food in which the content of the seasoning obtained by fermenting beans or wheat is reduced" may refer to the same type of food as the usual seasoning-containing food, in which the content of the seasoning obtained by fermenting beans or wheat is 0.9 times or less, 0.8 times or less, 0.7 times or less, 0.6 times or less, or 0.5 times or less, of that in the usual seasoning-containing food. The food in which the content of the seasoning obtained by fermenting beans or wheat is reduced, may be, for example, food containing a seasoning obtained by fermenting beans or wheat, such as any of the foods exemplified above, which is produced so that the content of the seasoning obtained by fermenting beans or wheat is lower than usual. The usual seasoning-containing food may be, for example, food containing a seasoning obtained by fermenting beans or wheat, such as any of the foods exemplified above, which is produced so as to contain the usual amount of the seasoning obtained by fermenting beans or wheat. The same applies to "food in which the content of the seasoning containing rice malt or miso is reduced" and "food in which the content of the seasoning obtained by fermenting beans or wheat and containing rice malt or miso is reduced". According to the present invention, for example, a decrease in matured flavor due to a reduction in the content of the seasoning obtained by fermenting beans or wheat, the seasoning containing rice malt or miso, or the seasoning obtained by fermenting beans or wheat and containing rice malt or miso, may be compensated. That is, the definition of the "improvement in matured flavor" may include a compensation for a decrease in the matured flavor due to a reduction in the content of the seasoning obtained by fermenting beans or wheat, the seasoning containing rice malt or miso, or the seasoning obtained by fermenting beans or wheat and containing rice malt or miso.

The food according to the present invention may be milk-containing food and/or a dairy product, in which the content of the milk and/or the dairy product is/are reduced. The "food in which the content of the milk and/or the dairy product is reduced" refers to food in which the content of the milk and/or the dairy product is lower than usual (namely, food in which the content of the milk and/or the dairy product is lower than the usual content of the milk and/or the dairy product). The food containing the usual amount of milk and/or a dairy product (namely, food having the usual content of milk and/or the dairy product) is also referred to as "usual milk and/or dairy product-containing food". That is, the "food in which the content of the milk and/or the dairy product is reduced" may refer specifically to the same type of food as the usual milk and/or dairy product-containing food, in which the content of the milk and/or the dairy product is lower than that in the usual milk and/or dairy product-containing food. More specifically, the "food in which the content of the milk and/or the dairy product is reduced" may refer to the same type of food as the usual milk and/or dairy product-containing food, in which the content of the milk and/or the dairy product is 0.9 times or less, 0.8 times or less, 0.7 times or less, 0.6 times or less, or 0.5 times or less, of that in the usual milk and/or dairy product-containing food. The food in which the content of the milk and/or the dairy product is reduced, may be, for example, milk-containing food and/or a dairy product, such as any of the foods exemplified above, which is produced so that the content of the milk and/or the dairy product is lower than usual. The usual milk and/or dairy product-containing food may be, for example, milk-containing food and/or a dairy product, such as any of the foods exemplified above, which is produced so as to contain the usual amount of the milk and/or the dairy product. According to the present invention, for example, a decrease in the matured flavor due to a reduction in the content of the milk and/or the dairy product may be compensated. That is, the definition of the "improvement in matured flavor" may include a compensation for a decrease in the matured flavor due to a reduction in the content of the milk and/or the dairy product.

The method according to the present invention may include a step of producing the active ingredient, before a step of using the active ingredient (for example, a step of adding the active ingredient to a raw material of the food). The active ingredient to be used in the method according to the present invention may be one produced by the above-described method of producing the active ingredient. Accordingly, the step of producing the active ingredient in the method according to the present invention may be a step of producing the active ingredient by the above-described method of producing the active ingredient. Specifically, the step of producing the active ingredient, in the method according to the present invention, may be a step of culturing yeast in a culture medium containing a plant belonging to the family *Solanaceae,* to obtain a cultured product. The "cultured product" described above is also referred to as "fermented product".

The method according to the present invention may include a step of producing the composition according to the present invention, before a step of using the active ingredient (for example, a step of adding the active ingredient to a raw material of the food).

### 4. Use of Active Ingredient

The present invention discloses the use of the active ingredient in any of the applications exemplified above. That is, the present invention discloses, for example, the use of the active ingredient for improving a flavor of food or producing food, or the use of the active ingredient in the production of a composition for improving a flavor of food or producing food.

Further, the present invention discloses the active ingredient for use in any of the applications exemplified above. That is, the present invention discloses, for example, the active ingredient for use in the improvement of the flavor of food or the production of food, or the active ingredient for use in the production of a composition for improving a flavor of food or producing food.

### EXAMPLES

The present invention will now be described more specifically, with reference to non-limiting examples.

### Example 1 Evaluation of Impact of Differences in Nitrogen Sources on Tomato-like Flavor Improving Effect by Yeast-Fermented Liquid

Salt-tolerant yeast, *Zygosaccharomyces rouxii* (NBRC 1130) was used as the yeast. First, yeast cells from a glycerol stock were seeded on a growth plate (containing 1% yeast extract, 1% peptone, 1% glucose and 2% agar), and subjected to static culture at 30°C for 48 hours, to grow the cells on the plate. Yeast bacterial cells were scraped using a loop, from the plate in which the cells had been grown, and suspended in saline (salt: 0.85%) to prepare a seeding liquid to be seeded in each main fermentation culture medium. Subsequently, each culture medium was prepared at the composition shown in Table 1, poured into a 500-mL Sakaguchi flask in an amount of 100 mL per flask, and sterilized at 80°C for 30 minutes, to prepare each main fermentation culture medium. A quantity of 0.6 mL of the seeding liquid was added to 100 mL of each main fermentation culture medium, and main fermentation was carried out by reciprocal shaking culture at 30°C and 120 rpm for 118 hours, to obtain each yeast-fermented liquid.

The fermentation results are shown in Table 2. The generation of 2-phenylethanol (2-PE), ethyl acetate and diacetyl, which are known as the constituent components of tomato flavor, was confirmed in the yeast-fermented liquid, regardless of the type of the nitrogen source used.

Each yeast-fermented liquid was added to RAGU Traditional Pasta Sauce (manufactured by Mizkan Ltd.), at a concentration of 0.1%, and four experts performed the sensory evaluation of the tomato-like-flavor-improving effect by each yeast-fermented liquid. The fresh flavor of tomato and the ripe flavor of tomato were evaluated as the tomato-like flavor. The evaluation criteria were as follows. The score was determined by the consensus of the four experts.
++: There is a strong effect of improving the fresh flavor and the ripe flavor of tomato (this assumes that there is an effect at a degree clearly distinguishable even by a general panel of people who are not experts in sensory evaluation).
+: There is an effect of improving the fresh flavor and the ripe flavor of tomato (this assumes that there is an effect at a degree which is clearly distinguishable by the experts of sensory evaluation, but which is difficult to be distinguished by a general panel of people who are not experts in sensory evaluation).

The results of the sensory evaluation are shown in Table 3. For convenience of explanation, the sample in which tomato is used as the nitrogen source is described as "Positive Control". It has been confirmed that the fresh flavor of tomato and the ripe flavor of tomato are increased by the addition of the yeast-fermented liquid, regardless of the type of the nitrogen source used. In particular, the highest tomato-like-flavor-improving effect was observed in cases where the yeast-fermented liquid using tomato as the nitrogen source was added. There is a possibility that components such as 2-PE, ethyl acetate and diacetyl which are generated when a plant belonging to the family *Solanaceae,* such as tomato, is fermented with yeast, contribute to the tomato-like-flavor-improving effect.

**[Table 1]**

| Table 1 Culture medium composition | | | |
|---|---|---|---|
| Sample | Tomato | Red bell pepper | Soy sauce |
| City water (%) | 81.85 | 81.85 | 81.85 |
| Glucose (%) | 6.0 | 6.0 | 6.0 |
| L-Phe (%) | 0.15 | 0.15 | 0.15 |
| Salt (%) | 8.0 | 8.0 | 8.0 |
| Nitrogen source (%) | Tomato paste 4.0 | Red bell pepper paste 4.0 | Soy sauce 4.0 |
| Total | 100.00 | 100.00 | 100.00 |

**[Table 2]**

| Table 2 Fermentation results | | | |
|---|---|---|---|
| Sample | Tomato | Red bell pepper | Soy sauce |
| OD (620nm) | 0.893 | 0.859 | 0.727 |
| Residual Sugar (g/L) | 0.78 | 0 | 0 |
| 2-PE (ppm) | 985.0 | 1014.5 | 846.5 |
| Ethyl acetate (ppm) | 19.1 | 19.8 | 13.7 |
| Diacetyl (ppm) | 7.6 | 6.6 | 7.2 |

**[Table 3]**

| Table 3 Results of sensory evaluation | | |
|---|---|---|
| Sample | Evaluation | Comment |
| Tomato | + + | Positive Control |
| Red bell pepper | + | Weaker than Positive Control, but has function |
| Soy sauce | + | Weaker than Positive Control, but has function |

### Example 2 Evaluation of Impact of Differences in Yeast Types on Tomato-like Flavor Improving Effect by Yeast-Fermented Liquid

Three strains of yeast, namely, a budding yeast *Saccharomyces cerevisiae* (NBRC 10217), a fission yeast *Schizosaccharomyces pombe* (NBRC 1628) and salt-tolerant yeast *Zygosaccharomyces rouxii* (NBRC 1130) were used as the yeasts. First, each type of yeast cells from a glycerol stock were seeded on a growth plate (containing 1% yeast extract, 1% peptone, 1% glucose, and 2% agar), and subjected to static culture at 30°C for 48 hours, to grow the cells on the plate. Yeast bacterial cells of each type were scraped using a loop, from each plate in which the cells had been grown, and suspended in saline (salt: 0.85%) to prepare each seeding liquid to be seeded in a main fermentation culture medium. Subsequently, a culture medium was prepared at the composition shown in Table 4, poured into a 500-mL Sakaguchi flask in an amount of 100 mL per flask, and sterilized at 80°C for 30 minutes, to prepare a main fermentation culture medium. A quantity of 0.6 mL of each seeding liquid was added to 100 mL of the main fermentation culture medium, and main fermentation was carried out by reciprocal shaking culture at 30°C and 120 rpm for 66 hours.

The fermentation results are shown in Table 5. The generation of 2-phenylethanol (2-PE), ethyl acetate and diacetyl, which are known as the constituent components of tomato flavor, was confirmed in the yeast-fermented liquid, regardless of the type of the yeast used.

Each fermented liquid was added to RAGU Traditional Pasta Sauce (manufactured by Mizkan Ltd.), at a concentration of 0.1%, and four experts performed the sensory evaluation of the tomato-like-flavor-improving effect by each yeast-fermented liquid. The fresh flavor of tomato and the ripe flavor of tomato were evaluated as the tomato-like flavor. The sensory evaluation was performed for the fresh flavor and the ripe flavor of tomato, each based on 5-point scale, and the evaluation criteria were as follows. The score was determined by the consensus of the four experts.
5 points: There is an extremely strong effect of improving the fresh flavor or the ripe flavor of tomato (this assumes that there is an effect at a degree clearly distinguishable even by a general panel of people who are not experts in sensory evaluation, which is a particularly strong effect).
3 points: There is an effect of improving the fresh flavor or the ripe flavor of tomato (this assumes that there is an effect at a degree which is clearly distinguishable by the experts of sensory evaluation, but which is difficult to be distinguished by a general panel of people who are not experts in sensory evaluation).
1 point: There is a very slight effect of improving the fresh flavor or the ripe flavor of tomato (this assumes that there is an effect at a degree which is difficult to be distinguished even by the experts of sensory evaluation).
0 points: There is no effect of improving the fresh flavor or the ripe flavor of tomato.

The results of the sensory evaluation are shown in Table 6. It has been confirmed that the fresh flavor of tomato is increased by the addition of the yeast-fermented liquid, regardless of the type of the yeast used. Further, in cases where Z. *rouxii* or *S*. *cerevisiae* was used, it has been confirmed that the ripe flavor of tomato is increased by the addition of the yeast-fermented liquid. In cases where the fermented liquid of Z. *rouxii* was added, in particular, a high improving effect was observed for both the fresh flavor of tomato and the ripe flavor of tomato. There is a possibility that components such as 2-PE, ethyl acetate and diacetyl which are generated when a plant belonging to the family *Solanaceae,* such as tomato, is fermented with yeast, contribute to the tomato-like-flavor-improving effect.

**[Table 4]**

| Table 4 Culture medium composition | |
|---|---|
| City water (%) | 88.35 |
| Glucose (%) | 6.0 |
| L-Phe (%) | 0.15 |
| Salt (%) | 1.5 |
| Nitrogen source (%) | Tomato paste 4.0 |
| Total | 100.00 |

**[Table 5]**

| Table 5 Fermentation results | | | |
|---|---|---|---|
| Yeast type | *Z. rouxii* | *S. cerevisiae* | *S. pombe* |
| OD (620nm) | 0.402 | 0.269 | 0.346 |
| Residual sugar (g/L) | 0 | 0 | 0 |
| 2-PE (ppm) | 1081.3 | 1003.5 | 347.2 |
| Ethyl acetate (ppm) | 5.7 | 3.5 | 4.0 |
| Diacetyl (ppm) | 4.0 | 4.2 | 3.4 |

**[Table 6]**

| Table 6 Results of sensory evaluation | | |
|---|---|---|
| Yeast type | Fresh flavor of tomato | Ripe flavor of tomato |
| *Z. rouxii* | 5 | 5 |
| *S. cerevisiae* | 1 | 3 |
| *S. pombe* | 1 | 0 |

### Example 3 Confirmation of Effect of Adding L-phenylalanine (L-Phe)

Salt-tolerant yeast, *Zygosaccharomyces rouxii* (NBRC 1130) was used as the yeast. First, yeast cells from a glycerol stock were seeded on a growth plate (containing 1% yeast extract, 1% peptone, 1% glucose, and 2% agar), and subjected to static culture at 30°C for 48 hours, to grow the cells on the plate. Yeast bacterial cells were scraped using a loop, from the plate in which the cells had been grown, and suspended in saline (salt: 0.85%) to prepare a seeding liquid to be seeded in each main fermentation culture medium. Subsequently, each culture medium was prepared at the composition shown in Table 7, poured into a 500-mL Sakaguchi flask in an amount of 100 mL per flask, and sterilized at 80°C for 30 minutes, to prepare each main fermentation culture medium. Four types of culture media in which the added amounts of L-Phe are 0% (not added), 0.05%, 0.1% and 0.15% were prepared as the main fermentation culture media. A quantity of 0.6 mL of the seeding liquid was added to 100 mL of each main fermentation culture medium, and main fermentation was carried out by reciprocal shaking culture at 30°C and 120 rpm for 74 hours, to obtain each yeast-fermented liquid.

The fermentation results are shown in Table 8. The generation of 2-PE and ethyl acetate, which are known as the constituent components of tomato flavor, was confirmed, regardless of the main fermentation culture medium used. Further, increases in the generated amounts of 2-PE and ethyl acetate were confirmed in a manner dependent on the added amount of L-Phe at the time of fermentation.

Each yeast-fermented liquid or each main fermentation culture medium before the fermentation (before seeding the yeast cells) was added to RAGU Traditional Pasta Sauce (manufactured by Mizkan Ltd.), each at a concentration of 0.1%, and four experts performed the sensory evaluation of the tomato-like-flavor-improving effect by each yeast-fermented liquid. The fresh flavor of tomato and the ripe flavor of tomato were evaluated as the tomato-like flavor. The evaluation criteria were as follows. The score was determined by the consensus of the four experts.
++++: There is an extremely strong effect of improving the fresh flavor and the ripe flavor of tomato (this assumes that there is an effect at a degree clearly distinguishable even by a general panel of people who are not experts in sensory evaluation, which is a particularly strong effect (an effect stronger than ++).
++: There is a strong effect of improving the fresh flavor and the ripe flavor of tomato (this assumes that there is an effect at a degree clearly distinguishable even by a general panel of people who are not experts in sensory evaluation).
+: There is an effect of improving the fresh flavor and the ripe flavor of tomato (this assumes that there is an effect at a degree which is clearly distinguishable by the experts of sensory evaluation, but which is difficult to be distinguished by a general panel of people who are not experts in sensory evaluation).
-: There is no effect of improving the fresh flavor and the ripe flavor of tomato.

The results of the sensory evaluation are shown in Tables 9 and 10. It has been confirmed that the tomato-like flavor (the fresh flavor of tomato and the ripe flavor of tomato) is increased (Table 9), regardless of the main fermentation culture medium used. Further, an increase in the tomato-like-flavor-improving effect was confirmed in a manner dependent on the added amount of L-Phe at the time of fermentation. In particular, a high tomato-like-flavor-improving effect was obtained under the condition in which the fermentation was performed with the addition of 0.1% or more of L-Phe (Table 9). In contrast, in the case of adding each culture medium before the fermentation (before seeding the yeast cells), no effect of improving the tomato-like flavor was observed (Table 10). The above results have revealed that it is essential to ferment a plant belonging to the family *Solanaceae,* such as tomato, with yeast, for improving the tomato-like flavor, regardless of whether or not L-Phe is added at the time of fermentation. There is a possibility that components such as 2-PE and ethyl acetate which are generated when a plant belonging to the family *Solanaceae,* such as tomato, is fermented with yeast, contribute to the tomato-like-flavor-improving effect.

**[Table 7]**

| Table 7 Culture medium composition | | | | |
|---|---|---|---|---|
| No. | 1 | 2 | 3 | 4 |
| City water (%) | 88.50 | 88.45 | 88.40 | 88.35 |
| Glucose (%) | 6.0 | 6.0 | 6.0 | 6.0 |
| L-Phe (%) | 0 | 0.05 | 0.10 | 0.15 |
| Salt (%) | 1.5 | 1.5 | 1.5 | 1.5 |
| Nitrogen source (%) | Tomato paste 4.0 | Tomato paste 4.0 | Tomato paste 4.0 | Tomato paste 4.0 |
| Total | 100.00 | 100.00 | 100.00 | 100.00 |

**[Table 8]**

| Table 8 Fermentation results | | | | |
|---|---|---|---|---|
| No. | 1 | 2 | 3 | 4 |
| Added concentration of L-Phe (%) | 0 | 0.05 | 0.10 | 0.15 |
| OD (620nm) | 0.926 | 0.86 | 0.919 | 0.895 |
| Residual Sugar (g/L) | 0 | 0 | 0 | 0 |
| L-Phe (g/L) | 0.04 | 0.04 | 0.04 | 0.16 |
| 2-PE (ppm) | 9.5 | 364.3 | 711.8 | 967.4 |
| Ethyl acetate (ppm) | 5.4 | 8.4 | 12.3 | 10.4 |

**[Table 9]**

| Table 9 Results of sensory evaluation (after fermentation) | | | | |
|---|---|---|---|---|
| No. | 1 | 2 | 3 | 4 |
| Added concentration of L-Phe (%) | 0 | 0.05 | 0.1 | 0.15 |
| Evaluation | + | ++ | ++++ | ++++ |

**[Table 10]**

| Table 10 Results of sensory evaluation (before fermentation) | | | | |
|---|---|---|---|---|
| No. | 1 | 2 | 3 | 4 |
| Added concentration of L-Phe (%) | 0 | 0.05 | 0.1 | 0.15 |
| Evaluation | - | Not performed | Not performed | - |

### Example 4 Confirmation of Effect of Improving Spicy Flavor

(1) Salt-tolerant yeast, *Zygosaccharomyces rouxii* was used as the yeast. First, a frozen stock containing yeast bacterial cells was seed-cultured. The resulting seed culture liquid was added to the main fermentation culture medium of Example 1 shown in Table 1 in which tomato paste was used as the nitrogen source, at a concentration of 0.5%, and main fermentation was carried out at 30°C and 65 Hz for 64 hours, to obtain a yeast-fermented liquid. As a result of the fermentation, the generation of 2-PE and ethyl acetate, which are known as the constituent components of tomato flavor, was confirmed. To 89.3% of the resulting yeast-fermented liquid, 5.1% of starch (Potato Starchride AH-5, manufactured by Matsutani Chemical Industry Co., Ltd.) and 5.6% of dextrin (Sandec #70, manufactured by Sanwa Starch Co., Ltd.) were added and dissolved, and the resulting solution was dried using a drum dryer, followed by crushing to obtain a yeast-fermented liquid powder. The content of the active ingredient (namely, the yeast-fermented product of a plant belonging to the family *Solanaceae)* in the resulting yeast-fermented liquid powder was 500% (w/w), in terms of the content of the original cultured product (namely, the yeast-fermented liquid).

The yeast-fermented liquid powder, 2-PE or a yeast extract was added to European Beef Curry, in the amount shown in Table 11, and six experts performed the sensory evaluation of the spicy flavor-improving effect by each material. The pungency and the stimulus intensity, as well as the refreshing flavor and the gorgeousness imparted by spices, were evaluated as the spicy flavor. The evaluation criteria were as follows. The score was determined by the consensus of the six experts.
++++: There is an extremely strong effect of improving the spicy flavor (this assumes that there is an effect at a degree clearly distinguishable even by a general panel of people who are not experts in sensory evaluation, which is a particularly strong effect (an effect stronger than ++).
++: There is a strong effect of improving the spicy flavor (this assumes that there is an effect at a degree clearly distinguishable even by a general panel of people who are not experts in sensory evaluation).
+: There is an effect of improving the spicy flavor (this assumes that there is an effect at a degree which is clearly distinguishable by the experts of sensory evaluation, but which is difficult to be distinguished by a general panel of people who are not experts in sensory evaluation).
-: There is no effect of improving the spicy flavor.

The results of the sensory evaluation are shown in Table 11. It has been confirmed that the addition of the yeast-fermented liquid powder causes a marked increase in the spicy flavor, as compared to the case of adding 2-PE or any one of the yeast extracts. By adding the yeast-fermented liquid powder, the spicy flavor stood out from the initial taste, the spicy flavor is enhanced from the initial taste to the middle taste, and an improvement in the spicy flavor continued even from the middle taste to the aftertaste. In addition, not only the pungency and the stimulus intensity of the spicy flavor, but also the refreshing, gorgeous and fruity spicy flavor were enhanced, and the richness and thickness were also imparted.

### [Table 11]

**Table 11**

| Material | Added concentration | Evaluation of spicy flavor |
|---|---|---|
| Yeast-fermented liquid powder | 0.15% | ++++ |
| 2-PE | 0.05% | + to ++ |
| Yeast Extract (*1) | 0.10% | ++ |
| Yeast Extract (*2) | 0.10% | ++ |
| Yeast Extract (*3) | 0.10% | ++ |

| | | |
|---|---|---|
| (*1) Yeastock GT-Pd (manufactured by Asahi Group Foods, Ltd.) (*2) Yeast extract powder SG010 (manufactured by Angel Yeast Co., Ltd.) (*3) AROMILD UG-8 (manufactured by KOHJIN Life Sciences Co., Ltd.) | | |

(2)
Separately, the yeast-fermented liquid powder was added in the amount shown in Table 12, to each of various commercially available curries varying in the type of tomato raw material, containing or not containing a flavoring agent, and varying in the type of spices, and three experts performed the sensory evaluation of the spicy flavor-improving effect, relative to the control to which the yeast-fermented liquid powder had not been added. The pungency and the stimulus intensity, as well as the refreshing flavor and the gorgeousness imparted by spices, were evaluated as the spicy flavor. The evaluation criteria were the same as those in Example 4 (1). The score was determined by the consensus of the three experts.

The results of the sensory evaluation are shown in Table 12. It has been confirmed that the addition of the yeast-fermented liquid powder causes a marked increase in the spicy flavor as compared to the control to which the yeast-fermented liquid powder is not added, regardless of the type of the curry. By adding the yeast-fermented liquid powder, an enhancement in the refreshing spicy flavor, and enhancements in the fruity flavor of tomato and the spicy flavor have been confirmed.

### [Table 12]

**Table 12**

| No. | 1 | 2 | 3 |
|---|---|---|---|
| Tomato raw material | Tomato processed product | Tomato paste | Tomato paste |
| Spices, etc. | curry roux, fruit chutney, spice processed food, cumin, curry powder, coriander, cardamom, mustard, caraway, cinnamon, fennel, basil, saffron-flavored seasoning, lemongrass | sauteed curry paste, garlic paste, chutney, spices, garlic powder/ spice extracts | spices |
| With or without flavoring agent | No | Yes | Yes |
| Addition rate | 0.15% | 0.15% | 0.15% |
| Evaluation of spicy flavor | + + + + | ++++ | ++++ |

| | | | |
|---|---|---|---|
| No. 1. "European Beef Curry with bursting aroma of spices, supervised by TOMATO in Ogikubo" (manufactured by Musashino Co., Ltd.) No. 2. "European Beef Curry - Beef and Bouillon Flavor" (manufactured by House Foods, Co., Ltd.), * a retort curry No. 3. "Soy Meat Keema Curry" (manufactured by S&B Foods Inc.) | | | |

(3)
Separately, the yeast-fermented liquid powder was added to Sichuan style Mapo tofu, at a concentration of 0.2%, and eight experts performed the sensory evaluation of the spicy flavor-improving effect, relative to the control to which the yeast-fermented liquid powder had not been added. The pungency and the stimulus intensity, as well as the refreshing flavor and the gorgeousness imparted by spices, were evaluated as the spicy flavor. As a result of the evaluation, a marked effect of improving the spicy flavor was observed in the case of adding the yeast-fermented liquid powder, as compared to the control to which the yeast-fermented liquid powder had not been added. In particular, the flavor of Sichuan pepper was enhanced by the addition of the yeast-fermented liquid powder.

### Example 5 Confirmation of Effect of Improving Matured flavor

The yeast-fermented liquid powder was obtained in the same manner as in Example 4. The yeast-fermented liquid powder was added to Sichuan style Mapo rice bowl, at a concentration of 0.05%, 0.1%, 0.2% or 0.3%, and four experts performed the sensory evaluation of the matured flavor-improving effect, relative to the control to which the yeast-fermented liquid powder had not been added. The matured aroma, flavor and richness of douchi, tianmianjiang and doubanjiang were evaluated as the matured flavor.

A marked effect of improving the matured flavor was observed regardless of the concentration of the yeast-fermented liquid powder added to the Sichuan style Mapo rice bowl, as compared to the control to which the yeast-fermented liquid powder had not been added. In particular, the flavor of douchi was enhanced by the addition of the yeast-fermented liquid powder.

### Example 6 Evaluation of Heat Resistance of Tomato-like Flavor Improving Effect

(1) The yeast-fermented liquid was obtained in the same manner as in Example 4. The yeast-fermented liquid or a commercially available tomato-flavored seasoning was added to 100 g of RAGU Tomato Sauce, in the amount shown in Table 13, filled into a retort pouch, and heated at 121°C for 10 minutes. Thereafter, each retort pouch was stored for 10 days in an airtight container, and then three experts performed the sensory evaluation of the tomato-like-flavor-improving effect, relative to the control which had been subjected to the same retort heat treatment without adding both the yeast-fermented liquid and the commercially available tomato-flavored seasoning. The score was determined by the consensus of the three experts.
   ++++: There is an extremely strong effect of improving the tomato-like flavor (this assumes that there is an effect at a degree clearly distinguishable even by a general
   panel of people who are not experts in sensory evaluation, which is a particularly strong effect (an effect stronger than ++).
   ++: There is a strong effect of improving the tomato-like flavor (this assumes that there is an effect at a degree clearly distinguishable even by a general panel of people who are not experts in sensory evaluation).
   +: There is an effect of improving the tomato-like flavor (this assumes that there is an effect at a degree which is clearly distinguishable by the experts of sensory evaluation, but which is difficult to be distinguished by a general panel of people who are not experts in sensory evaluation).
   -: There is no effect of improving the tomato-like flavor.

The results of the sensory evaluation are shown in Table 13. It has been confirmed that the addition of the yeast-fermented liquid causes a marked increase in the tomato-like flavor, as compared to the case of adding a commercially available tomato-flavored seasoning. Specifically, when the yeast-fermented liquid was added, the tomato-like fresh flavor was enhanced as compared to the case of adding another tomato-flavored seasoning, and the characteristics that the acidity and the tomato-like flavor stand out from the initial taste were observed. The above results have shown that a yeast-fermented product of a plant belonging to the family *Solanaceae,* such as tomato, exhibits a marked effect of improving the tomato-like flavor even after the heat treatment and storage. Further, since the fermented product of tomato fermented with *a Zygosaccharomyces rouxii* strain other than NBRC 1130 also exhibited the tomato-like-flavor-improving effect, it has been suggested that a fermented product of a plant belonging to the family *Solanaceae,* such as tomato, with *Zygosaccharomyces rouxii* exhibits the flavor-improving effect such as the tomato-like-flavor-improving effect, regardless of the type of the strain thereof.

### [Table 13]

**Table 13**

| No. | Added material | Added amount | Evaluation of Tomato-like Flavor |
|---|---|---|---|
| 1 | Yeast-fermented liquid powder | 0.15% | + + |
| 2 | Tomato flavor (manufactured by T. HASEGAWA Co., Ltd.) | 0.015% | + |
| 3 | Tomato extract powder (manufactured by Nikken Foods. Co., Ltd.) | 0.15% | + |

### Example 7 Evaluation of Heat Resistance of Tomato-like Flavor Improving Effect

(2)
The yeast-fermented liquid powder was obtained in the same manner as in Example 4. The yeast-fermented liquid powder was added to a tomato sauce or a hashed beef-style sauce, at a concentration of 0.05%, filled into a retort pouch, and heated at 121°C for 10 minutes. After cooling, two experts performed the sensory evaluation of the tomato-like-flavor-improving effect, relative to the control which had been subjected to the same retort heat treatment without adding the yeast-fermented liquid powder. As a result of the evaluation, an improvement in the tomato-like flavor has been confirmed by the addition of the yeast-fermented liquid powder, in each of the tomato sauce and the hashed beef-style sauce. When the yeast-fermented liquid powder was added, the tomato-like flavor as if the sauce had been concentrated was recognized in each of the tomato sauce and the hashed beef-style sauce, as compared to the control to which the yeast-fermented liquid powder had not been added, even after being subjected to the retort heat treatment, and the overall ripe flavor of tomato was enhanced.

### Example 8 Evaluation of Impact of Culture Supernatant and Yeast Bacterial Cells on Effect of Improving Tomato-like Flavor, Spicy Flavor and Matured Flavor

(1) The yeast-fermented liquid was obtained in the same manner as in Example 4, except that salt-tolerant yeast *Zygosaccharomyces rouxii* (NBRC 1130) was used as the yeast. A portion of the resulting yeast-fermented liquid was separated into the supernatant and precipitates by centrifugation. The resulting precipitates were suspended in the main fermentation culture medium in an amount equal to that of the main fermentation culture medium used for fermentation, to obtain a yeast bacterial cell dispersion liquid.

Further, to the main fermentation culture medium of Example 1 shown in Table 1 in which tomato paste was used as the nitrogen source, the resulting supernatant, yeast bacterial cell dispersion liquid or yeast-fermented liquid, starch and dextrin were added, dissolved, dried and then crushed, to obtain each powder, in the same manner as in Example 4. The resulting powders are also referred to as "main fermentation culture medium powder", "supernatant powder", "yeast bacterial cell dispersion liquid powder" and "yeast-fermented liquid powder 1", respectively.

Separately, a yeast-fermented liquid powder (also referred to as "yeast-fermented liquid powder 2") was obtained in the same manner as in Example 4.

The main fermentation culture medium powder, the supernatant powder, the yeast bacterial cell dispersion liquid powder, the yeast-fermented liquid powder 1 or the yeast-fermented liquid powder 2 was added to a tomato soup, in the amount shown in Table 14, and five experts performed the sensory evaluation of the tomato-like-flavor-improving effect by each material. The fresh flavor of tomato and the ripe flavor of tomato were each evaluated as the tomato-like flavor. The evaluation was carried out in accordance with the following evaluation criteria, on a scale from 1.0 to 5.0 points with an increment of 0.5 points, taking the tomato-like flavor of the tomato soup with no added material as 1.0 point. The average value of the evaluation results by the five experts was taken as the final score.
5.0 points: There is an extremely strong effect of improving the tomato-like flavor (this assumes that there is an effect at a degree clearly distinguishable even by a general panel of people who are not experts in sensory evaluation, which is a particularly strong effect (an effect stronger than 4.5 points).
4.5 points: There is an extremely strong effect of improving the tomato-like flavor (this assumes that there is an effect at a degree clearly distinguishable even by a general panel of people who are not experts in sensory evaluation, which is a particularly strong effect (an effect stronger than 4.0 points).
4.0 points: There is an extremely strong effect of improving the tomato-like flavor (this assumes that there is an effect at a degree clearly distinguishable even by a general panel of people who are not experts in sensory evaluation, which is a particularly strong effect (an effect stronger than 3.5 points).
3.5 points: There is a strong effect of improving the tomato-like flavor (this assumes that there is an effect at a degree clearly distinguishable even by a general panel of people who are not experts in sensory evaluation, which is a strong effect (an effect stronger than 3.0 points).
3.0 points: There is a strong effect of improving the tomato-like flavor (this assumes that there is an effect at a degree clearly distinguishable even by a general panel of people who are not experts in sensory evaluation).
2.5 points: There is an improvement effect (this assumes that there is an effect at a degree which is clearly distinguishable by the experts of sensory evaluation, namely, an effect stronger than 2.0 points, but which is difficult to be distinguished by a general panel of people who are not experts in sensory evaluation).
2.0 points: There is an effect of improving the tomato-like flavor (this assumes that there is an effect at a degree which is clearly distinguishable by the experts of sensory evaluation, but which is difficult to be distinguished by a general panel of people who are not experts in sensory evaluation).
1.5 points: There is a slight effect of improving the tomato-like flavor (this assumes that there is an effect at a degree which is distinguishable by the experts of sensory evaluation, but which is difficult to be distinguished by a general panel of people who are not experts in sensory evaluation).
1.0 point: There is no improvement effect.

The results of the sensory evaluation are shown in Table 14. Although an improvement in the tomato-like flavor (the fresh flavor of tomato and the ripe flavor of tomato) was confirmed regardless of the material added, a marked increase in the tomato-like flavor was confirmed when the yeast-fermented liquid powder 1 or the yeast-fermented liquid powder 2 was added, as compared to the case of adding another material. This has shown that a more marked effect of improving the tomato-like flavor can be obtained, when not only the culture supernatant portion, which is thought to contain a large amount of the fermented product, but also the yeast bacterial cells were added.

### [Table 14]

**Table 14**

| No. | 1 | 2 | 3 | 4 | 5 | 6 |
|---|---|---|---|---|---|---|
| Added material | - | Main fermentation culture medium powder | Supernatant powder | Yeast bacterial cell dispersion liquid powder | Yeast-fermented liquid powder 1 | Yeast-fermented liquid powder 2 |
| With or without fermentation | - | - | ○ | - | ○ | ○ |
| Presence or absence of yeast bacteria cells | - | - | - | ○ | ○ | ○ |
| Added amount | - | 0.01% | 0.01% | 0.01% | 0.01% | 0.01% |
| Fresh flavor of tomato | 1.0 | 1.5 | 2.5 | 1.2 | 4.2 | 4.5 |
| Ripe flavor of tomato | 1.0 | 1.9 | 1.4 | 2.2 | 3.5 | 3.7 |

(2)
Separately, the main fermentation culture medium powder, the supernatant powder, the yeast bacterial cell dispersion liquid powder, the yeast-fermented liquid powder 1 or the yeast-fermented liquid powder 2 was added to Sichuan style Mapo tofu, in the amount shown in Table 15, and four experts performed the sensory evaluation of each of the spicy flavor-improving effect and the matured flavor-improving effect by each material. The pungency and the stimulus intensity, as well as the refreshing flavor and the gorgeousness imparted by spices, were evaluated as the spicy flavor. The matured aroma, flavor and richness of douchi, tianmianjiang and doubanjiang were evaluated as the matured flavor. The evaluation was carried out in accordance with the following evaluation criteria, on a scale from 1.0 to 5.0 points with an increment of 0.5 points, taking the spicy flavor and the matured flavor of the Sichuan style Mapo tofu with no added material as 1.0 point, respectively. The average value of the evaluation results by the four experts was taken as the final score.
5.0 points: There is an extremely strong improvement effect (this assumes that there is an effect at a degree clearly distinguishable even by a general panel of people who are not experts in sensory evaluation, which is a particularly strong effect (an effect stronger than 4.5 points).
4.5 points: There is an extremely strong improvement effect (this assumes that there is an effect at a degree clearly distinguishable even by a general panel of people who are not experts in sensory evaluation, which is a particularly strong effect (an effect stronger than 4.0 points).
4.0 points: There is an extremely strong improvement effect (this assumes that there is an effect at a degree clearly distinguishable even by a general panel of people who are not experts in sensory evaluation, which is a particularly strong effect (an effect stronger than 3.5 points).
3.5 points: There is a strong improvement effect (this assumes that there is an effect at a degree clearly distinguishable even by a general panel of people who are not experts in sensory evaluation, which is a strong effect (an effect stronger than 3.0 points).
3.0 points: There is a strong improvement effect (this assumes that there is an effect at a degree clearly distinguishable even by a general panel of people who are not experts in sensory evaluation).
2.5 points: There is an improvement effect (this assumes that there is an effect at a degree which is clearly distinguishable by the experts of sensory evaluation, namely, an effect stronger than 2.0 points, but which is difficult to be distinguished by a general panel of people who are not experts in sensory evaluation).
2.0 points: There is an improvement effect (this assumes that there is an effect at a degree which is clearly distinguishable by the experts of sensory evaluation, but which is difficult to be distinguished by a general panel of people who are not experts in sensory evaluation).
1.5 points: There is a slight improvement effect (this assumes that there is an effect at a degree which is distinguishable by the experts of sensory evaluation, but which is difficult to be distinguished by a general panel of people who are not experts in sensory evaluation).
1.0 point: There is no improvement effect.

The results of the sensory evaluation are shown in Table 15. Although improvements in the spicy flavor and the matured flavor were confirmed regardless of the material added, marked increases in the spicy flavor and the matured flavor were confirmed when the yeast-fermented liquid powder 1 or the yeast-fermented liquid powder 2 was added, as compared to the case of adding another material. This has shown that a more marked effect of improving the spicy flavor and the matured flavor can be obtained, when not only the culture supernatant portion, which is thought to contain a large amount of the fermented product, but also the yeast bacterial cells were added.

### [Table 15]

**Table 15**

| No. | 1 | 2 | 3 | 4 | 5 | 5 |
|---|---|---|---|---|---|---|
| Added material | - | Main fermentation culture medium powder | Supernatant powder | Yeast bacterial cell dispersion liquid powder | Yeast-fermented liquid powder 1 | Yeast-fermented liquid powder 2 |
| With or without fermentation | - | - | ○ | - | ○ | ○ |
| Presence or absence of yeast bacteria cells | - | - | - | ○ | ○ | ○ |
| Added amount | - | 0.01% | 0.01% | 0.01% | 0.01% | 0.01% |
| Spicy flavor | 1.0 | 1.4 | 2.5 | 2.3 | 4.5 | 4.6 |
| Matured flavor | 1.0 | 1.4 | 2.5 | 2.3 | 4.3 | 4.6 |

### Example 9 Evaluation of Impact of With or Without Fermentation of Plant Belonging to Family Solanaceae with Yeast on Effect of Improving Tomato-like Flavor, Spicy Flavor and Matured Flavor

(1) The yeast-fermented liquid powder was obtained in the same manner as in Example 4.

The yeast-fermented liquid powder or a powder seasoning containing commercially available yeast bacterial cells was added to a tomato soup, in the amount shown in Table 16, and five experts performed the sensory evaluation of the tomato-like-flavor-improving effect by each material. The fresh flavor of tomato and the ripe flavor of tomato were each evaluated as the tomato-like flavor. The evaluation was carried out in accordance with the following evaluation criteria, on a scale from 1.0 to 5.0 points with an increment of 0.5 points, taking the tomato-like flavor (the fresh flavor or the ripe flavor) of the tomato soup with no added material as 1.0 point. The average value of the evaluation results by the five experts was taken as the final score.
5.0 points: There is an extremely strong effect of improving the tomato-like flavor (this assumes that there is an effect at a degree clearly distinguishable even by a general panel of people who are not experts in sensory evaluation, which is a particularly strong effect (an effect stronger than 4.5 points).
4.5 points: There is an extremely strong effect of improving the tomato-like flavor (this assumes that there is an effect at a degree clearly distinguishable even by a general panel of people who are not experts in sensory evaluation, which is a particularly strong effect (an effect stronger than 4.0 points).
4.0 points: There is an extremely strong effect of improving the tomato-like flavor (this assumes that there is an effect at a degree clearly distinguishable even by a general panel of people who are not experts in sensory evaluation, which is a particularly strong effect (an effect stronger than 3.5 points).
3.5 points: There is a strong effect of improving the tomato-like flavor (this assumes that there is an effect at a degree clearly distinguishable even by a general panel of people who are not experts in sensory evaluation, which is a strong effect (an effect stronger than 3.0 points).
3.0 points: There is a strong effect of improving the tomato-like flavor (this assumes that there is an effect at a degree clearly distinguishable even by a general panel of people who are not experts in sensory evaluation).
2.5 points: There is an effect of improving the tomato-like flavor (this assumes that there is an effect at a degree which is clearly distinguishable by the experts of sensory evaluation, namely, an effect stronger than 2.0 points, but which is difficult to be distinguished by a general panel of people who are not experts in sensory evaluation).
2.0 points: There is an effect of improving the tomato-like flavor (this assumes that there is an effect at a degree which is clearly distinguishable by the experts of sensory evaluation, but which is difficult to be distinguished by a general panel of people who are not experts in sensory evaluation).
1.5 points: There is a slight effect of improving the tomato-like flavor (this assumes that there is an effect at a degree which is distinguishable by the experts of sensory evaluation, but which is difficult to be distinguished by a general panel of people who are not experts in sensory evaluation).
1.0 point: There is no effect of improving the tomato-like flavor.

The results of the sensory evaluation are shown in Table 16. Although the tomato-like flavor was improved regardless of the material added, a marked increase in the tomato-like flavor was confirmed when the yeast-fermented liquid powder of the salt-tolerant yeast was added, as compared to the case of adding a powder seasoning containing commercially available yeast bacterial cells. This has suggested that a fermented product of a plant belonging to the family *Solanaceae,* such as tomato, with yeast, exhibits the flavor-improving effect such as the tomato-like-flavor-improving effect.

### [Table 16]

**Table 16**

| No. | 1 | 2 | 3 | 4 |
|---|---|---|---|---|
| Added material | No added material | Yeast-fermented liquid powder | Yeast powder (*1) | Yeast powder (*2) |
| Yeast Type | - | Salt-tolerant yeast | Baker's yeast | Torula yeast |
| Addition rate | - | 0.01% | 0.01% | 0.01% |
| Fresh flavor of tomato | 1.0 | 4.2 | 1.8 | 1.4 |
| Ripe flavor of tomato | 1.0 | 3.5 | 1.2 | 2.3 |

| | | | | |
|---|---|---|---|---|
| *1 Hyper Yeast HG-DY (manufactured by Asahi Group Foods, Ltd.) *1 AROMAWAY^{®} (manufactured by Mitsubishi Corporation Life Sciences Limited) | | | | |

(2)
Separately, the yeast-fermented liquid powder or a powder seasoning containing commercially available yeast bacterial cells was added to Sichuan style Mapo tofu, in the amount shown in Table 17, and four experts performed the sensory evaluation of each of the spicy flavor-improving effect and the matured flavor-improving effect by each material. The pungency and the stimulus intensity, as well as the refreshing flavor and the gorgeousness imparted by spices, were evaluated as the spicy flavor. The matured aroma, flavor and richness of douchi, tianmianjiang and doubanjiang were evaluated as the matured flavor. The evaluation was carried out in accordance with the following evaluation criteria, on a scale from 1.0 to 5.0 points with an increment of 0.5 points, taking the spicy flavor and the matured flavor of the Sichuan style Mapo tofu with no added material as 1.0 point, respectively. The average value of the evaluation results by the four experts was taken as the final score.
5.0 points: There is an extremely strong improvement effect (this assumes that there is an effect at a degree clearly distinguishable even by a general panel of people who are not experts in sensory evaluation, which is a particularly strong effect (an effect stronger than 4.5 points).
4.5 points: There is an extremely strong improvement effect (this assumes that there is an effect at a degree clearly distinguishable even by a general panel of people who are not experts in sensory evaluation, which is a particularly strong effect (an effect stronger than 4.0 points).
4.0 points: There is an extremely strong improvement effect (this assumes that there is an effect at a degree clearly distinguishable even by a general panel of people who are not experts in sensory evaluation, which is a particularly strong effect (an effect stronger than 3.5 points).
3.5 points: There is a strong improvement effect (this assumes that there is an effect at a degree clearly distinguishable even by a general panel of people who are not experts in sensory evaluation, which is a strong effect (an effect stronger than 3.0 points).
3.0 points: There is a strong improvement effect (this assumes that there is an effect at a degree clearly distinguishable even by a general panel of people who are not experts in sensory evaluation).
2.5 points: There is an improvement effect (this assumes that there is an effect at a degree which is clearly distinguishable by the experts of sensory evaluation, namely, an effect stronger than 2.0 points, but which is difficult to be distinguished by a general panel of people who are not experts in sensory evaluation).
2.0 points: There is an improvement effect (this assumes that there is an effect at a degree which is clearly distinguishable by the experts of sensory evaluation, but which is difficult to be distinguished by a general panel of people who are not experts in sensory evaluation).
1.5 points: There is a slight improvement effect (this assumes that there is an effect at a degree which is distinguishable by the experts of sensory evaluation, but which is difficult to be distinguished by a general panel of people who are not experts in sensory evaluation).
1.0 point: There is no improvement effect.

The results of the sensory evaluation are shown in Table 17. Although improvements in the spicy flavor and the matured flavor were confirmed regardless of the material added, marked increases in the spicy flavor and the matured flavor were confirmed when the yeast-fermented liquid powder was added, as compared to the case of adding another material. This has suggested that marked improvements in the spicy flavor and the matured flavor can be achieved by the fermentation of a plant belonging to the family *Solanaceae,* such as tomato, with yeast.

### [Table 17]

**Table 17**

| No. | 1 | 2 | 3 | 4 |
|---|---|---|---|---|
| Added material | No added material | Yeast-fermented liquid powder | Yeast powder (*1) | Yeast powder (*2) |
| Yeast Type | - | Salt-tolerant yeast | Baker's yeast | Torula yeast |
| Added amount | - | 0.01% | 0.01% | 0.01% |
| Spicy flavor | 1.0 | 4.5 | 2.6 | 2.6 |
| Matured flavor | 1.0 | 4.3 | 2.8 | 2.8 |

| | | | | |
|---|---|---|---|---|
| *1 Hyper Yeast HG-DY (manufactured by Asahi Group Foods, Ltd.) *1 AROMAWAY^{®} (manufactured by Mitsubishi Corporation Life Sciences Limited) | | | | |

### Example 10 Evaluation of Impact of Added Amount of Yeast-Fermented Liquid Powder to Food on Effect of Improving Tomato-like Flavor, Spicy Flavor and Matured Flavor

(1) The yeast-fermented liquid powder was obtained in the same manner as in Example 4.

The yeast-fermented liquid powder was added in each of the amounts shown in Table 18, to a commercially available potato snack containing cheese raw materials and spices, and five experts performed the sensory evaluation of each of the matured flavor-improving effect and the spicy flavor-improving effect, relative to the control to which the yeast-fermented liquid powder had not been added. The richness and the thickness of a dairy product were evaluated as the matured flavor. The pungency and the stimulus intensity, as well as the refreshing flavor and the gorgeousness imparted by spices, were evaluated as the spicy flavor. The evaluation criteria were as follows. The score was determined by the consensus of the five experts.
++++: There is an extremely strong improvement effect (this assumes that there is an effect at a degree clearly distinguishable even by a general panel of people who are not experts in sensory evaluation, which is a particularly strong effect (an effect stronger than ++).
++: There is a strong improvement effect (this assumes that there is an effect at a degree clearly distinguishable even by a general panel of people who are not experts in sensory evaluation).
+: There is an improvement effect (this assumes that there is an effect at a degree which is clearly distinguishable by the experts of sensory evaluation, but which is difficult to be distinguished by a general panel of people who are not experts in sensory evaluation).
-: There is no improvement effect.

The results of the sensory evaluation are shown in Table 18. In each of the potato snacks, an improvement in the matured flavor could be confirmed at an addition rate of the yeast-fermented liquid powder of 0.00001%, and a more marked improvement could be confirmed at an addition rate of 0.0001% or more. Further, an improvement in the spicy flavor could be confirmed at an addition rate of the yeast-fermented liquid powder of 0.00001%, and an increase in the effect of improving the spicy flavor could be confirmed in a manner dependent on the addition rate of the yeast-fermented liquid powder. This has suggested that it is possible to enhance the matured flavor with cheese-like sweetness, richness and thickness, as well as the spicy flavor with chili and black pepper-like pungency and strong stimulus, by adding 0.00001% or more of the yeast-fermented liquid powder to each potato snack.

### [Table 18]

**Table 18**

| Food | Potato snack 1 (*1) | | | | Potato snack 2 (*2) | | | |
|---|---|---|---|---|---|---|---|---|
| Cheese raw materials | Cheese-flavored flakes, Cheese powder | | | | Cheese-flavored flakes, Cheese powder | | | |
| Spices, etc. | Garlic powder, Red chili pepper, Parsley, Oregano/Spice extracts | | | | Red chili pepper, Garlic powder, Green chili pepper, Habanero/Spice extracts | | | |
| Other raw material | - | | | | Miso powder | | | |
| Added amount | 0.00001% | 0.0001% | 0.001% | 0.01% | 0.00001% | 0.0001% | 0.001% | 0.01% |
| Matured flavor | + | ++++ | ++++ | + +++ | + | ++++ | ++++ | ++++ |
| Spicy flavor | + | + | ++ | ++++ | + | ++ | ++++ | ++++ |

| | | | | | | | | |
|---|---|---|---|---|---|---|---|---|
| *1 "Pizza Potato" (manufactured by Calbee Co. Ltd.) *2 "Hot Pizza Potato" (manufactured by Calbee Co. Ltd.) | | | | | | | | |

(2)
Separately, the yeast-fermented liquid powder was added in each of the amounts shown in Table 19, to a commercially available corn snack containing tomato raw materials and flavoring agents, and five experts performed the sensory evaluation of the tomato-like-flavor-improving effect, relative to the control to which the yeast-fermented liquid powder had not been added. The fresh flavor of tomato and the ripe flavor of tomato were evaluated as the tomato-like flavor. The evaluation criteria were as follows. The score was determined by the consensus of the five experts.
++++: There is an extremely strong improvement effect (this assumes that there is an effect at a degree clearly distinguishable even by a general panel of people who are not experts in sensory evaluation, which is a particularly strong effect (an effect stronger than ++).
++: There is a strong improvement effect (this assumes that there is an effect at a degree clearly distinguishable even by a general panel of people who are not experts in sensory evaluation).
+: There is an improvement effect (this assumes that there is an effect at a degree which is clearly distinguishable by the experts of sensory evaluation, but which is difficult to be distinguished by a general panel of people who are not experts in sensory evaluation).
-: There is no improvement effect.

The results of the sensory evaluation are shown in Table 19. An improvement in the tomato-like flavor could be confirmed at an addition rate of the yeast-fermented liquid powder of 0.00001%, and a clear improvement effect could be confirmed at an addition rate of 0.0001%, and more markedly at an addition rate of 0.001% or more. This has suggested that it is possible to enhance the fresh flavor of tomato with acidity as well as the ripe flavor of tomato with sweetness and umami, by adding 0.00001% or more of the yeast-fermented liquid powder to the corn snack.

### [Table 19]

**Table 19**

| | | | | |
|---|---|---|---|---|
| Food | Chili and tomato-flavored corn snack (*) | | | |
| Tomato raw materials | Tomato-flavored seasoning, Tomato | | | |
| Spices, etc. | Spices/Spice extracts | | | |
| Added amount | 0.00001% | 0.0001% | 0.001% | 0.01% |
| Tomato-like Flavor | + | ++ | ++++ | ++++ |

| | | | | |
|---|---|---|---|---|
| * "DONTACOS Chili Tomato" (manufactured by Koikeya Co., Ltd.) | | | | |

(3)
Separately, the yeast-fermented liquid powder was added in each of the amounts shown in Table 20, to a commercially available potato snack containing a wasabi raw material, and five experts performed the sensory evaluation of the spicy flavor-improving effect, relative to the control to which the yeast-fermented liquid powder had not been added. Wasabi-like pungency and stimulus intensity as well as refreshing flavor and gorgeousness were evaluated, as the spicy flavor. The evaluation criteria were as follows. The score was determined by the consensus of the five experts.
++++: There is an extremely strong improvement effect (this assumes that there is an effect at a degree clearly distinguishable even by a general panel of people who are not experts in sensory evaluation, which is a particularly strong effect (an effect stronger than ++).
++: There is a strong improvement effect (this assumes that there is an effect at a degree clearly distinguishable even by a general panel of people who are not experts in sensory evaluation).
+: There is an improvement effect (this assumes that there is an effect at a degree which is clearly distinguishable by the experts of sensory evaluation, but which is difficult to be distinguished by a general panel of people who are not experts in sensory evaluation).
-: There is no improvement effect.

The results of the sensory evaluation are shown in Table 20. An improvement in the spicy flavor could be confirmed at an addition rate of the yeast-fermented liquid powder of 0.00001%, and a clear improvement effect could be confirmed at an addition rate of 0.0001%, and more markedly at an addition rate of 0.001% or more. This has suggested that it is possible to enhance a refreshing spicy flavor that goes through the nose, and to enhance the spicy flavor with a stimulus that remains on the tongue, by adding 0.00001% or more of the yeast-fermented liquid powder to the potato snack.

### [Table 20]

**Table 20**

| | | | | |
|---|---|---|---|---|
| Food | Wasabi-flavored potato snack (*) | | | |
| Spices, etc. | Garlic powder, Red chili pepper, Wasabi powder/Spice extracts | | | |
| Added amount | 0.00001% | 0.0001% | 0.001% | 0.01% |
| Spicy flavor | + | ++ | ++++ | ++++ |

| | | | | |
|---|---|---|---|---|
| * "Wasabeef" (manufactured by YAMAYOSHI SEIKA Co., Ltd.) | | | | |

(4)
Separately, to mashed potato flakes, Hokkaido Jaga-mash, plain (manufactured by Calbee Co. Ltd.), hot water in an amount (in terms of mass) six times the amount of the flakes was added and mixed, to prepare mashed potatoes. To the thus prepared mashed potatoes, a prepared wasabi paste in a tube was added at a concentration of 10% and mixed thoroughly, and an aqueous solution of the yeast-fermented liquid powder was further added thereto so as to achieve each added amount shown in Table 21. Thereafter, five experts performed the sensory evaluation of the spicy flavor-improving effect, relative to the control to which the yeast-fermented liquid powder had not been added. The taste with wasabi-like pungency and stimulus, as well as wasabi-like refreshing flavor that goes through the nose and gorgeous flavor, were evaluated as the spicy flavor. The evaluation criteria were as follows. The score was determined by the consensus of the five experts.
++++: There is an extremely strong improvement effect (this assumes that there is an effect at a degree clearly distinguishable even by a general panel of people who are not experts in sensory evaluation, which is a particularly strong effect (an effect stronger than ++).
++: There is a strong improvement effect (this assumes that there is an effect at a degree clearly distinguishable even by a general panel of people who are not experts in sensory evaluation).
+: There is an improvement effect (this assumes that there is an effect at a degree which is clearly distinguishable by the experts of sensory evaluation, but which is difficult to be distinguished by a general panel of people who are not experts in sensory evaluation).
-: There is no improvement effect.

The results of the sensory evaluation are shown in Table 21. A marked improvement in the spicy flavor in the category of taste could be confirmed at an addition rate of the yeast-fermented liquid powder of 0.001 ppb. Further, an improvement in the spicy flavor in the category of flavor could be confirmed at an addition rate of the yeast-fermented liquid powder of 0.001 ppb, a clear improvement could be confirmed at an addition rate of 0.01 ppb, and more clearly at an addition rate of 0.1 ppb or more. This has suggested that it is possible to enhance the spicy flavor with a wasabi-like stimulus that remains on the tongue, and a wasabi-like refreshing flavor that goes through the nose, by adding 0.001 ppb or more of the yeast-fermented liquid powder to the wasabi paste.

### [Table 21]

**Table 21**

| Food | Prepared wasabi paste in tube (*) | | | | | |
|---|---|---|---|---|---|---|
| Added amount (ppb) | 0.001 | 0.01 | 0.1 | 1 | 10 | 100 |
| Taste | + + + + | + + + + | + + + + | + + + + | + + + + | + + + + |
| Flavor | + | + + | + + + + | + + + + | + + + + | + + + + |

| | | | | | | |
|---|---|---|---|---|---|---|
| * "Special selection - Fresh Wasabi Paste with Genuine Aroma" (manufactured by House Foods, Co., Ltd.) | | | | | | |

(5)
Separately, an aqueous solution of the yeast-fermented liquid powder was added to Sichuan style Mapo tofu, so as to achieve each added amount shown in Table 22, and five experts performed the sensory evaluation of the matured flavor-improving effect and the spicy flavor-improving effect, relative to the control to which the yeast-fermented liquid powder had not been added. The matured aroma, flavor and richness of douchi, tianmianjiang and doubanjiang were evaluated as the matured flavor. The pungency and the stimulus intensity, as well as the refreshing flavor and the gorgeousness imparted by spices, were evaluated as the spicy flavor. The evaluation criteria were as follows. The score was determined by the consensus of the five experts.
++++: There is an extremely strong improvement effect (this assumes that there is an effect at a degree clearly distinguishable even by a general panel of people who are not experts in sensory evaluation, which is a particularly strong effect (an effect stronger than ++).
++: There is a strong improvement effect (this assumes that there is an effect at a degree clearly distinguishable even by a general panel of people who are not experts in sensory evaluation).
+: There is an improvement effect (this assumes that there is an effect at a degree which is clearly distinguishable by the experts of sensory evaluation, but which is difficult to be distinguished by a general panel of people who are not experts in sensory evaluation).
-: There is no improvement effect.

The results of the sensory evaluation are shown in Table 22. An improvement in the spicy flavor could be confirmed at an addition rate of the yeast-fermented liquid powder of 0.001 ppb, and a more marked improvement could be confirmed at an addition rate of 0.01 ppb or more. This has suggested that it is possible to enhance a refreshing spicy flavor that goes through the nose, and the spicy flavor with a stimulus that remains on the tongue, by adding 0.001 ppb or more of the yeast-fermented liquid powder to the Sichuan style Mapo tofu. A marked improvement in the matured flavor could be confirmed at an addition rate of the yeast-fermented liquid powder of 0.001 ppb or more. This has suggested that it is possible to improve the matured flavor with the matured aroma, flavor and richness of douchi, tianmianjiang and doubanjiang, by adding 0.001 ppb or more of the yeast-fermented liquid powder to the Sichuan style Mapo tofu.

### [Table 22]

**Table 22**

| Food | Sichuan style Mapo tofu | | | | | |
|---|---|---|---|---|---|---|
| Spices, etc. | Tianmianjiang, Garlic, Doubanjiang, Douchi, Chili oil, Spice preparation, Chili pepper, Sichuan pepper | | | | | |
| Added amount (ppb) | 0.001 | 0.01 | 0.1 | 1 | 10 | 100 |
| Spicy flavor | + | + + + + | + + + + | + + + + | + + + + | + + + + |
| Matured flavor | + + | + + + + | + + + + | + + + + | + + + + | + + + + |

(6)
Separately, an aqueous solution of the yeast-fermented liquid powder was added to a tomato soup containing tomato raw materials, so as to achieve each added amount shown in Table 23, and five experts performed the sensory evaluation of the tomato-like-flavor-improving effect, relative to the control to which the yeast-fermented liquid powder had not been added. The fresh flavor of tomato and the ripe flavor of tomato were evaluated as the tomato-like flavor. The evaluation criteria were as follows. The score was determined by the consensus of the five experts.
++++: There is an extremely strong improvement effect (this assumes that there is an effect at a degree clearly distinguishable even by a general panel of people who are not experts in sensory evaluation, which is a particularly strong effect (an effect stronger than ++).
++: There is a strong improvement effect (this assumes that there is an effect at a degree clearly distinguishable even by a general panel of people who are not experts in sensory evaluation).
+: There is an improvement effect (this assumes that there is an effect at a degree which is clearly distinguishable by the experts of sensory evaluation, but which is difficult to be distinguished by a general panel of people who are not experts in sensory evaluation).
-: There is no improvement effect.

The results of the sensory evaluation are shown in Table 23.

An improvement in the tomato-like flavor could be confirmed at an addition rate of the yeast-fermented liquid powder of 0.001 ppb, and a more marked improvement could be confirmed at an addition rate of 0.01 ppb or more. This has suggested that it is possible to improve the tomato-like flavor, by adding 0.001 ppb or more the yeast-fermented liquid powder to the tomato soup.

### [Table 23]

**Table 23**

| Material | Tomato cup soup | | | | | |
|---|---|---|---|---|---|---|
| Tomato raw materials | Tomato powder (those made domestically and/or in Spain), Tomato extract, Fermented tomato powder, Floating soup ingredient (dried tomato) | | | | | |
| Added amount (ppb) | 0.001 | 0.01 | 0.1 | 1 | 10 | 100 |
| Fresh flavor | + + | + + + + | + + + + | + + + + | + + + + | + + + + |
| Ripe flavor | + | + | + + | + + + + | + + + + | + + + + |

### Example 11 Spice Screening Evaluation

The yeast-fermented liquid powder was obtained in the same manner as in Example 4.

To mashed potato flakes, Hokkaido Jaga-mash, plain (manufactured by Calbee Co. Ltd.), hot water in an amount (in terms of mass) six times the amount of the flakes was added and mixed, to prepare mashed potatoes. To the thus prepared mashed potatoes, a spice was added in the addition rate shown in Table 24 and mixed thoroughly, and the yeast-fermented liquid powder was added thereto at a concentration of 0.01%. Thereafter, four experts performed the sensory evaluation of the spicy flavor-improving effect, relative to the control to which the yeast-fermented liquid powder had not been added. The pungency and the stimulus intensity as well as the refreshing flavor and the gorgeousness that are specific to each corresponding spice, were evaluated as the spicy flavor. The evaluation criteria were as follows. The score was determined by the consensus of the four experts.
++++: There is an extremely strong improvement effect (this assumes that there is an effect at a degree clearly distinguishable even by a general panel of people who are not experts in sensory evaluation, which is a particularly strong effect (an effect stronger than ++).
++: There is a strong improvement effect (this assumes that there is an effect at a degree clearly distinguishable even by a general panel of people who are not experts in sensory evaluation).
+: There is an improvement effect (this assumes that there is an effect at a degree which is clearly distinguishable by the experts of sensory evaluation, but which is difficult to be distinguished by a general panel of people who are not experts in sensory evaluation).
-: There is no improvement effect.

The results of the sensory evaluation are shown in Table 24. The spicy flavor-improving effect could be confirmed by the addition of the yeast-fermented liquid powder, regardless of the type of the spice added. This has suggested that the addition of the yeast-fermented liquid powder enhances: the spicy flavor with pungency and stimulus, represented by those of Japanese pepper, Sichuan pepper, white pepper, black pepper, chili pepper and ginger; the spicy flavor of wasabi; the spicy flavor with sweetness, refreshingness and gorgeousness, represented by those of star anise, anise, fennel, clove, cumin, nutmeg, cardamom, cinnamon, basil, oregano and parsley; and the spicy flavor with coolness represented by that of peppermint.

### [Table 24]

**Table 24**

| No. | 1 | 2 | 3 | 4 | 5 | 6 | 7 | 8 |
|---|---|---|---|---|---|---|---|---|
| Spice type | Coriander | Cinnamon | Oregano | Basil | Parsley | Peppermint | Lemongrass | Fruit chutney |
| Added amount of Spice | 0.20% | 0.20% | 0.20% | 0.30% | 0.30% | 0.30% | 0.20% | 0.20% |
| Evaluation | + | + | + | + | + | + | + | + |

| No. | 9 | 10 | 11 | 12 | 13 | 14 | 15 | 16 |
|---|---|---|---|---|---|---|---|---|
| Spice type | Cardamom | Nutmeg | Cumin | Ginger | Clove | White pepper | Black pepper | Chili pepper |
| Added amount of Spice | 0.20% | 0.30% | 0.20% | 0.30% | 0.30% | 0.20% | 0.20% | 0.20% |
| Evaluation | + + | + + | + + | + + + + | + + + + | + + + + | + + + + | + + + + |

| No. | 17 | 18 | 19 | 20 | 21 | 22 | 23 | |
|---|---|---|---|---|---|---|---|---|
| Spice type | Japanese pepper | Sichuan pepper | Star anise | Anise | Fennel | Wasabi | Garlic | |
| Added amount of Spice | 0.20% | 0.20% | 0.20% | 0.30% | *0.20%* | 0.20% | 0.20% | |
| Evaluation | + + + + | + + + + | + + + + | + + + + | + + + + | + + + + | + + + + | |

### Example 12 Evaluation of Heat Resistance of Spicy Flavor Improving Effect and Matured Flavor Improving Effect

(1) The yeast-fermented liquid powder was obtained in the same manner as in Example 4.

Sichuan pepper, chili pepper, douchi, tianmianjiang, the yeast-fermented liquid powder, 2-PE or a commercially available yeast extract was added to Sichuan style Mapo tofu, at the concentration shown in Table 25, filled into a retort pouch, and heated at 126°C for 20 minutes. After cooling, seven experts performed the sensory evaluation of the effect of improving the spicy flavor and the matured flavor before and after the retort heat treatment. Chili pepper-like pungency and stimulus intensity, as well as Sichuan pepper-like refreshing flavor and gorgeousness, were each evaluated as the spicy flavor. A unique fermented flavor with douchi-like umami and bitterness, as well as a mild matured flavor with tianmianjiang-like sweetness and umami, were each evaluated as the matured flavor. As the evaluation criteria, the spicy flavor and the matured flavor of the Sichuan style Mapo tofu with no added material and before the heating were each taken as 3.0 points, and the spicy flavor and the matured flavor of the Sichuan style Mapo tofu with no added material and after the heating were each taken as 1.0 point. Further, the intensity of pungency and the intensity of the refreshing flavor, of the Sichuan style Mapo tofu to which chili pepper or Sichuan pepper was added and which is before the heating, were each taken as 5.0 points, and the fermented flavor and the mild matured flavor, of the Sichuan style Mapo tofu to which douchi or tianmianjiang was added and which is before the heating, were each taken as 5.0 points. The score was determined on a scale from 1.0 to 5.0 points with an increment of 0.5 points. A difference of 0.5 points assumes that there is an effect at a degree clearly distinguishable by those who are experts in the sensory evaluation, and a difference of 1.0 point assumes that there is an effect at a degree clearly distinguishable even by a general panel of people who are not experts in sensory evaluation. The average value of the evaluation results by the seven experts was taken as the final score.

The results of the sensory evaluation are shown in Table 25. It has been confirmed that the addition of the yeast-fermented liquid powder causes marked increases in the spicy flavor and the matured flavor, as compared to the case of adding 2-PE or a commercially available yeast extract. Further, in cases where the yeast-fermented liquid powder was added, the same levels of the spicy flavor and the brewed flavor as the Sichuan style Mapo tofu to which a raw material such as chili pepper, Sichuan pepper, douchi or tianmianjiang had been added were maintained, even after the retort treatment. This has shown that the yeast-fermented liquid powder exhibits the effect of improving the spicy flavor and the matured flavor even after the heat treatment.

### [Table 25]

**Table 25**

| No. | | 6 | | 7 | | 8 | | 9 | | 10 | | 11 | |
|---|---|---|---|---|---|---|---|---|---|---|---|---|---|
| Added material | | Yeast-fermented liquid powder | | Yeast-fermented liquid powder | | 2-PE | | Yeast Extract (*1) | | Yeast Extract (*2) | | Yeast Extract (*3) | |
| Added amount | | 0.01% | | 0.1 ppb | | 0.05% | | 0.10% | | 0.10% | | 0.10% | |
| Heating | | Before | After | Before | After | Before | After | Before | After | Before | After | Before | After |
| Spicy flavor | Pungency (chili pepper) | 3.6 | 2.9 | 4.3 | 3.6 | 3.4 | 2.9 | 3.5 | 2.7 | 3.2 | 2.6 | 3.3 | 2.6 |
| | Refreshing flavor (Sichuan pepper) | 3.9 | 2.9 | 4.3 | 3.3 | 3.4 | 2.7 | 3.3 | 2.1 | 3.1 | 2.2 | 3.0 | 2.2 |
| Matured flavor | Fermented flavor (douchi) | 4.5 | 3.5 | 4.1 | 3.3 | 4.1 | 3.0 | 3.7 | 2.4 | 3.6 | 2.7 | 3.5 | 2.6 |
| | Mildness (tianmianjiang) | 4.2 | 3.4 | 3.5 | 29 | 4.1 | 2.7 | 3.9 | 2.9 | 4.0 | 3.1 | 3.8 | 2.8 |

| | | | | | | | | | | | | | |
|---|---|---|---|---|---|---|---|---|---|---|---|---|---|
| *1 Yeastock GT-Pd (manufactured by Asahi Group Foods, Ltd.) *2 Yeast extract powder SG010 (manufactured by Angel Yeast Co., Ltd.) *3 AROMILD^{®} UG-8 (manufactured by KOHJIN Life Sciences Co., Ltd.) | | | | | | | | | | | | | |

(2)
Separately, the yeast-fermented liquid powder, a commercially available tomato-flavored seasoning powder or a commercially available yeast extract was added to a retort curry (retort Java Curry, medium spicy (manufactured by House Foods, Co., Ltd.), at the concentration shown in Table 26, filled into a retort pouch, and heated at 126°C for 20 minutes. After cooling, five experts performed the sensory evaluation of the effect of improving the spicy flavor and the matured flavor before and after the retort heat treatment. Chili pepper- or black pepper-like pungency and stimulus intensity, as well as the refreshing flavor and gorgeousness imparted by spices such as cardamom, coriander and clove, were each evaluated as the spicy flavor. The richness of vegetables and spices as well as the thickness of matured curry were evaluated as the matured flavor. As the evaluation criteria, the spicy flavor and the matured flavor of the retort curry with no added material and before the heating were each taken as 3.0 points, and the spicy flavor and the matured flavor of the retort curry with no added material and after the heating were each taken as 1.0 point. The score was determined on a scale from 1.0 to 5.0 points with an increment of 0.5 points. A difference of 0.5 points assumes that there is an effect at a degree clearly distinguishable by those who are experts in the sensory evaluation, and a difference of 1.0 point assumes that there is an effect at a degree clearly distinguishable even by a general panel of people who are not experts in sensory evaluation. The average value of the evaluation results by the five experts was taken as the final score.

The results of the sensory evaluation are shown in Table 26. It has been confirmed that the addition of the yeast-fermented liquid powder causes marked increases in the spicy flavor and the matured flavor, as compared to the case of adding 2-PE or a commercially available yeast extract. Further, in cases where the yeast-fermented liquid powder was added, the spicy flavor with pungency and stimulus intensity as well as refreshingness and gorgeousness, and the matured flavor such as the richness of vegetables and spices as well as the thickness resembling that of matured curry, were improved, even after the retort heat treatment. This has shown that the yeast-fermented liquid powder exhibits the function of improving the spicy flavor and the matured flavor even after the heat treatment.

### [Table 26]

**Table 26**

| No. | | 1 | | 2 | | 3 | | 4 | | 5 | | 6 | | 7 | | 8 | |
|---|---|---|---|---|---|---|---|---|---|---|---|---|---|---|---|---|---|
| Added material | | No added material | | Yeast-fermented liquid powder | | Tomato powder 1 | | Tomato powder 2 | | Tomato flavor (manufactured by T. HASEGAWA Co., Ltd.) | | Yeast Extract (*1) | | Yeast Extract (*2) | | Yeast Extract (*3) | |
| Added amount | | - | | 0.001% | | 0.20% | | 0.20% | | 0.01% | | 0.10% | | 0.10% | | 0.10% | |
| Heating | | Before | After | Before | After | Before | After | Before | After | Before | After | Before | After | Before | After | Before | After |
| Spicy flavor | Pungency | 3.0 | 1.0 | 3.6 | 3.8 | 2.5 | 1.3 | 2.8 | 1.9 | 2.1 | 13 | 2.9 | 2.1 | 2.9 | 2.6 | 2.8 | 2.1 |
| | Refreshing flavor | 3.0 | 1.0 | 4.8 | 4.1 | 3.3 | 2.0 | 3.5 | 1.9 | 2.8 | 1.6 | 3.0 | 2.0 | 3.3 | 2.0 | 3.1 | 2.1 |
| Matured flavor | | 3.0 | 1.0 | 4.0 | 3.2 | 3.3 | 2.3 | 3.1 | 2.1 | 3.3 | 1.7 | 3.6 | 2.7 | 3.6 | 2.8 | 3.9 | 2.9 |

| | | | | | | | | | | | | | | | | | |
|---|---|---|---|---|---|---|---|---|---|---|---|---|---|---|---|---|---|
| *1 Yeastock GT-Pd (manufactured by Asahi Group Foods, Ltd.) *2 Yeast extract powder SG010 (manufactured by Angel Yeast Co., Ltd.) *3 AROMILD^{®} UG-8 (manufactured by KOHJIN Life Sciences Co., Ltd.) | | | | | | | | | | | | | | | | | |

(3)
The yeast-fermented liquid powder or a commercially available tomato-flavored seasoning was added to RAGU Traditional Pasta Sauce (manufactured by Mizkan Ltd.), at the concentration shown in Table 27, filled into a retort pouch, and heated at 126°C for 20 minutes. After cooling, six experts performed the sensory evaluation of the tomato-like-flavor-improving effect before and after the retort heat treatment. The fresh flavor and the ripe flavor of tomato as well as the raw tomato-like flavor were evaluated as the tomato-like flavor. As the evaluation criteria, the tomato-like flavor of the tomato sauce with no added material and before the heating was taken as 3.0 points, and the tomato-like flavor of the tomato sauce with no added material and after the heating was taken as 1.0 point. The score was determined on a scale from 1.0 to 5.0 points with an increment of 0.5 points. A difference of 0.5 points assumes that there is an effect at a degree clearly distinguishable by those who are experts in the sensory evaluation, and a difference of 1.0 point assumes that there is an effect at a degree clearly distinguishable even by a general panel of people who are not experts in sensory evaluation. The average value of the evaluation results by the six experts was taken as the final score.

The results of the sensory evaluation are shown in Table 27. It has been confirmed that the addition of the yeast-fermented liquid powder causes a marked increase in the tomato-like flavor, as compared to the case of adding a tomato-flavored seasoning. Further, in cases where the yeast-fermented liquid powder was added, the characteristics that the acidity and the tomato-like flavor stand out from the initial taste were observed, even after the retort heat treatment. This has shown that the yeast-fermented liquid powder exhibits the tomato-like flavor-improving function even after the heat treatment.

### [Table 27]

**Table 27**

| No. | | 1 | | 2 | | 3 | | 4 | | 5 6 | | | |
|---|---|---|---|---|---|---|---|---|---|---|---|---|---|
| Added material | | No added material | | Yeast-fermented liquid powder | | Tomato powder 1 | | Tomato powder 2 | | Tomato flavor (manufactured by T. HASEGAWA Co., Ltd.) | | Tomato paste (manufactured by Kagome Co., Ltd.) | |
| Added amount | | - | | 0.01% | | 0.10% | | 0.10% | | 0.015% | | 0.50% | |
| Heating | | Before | After | Before | After | Before | After | Before | After | Before | After | Before | After |
| Tomato-like flavor | Fresh flavor | 3.0 | 1.0 | 4.3 | 3.3 | 2.9 | 2.0 | 3.8 | 2.3 | 4.0 | 2.8 | 3.3 | 2.1 |
| | Ripe flavor | 3.0 | 1.0 | 3.7 | 3.3 | 3.2 | 2.0 | 3.2 | 1.9 | 2.8 | 2.4 | 3.4 | 2.5 |
| | Raw tomato-like flavor | 3.0 | 1.0 | 3.5 | 2.9 | 3.3 | 1.5 | 3.4 | 2.4 | 4.1 | 2.8 | 3.3 | 2.3 |

### INDUSTRIAL AVAILABILITY

According to the present invention, it is possible to improve a flavor of food.

## Claims

1. A method of producing a composition for improving a flavor of food, the method comprising a step of culturing yeast in a culture medium containing a plant belonging to the family *Solanaceae,* to obtain a fermented product,
wherein the composition contains the fermented product.

2. The method according to claim 1, wherein the fermented product contains 2-phenylethanol.

3. The method according to claim 1, wherein the fermented product contains bacterial cells of the yeast.

4. A method of producing a composition comprising 2-phenylethanol, the method comprising a step of culturing yeast in a culture medium containing a plant belonging to the family *Solanaceae,* to obtain a fermented product containing 2-phenylethanol,
wherein the composition contains the fermented product.

5. The method according to claim 4, wherein the composition is a composition for improving a flavor of food.

6. The method according to any one of claims 1, 2, 3 and 5, wherein the improvement of the flavor is one or more selected from the group consisting of an improvement in tomato-like flavor, an improvement in spicy flavor and an improvement in matured flavor, in the food.

7. The method according to claim 6, wherein the tomato-like flavor is one or more selected from the group consisting of a fresh flavor of tomato, a ripe flavor of tomato, and a raw tomato-like flavor.

8. The method according to any one of claims 2 to 5, wherein the fermented product further contains ethyl acetate and/or diacetyl.

9. The method according to any one of claims 1 to 5, wherein the yeast is salt-tolerant yeast.

10. The method according to any one of claims 1 to 5, wherein the yeast is yeast belonging to the genus *Zygosaccharomyces,* yeast belonging to the genus *Saccharomyces,* yeast belonging to the genus *Pichia,* yeast belonging to the genus *Candida,* yeast belonging to the genus *Hansenula,* or yeast belonging to the genus *Schizosaccharomyces.*

11. The method according to any one of claims 1 to 5, wherein the yeast is *Zygosaccharomyces rouxii, Zygosaccharomyces sapae, Pichia farinosa, Candida versatilis, Candida etschelsii, Saccharomyces cerevisiae* or *Schizosaccharomyces pombe.*

12. The method according to any one of claims 1 to 5, wherein the yeast is *Zygosaccharomyces rouxii.*

13. The method according to any one of claims 1 to 5, wherein the plant belonging to the family *Solanaceae* is a plant belonging to the genus *Solanum* or a plant belonging to the genus *Capsicum.*

14. The method according to any one of claims 1 to 5, wherein the plant belonging to the family *Solanaceae* is tomato, eggplant, green bell pepper, paprika, Shishito pepper, or chili pepper.

15. The method according to any one of claims 1 to 5, wherein the plant belonging to the family *Solanaceae* is tomato.

16. The method according to any one of claims 1 to 5, wherein the content of the plant belonging to the family *Solanaceae* in the culture medium, in terms of salt-free soluble solid content, is from 0.1 to 10% (w/w).

17. The method according to any one of claims 1 to 5, wherein the culture medium further contains phenylalanine.

18. The method according to claim 17, wherein the content of phenylalanine in the culture medium is from 0.02 to 2% (w/w).

19. The method according to any one of claims 1 to 5, wherein the culture medium further contains sodium chloride.

20. The method according to any one of claims 1 to 5, wherein the content of sodium chloride in the culture medium is from 0 to 20% (w/w).

21. The method according to any one of claims 1 to 5, wherein the composition is a seasoning.

22. The method according to any one of claims 1 to 5, further comprising a step of drying and powdering the fermented product.

23. The method according to any one of claims 1 to 5, wherein the food is tomato-containing food.

24. The method according to any one of claims 1 to 5, wherein the food is spice-containing food.

25. The method according to claim 24, wherein the spice is one or more spices selected from the group consisting of cardamom, bay leaf, coriander, clove, nutmeg, mace, allspice, cinnamon, fennel, cumin, ginger, pepper, caraway, anise, basil, parsley, sage, thyme, oregano, rosemary, celery seed, mint, garden cress, dill, marjoram, knotweed, turmeric, lemongrass, chili pepper, Japanese pepper, garlic, shallot, onion, peppermint, Sichuan pepper, star anise, wasabi, and celery.

26. The method according to any one of claims 1 to 5, wherein the food is food containing: a seasoning obtained by fermenting beans or wheat; a seasoning containing rice malt or miso; or a seasoning obtained by fermenting beans or wheat and containing rice malt or miso.

27. The method according to any one of claims 1 to 5, wherein the food is milk-containing food and/or a dairy product.

28. A composition produced by the method according to any one of claims 1 to 5.

29. A composition for improving a flavor of food,
wherein the composition contains a yeast-fermented product of a plant belonging to the family *Solanaceae.*

30. The composition according to claim 29, wherein the improvement of the flavor is one or more selected from the group consisting of an improvement in tomato-like flavor, an improvement in spicy flavor and an improvement in matured flavor, in the food.

31. The composition according to claim 29 or 30, wherein the fermented product contains bacterial cells of the yeast.

32. The composition according to claim 29 or 30, wherein the fermented product contains 2-phenylethanol.

33. The composition according to claim 32, wherein the fermented product further contains ethyl acetate and/or diacetyl.

34. The composition according to claim 29 or 30, wherein the fermented product is produced by a step of culturing yeast in a culture medium containing the plant.

35. The composition according to claim 29 or 30, wherein the yeast is *Zygosaccharomyces rouxii.*

36. The composition according to claim 29 or 30, wherein the plant belonging to the family *Solanaceae* is tomato.

37. The composition according to claim 34, wherein the culture medium further contains phenylalanine.

38. The composition according to claim 29 or 30, wherein the food is tomato-containing food.

39. The composition according to claim 29 or 30, wherein the food is spice-containing food.

40. The composition according to claim 39, wherein the spice is one or more spices selected from the group consisting of cardamom, bay leaf, coriander, clove, nutmeg, mace, allspice, cinnamon, fennel, cumin, ginger, pepper, caraway, anise, basil, parsley, sage, thyme, oregano, rosemary, celery seed, mint, garden cress, dill, marjoram, knotweed, turmeric, lemongrass, chili pepper, Japanese pepper, garlic, shallot, onion, peppermint, Sichuan pepper, star anise, wasabi, and celery.

41. The composition according to claim 29 or 30, wherein the food is food containing: a seasoning obtained by fermenting beans or wheat; a seasoning containing rice malt or miso; or a seasoning obtained by fermenting beans or wheat and containing rice malt or miso.

42. The composition according to claim 29 or 30, wherein the food is milk-containing food and/or a dairy product.

43. A method of improving a flavor of food, the method comprising a step of adding a yeast-fermented product of a plant belonging to the family *Solanaceae* to a raw material of the food.

44. The method according to claim 43, wherein the improvement of the flavor is one or more selected from the group consisting of an improvement in tomato-like flavor, an improvement in spicy flavor and an improvement in matured flavor, in the food.

45. The method according to claim 43 or 44, wherein the fermented product contains bacterial cells of the yeast.

46. The method according to claim 43 or 44, wherein the fermented product contains 2-phenylethanol.

47. The method according to claim 46, wherein the fermented product further contains ethyl acetate and/or diacetyl.

48. The method according to claim 43 or 44, wherein the fermented product is produced by a step of culturing yeast in a culture medium containing the plant.

49. The method according to claim 43 or 44, wherein the yeast is *Zygosaccharomyces rouxii.*

50. The method according to claim 43 or 44, wherein the plant belonging to the family *Solanaceae* is tomato.

51. The method according to claim 48, wherein the culture medium further contains phenylalanine.

52. The method according to claim 43 or 44, wherein the food is tomato-containing food.

53. The method according to claim 43 or 44, wherein the food is spice-containing food.

54. The method according to claim 53, wherein the spice is one or more spices selected from the group consisting of cardamom, bay leaf, coriander, clove, nutmeg, mace, allspice, cinnamon, fennel, cumin, ginger, pepper, caraway, anise, basil, parsley, sage, thyme, oregano, rosemary, celery seed, mint, garden cress, dill, marjoram, knotweed, turmeric, lemongrass, chili pepper, Japanese pepper, garlic, shallot, onion, peppermint, Sichuan pepper, star anise, wasabi and celery.

55. The method according to claim 43 or 44, wherein the food is milk-containing food and/or a dairy product.

56. The method according to claim 43 or 44, wherein the food is food containing: a seasoning obtained by fermenting beans or wheat; a seasoning containing rice malt or miso; or a seasoning obtained by fermenting beans or wheat and containing rice malt or miso.

57. The method according to claim 43 or 44, wherein the fermented product is added such that the eating concentration, in terms of the eating concentration of the original fermented product, is from 1 × 10⁻¹⁴ to 10% (w/w).

58. The method according to claim 46, wherein the fermented product is added such that the eating concentration, in terms of the eating concentration of 2-phenylethanol, is from 1 × 10⁻¹³ to 100 ppm (w/w).
